# EUROPEAN PATENT APPLICATION

(11) **EP 1 360 964 A1**
(43) Date of publication of application: **12.11.2003**
(21) Application number: 02742457.1
(22) Date of filing: 18.01.2002
(51) Int. Cl.: A61K 45/00, A61K 38/09, A61K 38/22, A61K 31/711, A61P 5/12, A61P 5/10, A61P 15/08, A61P 15/12, A61P 3/04, A61P 35/00, A61P 13/08, A61P 15/00

(54) **USE OF GALANIN-LIKE PEPTIDE**

(30) Priority: 19.01.2001 JP 2001012094
(71) Applicant: Takeda Chemical Industries, Ltd., Osaka-shi, Osaka 541-0045 (JP)
(72) Inventor: MATSUMOTO, Hirokazu, Tsukuba-shi, Ibaraki 305-0821 (JP); NOGUCHI, Jiro, Tsukuba-shi, Ibaraki 305-0051 (JP); OOTAKI, Tetsuya, Tsukuba-shi, Ibaraki 305-0821 (JP)
(74) Representative: Rickard, Timothy Mark Adrian
(86) International application number: JP0200313
(87) International publication number: WO02066064

(57) **Abstract**

The present invention is intended to provide a novel use of a galanin-like peptide (GALP), more specifically, an agent regulating the luteinizing hormone (LH) secretion comprising GALP, etc. The agent regulating the luteinizing hormone (LH) secretion are useful as a prophylactic and/or therapeutic agent for infertility, menoxenia, menopausal disorder, dyspituitarism, obesity, etc.

Sequence Listing

<110> Takeda Chemical Industries, Ltd.

<120> Use of Galanin-like Peptide

<130> P01-0291PCT

<140> PCT/JP02/00313
<141> 2002-01-18

<150> JP 2001-12094
<151> 2001-01-19

<160> 72

<210> 1
<211> 346
<212> PRT
<213> Rat

<400> 1
Met Glu Leu Ala Pro Val Asn Leu Ser Glu Gly Asn Gly Ser Asp Pro
1               5                   10                  15
Glu Pro Pro Ala Glu Pro Arg Pro Leu Phe Gly Ile Gly Val Glu Asn
            20                  25                  30
Phe Ile Thr Leu Val Val Phe Gly Leu Ile Phe Ala Met Gly Val Leu
        35                  40                  45
Gly Asn Ser Leu Val Ile Thr Val Leu Ala Arg Ser Lys Pro Gly Lys

    50                  55                  60
Pro Arg Ser Thr Thr Asn Leu Phe Ile Leu Asn Leu Ser Ile Ala Asp
65                  70                  75                  80
Leu Ala Tyr Leu Leu Phe Cys Ile Pro Phe Gln Ala Thr Val Tyr Ala
                85                  90                  95
Leu Pro Thr Trp Val Leu Gly Ala Phe Ile Cys Lys Phe Ile His Tyr
            100                 105                 110
Phe Phe Thr Val Ser Met Leu Val Ser Ile Phe Thr Leu Ala Ala Met
        115                 120                 125
Ser Val Asp Arg Tyr Val Ala Ile Val His Ser Arg Arg Ser Ser Ser
    130                 135                 140
Leu Arg Val Ser Arg Asn Ala Leu Leu Gly Val Gly Phe Ile Trp Ala
145                 150                 155                 160
Leu Ser Ile Ala Met Ala Ser Pro Val Ala Tyr Tyr Gln Arg Leu Phe
                165                 170                 175
His Arg Asp Ser Asn Gln Thr Phe Cys Trp Glu His Trp Pro Asn Gln
            180                 185                 190
Leu His Lys Lys Ala Tyr Val Val Cys Thr Phe Val Phe Gly Tyr Leu
        195                 200                 205
Leu Pro Leu Leu Leu Ile Cys Phe Cys Tyr Ala Lys Val Leu Asn His
    210                 215                 220
Leu His Lys Lys Leu Lys Asn Met Ser Lys Lys Ser Glu Ala Ser Lys
225                 230                 235                 240
Lys Lys Thr Ala Gln Thr Val Leu Val Val Val Val Val Phe Gly Ile
                245                 250                 255
Ser Trp Leu Pro His His Val Ile His Leu Trp Ala Glu Phe Gly Ala
            260                 265                 270
Phe Pro Leu Thr Pro Ala Ser Phe Phe Phe Arg Ile Thr Ala His Cys
        275                 280                 285

Leu Ala Tyr Ser Asn Ser Ser Val Asn Pro Ile Ile Tyr Ala Phe Leu
    290                 295                 300
Ser Glu Asn Phe Arg Lys Ala Tyr Lys Gln Val Phe Lys Cys Arg Val
305                 310                 315                 320
Cys Asn Glu Ser Pro His Gly Asp Ala Lys Glu Lys Asn Arg Ile Asp
                325                 330                 335
Thr Pro Pro Ser Thr Asn Cys Thr His Val
            340                 345 346

<210> 2
<211> 372
<212> PRT
<213> Rat

<400> 2
Met Asn Gly Ser Gly Ser Gln Gly Ala Glu Asn Thr Ser Gln Glu Gly
 1               5                  10                  15
Gly Ser Gly Gly Trp Gln Pro Glu Ala Val Leu Val Pro Leu Phe Phe
            20                  25                  30
Ala Leu Ile Phe Leu Val Gly Thr Val Gly Asn Ala Leu Val Leu Ala
        35                  40                  45
Val Leu Leu Arg Gly Gly Gln Ala Val Ser Thr Thr Asn Leu Phe Ile
    50                  55                  60
Leu Asn Leu Gly Val Ala Asp Leu Cys Phe Ile Leu Cys Cys Val Pro
 65                 70                  75                  80
Phe Gln Ala Thr Ile Tyr Thr Leu Asp Asp Trp Val Phe Gly Ser Leu
                85                  90                  95
Leu Cys Lys Ala Val His Phe Leu Ile Phe Leu Thr Met His Ala Ser
            100                 105                 110

Ser Phe Thr Leu Ala Ala Val Ser Leu Asp Arg Tyr Leu Ala Ile Arg
        115                 120                 125
Tyr Pro Leu His Ser Arg Glu Leu Arg Thr Pro Arg Asn Ala Leu Ala
    130                 135                 140
Ala Ile Gly Leu Ile Trp Gly Leu Ala Leu Leu Phe Ser Gly Pro Tyr
145                 150                 155                 160
Leu Ser Tyr Tyr Arg Gln Ser Gln Leu Ala Asn Leu Thr Val Cys His
                165                 170                 175
Pro Ala Trp Ser Ala Pro Arg Arg Arg Ala Met Asp Leu Cys Thr Phe
            180                 185                 190
Val Phe Ser Tyr Leu Leu Pro Val Leu Val Leu Ser Leu Thr Tyr Ala
        195                 200                 205
Arg Thr Leu Arg Tyr Leu Trp Arg Thr Val Asp Pro Val Thr Ala Gly
    210                 215                 220
Ser Gly Ser Gln Arg Ala Lys Arg Lys Val Thr Arg Met Ile Ile Ile
225                 230                 235                 240
Val Ala Val Leu Phe Cys Leu Cys Trp Met Pro His His Ala Leu Ile
                245                 250                 255
Leu Cys Val Trp Phe Gly Arg Phe Pro Leu Thr Arg Ala Thr Tyr Ala
            260                 265                 270
Leu Arg Ile Leu Ser His Leu Val Ser Tyr Ala Asn Ser Cys Val Asn
        275                 280                 285
Pro Ile Val Tyr Ala Leu Val Ser Lys His Phe Arg Lys Gly Phe Arg
    290                 295                 300
Lys Ile Cys Ala Gly Leu Leu Arg Pro Ala Pro Arg Arg Ala Ser Gly
305                 310                 315                 320
Arg Val Ser Ile Leu Ala Pro Gly Asn His Ser Gly Ser Met Leu Glu
                325                 330                 335
Gln Glu Ser Thr Asp Leu Thr Gln Val Ser Glu Ala Ala Gly Pro Leu

            340                 345                 350
Val Pro Pro Pro Ala Leu Pro Asn Cys Thr Ala Ser Ser Arg Thr Leu
        355                 360                 365
Asp Pro Ala Cys
370 372

<210> 3
<211> 370
<212> PRT
<213> Rat

<400> 3
Met Ala Asp Ile Gln Asn Ile Ser Leu Asp Ser Pro Gly Ser Val Gly
1               5                   10                  15
Ala Val Ala Val Pro Val Ile Phe Ala Leu Ile Phe Leu Leu Gly Met
            20                  25                  30
Val Gly Asn Gly Leu Val Leu Ala Val Leu Leu Gln Pro Gly Pro Ser
        35                  40                  45
Ala Trp Gln Glu Pro Ser Ser Thr Thr Asp Leu Phe Ile Leu Asn Leu
    50                  55                  60
Ala Val Ala Asp Leu Cys Phe Ile Leu Cys Cys Val Pro Phe Gln Ala
65                  70                  75                  80
Ala Ile Tyr Thr Leu Asp Ala Trp Leu Phe Gly Ala Phe Val Cys Lys
                85                  90                  95
Thr Val His Leu Leu Ile Tyr Leu Thr Met Tyr Ala Ser Ser Phe Thr
            100                 105                 110
Leu Ala Ala Val Ser Leu Asp Arg Tyr Leu Ala Val Arg His Pro Leu
        115                 120                 125
Arg Ser Arg Ala Leu Arg Thr Pro Arg Asn Ala Arg Ala Ala Val Gly

130                 135                 140
Leu Val Trp Leu Leu Ala Ala Leu Phe Ser Ala Pro Tyr Leu Ser Tyr
145                 150                 155                 160
Tyr Gly Thr Val Arg Tyr Gly Ala Leu Glu Leu Cys Val Pro Ala Trp
                165                 170                 175
Glu Asp Ala Arg Arg Arg Ala Leu Asp Val Ala Thr Phe Ala Ala Gly
            180                 185                 190
Tyr Leu Leu Pro Val Ala Val Val Ser Leu Ala Tyr Gly Arg Thr Leu
        195                 200                 205
Cys Phe Leu Trp Ala Ala Val Gly Pro Ala Gly Ala Ala Ala Ala Glu
    210                 215                 220
Ala Arg Arg Arg Ala Thr Gly Arg Ala Gly Arg Ala Met Leu Ala Val
225                 230                 235                 240
Ala Ala Leu Tyr Ala Leu Cys Trp Gly Pro His His Ala Leu Ile Leu
                245                 250                 255
Cys Phe Trp Tyr Gly Arg Phe Ala Phe Ser Pro Ala Thr Tyr Ala Cys
            260                 265                 270
Arg Leu Ala Ser His Cys Leu Ala Tyr Ala Asn Ser Cys Leu Asn Pro
        275                 280                 285
Leu Val Tyr Ser Leu Ala Ser Arg His Phe Arg Ala Arg Phe Arg Arg
    290                 295                 300
Leu Trp Pro Cys Gly Arg Arg Arg His Arg His His His Arg Ala His
305                 310                 315                 320
Arg Ala Leu Arg Arg Val Gln Pro Ala Ser Ser Gly Pro Ala Gly Tyr
                325                 330                 335
Pro Gly Asp Ala Arg Pro Arg Gly Trp Ser Met Glu Pro Arg Gly Asp
            340                 345                 350
Ala Leu Arg Gly Gly Gly Glu Thr Arg Leu Thr Leu Ser Pro Arg Gly
        355                 360                 365

Pro Gln

    370

<210> 4
<211> 1041
<212> DNA
<213> Rat

<400> 4
atggaactgg ctccggtgaa cctcagtgaa gggaatggga gcgaccctga acctccagcg 60
gaacccaggc cgctcttcgg catcggcgtg gagaacttca tcacgctggt ggtgtttggc 120
cttattttcg cgatgggcgt gctgggcaac agcctggtga tcaccgtgct ggcgcgcagc 180
aaaccgggca agccgcgcag caccaccaac ctgttcatcc tcaacctgag catcgcagac 240
ctggcctacc tgctcttctg catccctttc caggccaccg tgtacgcact gcccacctgg 300
gtgctgggcg ccttcatctg caagtttata cactacttct tcaccgtgtc catgctcgtg 360
agcatcttca ccctggccgc gatgtctgtg gatcgctatg tggccattgt gcattcacgg 420
cgctcctcct ccctcagggt gtcccgcaac gcgctgctgg gcgtgggctt catctgggcg 480
ctgtccatcg ctatggcctc gccggtggcc tactaccagc gcctttttca tcgggacagc 540
aaccaaacct tctgctggga gcactggccc aaccaactcc acaagaaggc ttacgtggtg 600
tgcactttcg tctttggtta ccttctgccc ttactgctca tctgcttttg ctatgccaag 660
gttctcaatc atctgcataa aaagttgaag aacatgtcaa aaaagtcaga ggcatccaag 720
aaaaagactg cacagactgt cctggtggtc gttgtggtat ttggcatatc atggctgccc 780
catcatgtca tccacctctg ggctgagttc ggagcattcc cgctgacccc agcttccttc 840
ttcttcagaa tcactgccca ctgcctggca tacagcaact cctcggtgaa ccccatcatc 900
tacgcctttc tctcagaaaa cttccggaag gcgtacaagc aagtgttcaa gtgccgtgtt 960
tgcaatgagt cgccgcacgg cgatgctaaa gaaaagaacc gaatagatac cccgccctcc 1020
accaactgca cccacgtgtg a 1041

<210> 5

<211> 1119

<212> DNA

<213> Rat

<400> 5

atgaatggct ccggcagcca gggcgcggag aacacgagcc aggaaggcgg tagcggcggc 60

tggcagcctg aggcggtcct tgtaccccta tttttcgcgc tcatcttcct cgtgggcacc 120

gtgggcaacg cgctggtgct ggcggtgctg ctgcgcggcg gccaggcggt cagcaccacc 180

aacctgttca tcctcaacct gggcgtggcc gacctgtgtt tcatcctgtg ctgcgtgcct 240

ttccaggcca ccatctacac cctggacgac tgggtgttcg gctcgctgct ctgcaaggct 300

gttcatttcc tcatctttct cactatgcac gccagcagct tcacgctggc cgccgtctcc 360

ctggacaggt atctggccat ccgctacccg ctgcactccc gagagttgcg cacacctcga 420

aacgcgctgg ccgccatcgg gctcatctgg gggctagcac tgctcttctc cgggccctac 480

ctgagctact accgtcagtc gcagctggcc aacctgacag tatgccaccc agcatggagc 540

gcacctcgac gtcgagccat ggacctctgc accttcgtct ttagctacct gctgccagtg 600

ctagtcctca gtctgaccta tgcgcgtacc ctgcgctacc tctggcgcac agtcgacccg 660

gtgactgcag gctcaggttc ccagcgcgcc aaacgcaagg tgacacggat gatcatcatc 720

gtggcggtgc ttttctgcct ctgttggatg ccccaccacg cgcttatcct ctgcgtgtgg 780

tttggtcgct tcccgctcac gcgtgccact tacgcgttgc gcatcctttc acacctagtt 840

tcctatgcca actcctgtgt caaccccatc gtttacgctc tggtctccaa gcatttccgt 900

aaaggtttcc gcaaaatctg cgcgggcctg ctgcgccctg ccccgaggcg agcttcgggc 960

cgagtgagca tcctggcgcc tgggaaccat agtggcagca tgctggaaca ggaatccaca 1020

gacctgacac aggtgagcga ggcagccggg ccccttgtcc caccacccgc acttcccaac 1080

tgcacagcct cgagtagaac cctggatccg gcttgttaa 1119

<210> 6

<211> 1113

<212> DNA

<213> Rat

<400> 6

atggctgaca tccagaacat ttcgctggac agcccaggga gcgtaggggc tgtggcagtg 60

cctgtgatct ttgccctcat cttcctgttg ggcatggtgg gcaatgggct ggtgttggct 120

gtgctactgc agcctggccc aagtgcctgg caggagccaa gcagtaccac agatctcttc 180

atcctcaact tggccgtggc cgacctttgc ttcatcctgt gctgcgtgcc cttccaggca 240

gccatctaca cactggatgc ctggctcttt ggggctttcg tgtgcaagac ggtacatctg 300

ctcatctacc tcaccatgta tgccagcagc ttcaccctgg cggccgtctc cctggacagg 360

tacctggctg tgcggcaccc actgcgctcc agagccctgc gcaccccgcg caacgcgcgc 420

gccgccgtgg ggctcgtgtg gctgctggcg gctctctttt ccgcgcccta cctaagctat 480

tacggcacgg tgcgctacgg cgcgctcgag ctctgcgtgc ccgcttggga ggacgcgcgg 540

cggcgcgcgc tggacgtggc caccttcgcc gcgggctacc tgctgccggt ggccgtggtg 600

agcctggcct acggacgcac gctatgtttc ctatgggccg ccgtgggtcc cgcgggcgcg 660

gcggcagcag aggcgcgcag acgggcgacc ggccgggcgg gacgcgccat gctggcagtg 720

gccgcgctct acgcgctttg ctggggcccg caccacgcgc tcatcctctg cttctggtac 780

ggccgcttcg ccttcagccc ggccacctac gcctgtcgcc tggcctcgca ctgcctcgcc 840

tacgccaact cctgccttaa cccgctcgtc tactcgctcg cctcgcgcca cttccgcgcg 900

cgcttccgcc gcctgtggcc ctgcggccgt cgccgccacc gccaccacca ccgcgctcat 960

cgagccctcc gtcgtgtcca gccggcgtct tcgggccccg ccggttatcc cggcgacgcc 1020

aggcctcgtg gttggagtat ggagcccaga ggggatgctc tgcgtggtgg tggagagact 1080

agactaaccc tgtcccccag gggacctcaa taa 1113

<210> 7

<211> 29

<212> PRT

<213> Porcine

<400> 7

Gly Trp Thr Leu Asn Ser Ala Gly Tyr Leu Leu Gly Pro His Ala Ile

1                   5                        10                       15

Asp Asn His Arg Ser Phe His Asp Lys Tyr Gly Leu Ala

                20                       25                   29

<210> 8

<211> 123

<212> PRT

<213> Porcine

<400> 8

Met Pro Arg Gly Cys Ala Leu Leu Leu Ala Ser Leu Leu Leu Ala Ser

1                   5                        10                       15

Ala Leu Ser Ala Thr Leu Gly Leu Gly Ser Pro Val Lys Glu Lys Arg

                20                       25                       30

Gly Trp Thr Leu Asn Ser Ala Gly Tyr Leu Leu Gly Pro His Ala Ile

           35                       40                       45

Asp Asn His Arg Ser Phe His Asp Lys Tyr Gly Leu Ala Gly Lys Arg

      50                       55                       60

Glu Leu Glu Pro Glu Asp Glu Ala Arg Pro Gly Gly Phe Asp Arg Leu

65                       70                       75                       80

Gln Ser Glu Asp Lys Ala Ile Arg Thr Ile Met Glu Phe Leu Ala Phe

                          85                       90                       95

Leu His Leu Lys Glu Ala Gly Ala Leu Gly Arg Leu Pro Gly Leu Pro

                100                      105                      110

Ser Ala Ala Ser Ser Glu Asp Ala Gly Gln Ser

           115                      120            123

<210> 9

<211> 38

<212> PRT
<213> Porcine

<400> 9
Leu Gly Ser Pro Val Lys Glu Lys Arg Gly Trp Thr Leu Asn Ser Ala
1               5                   10                  15
Gly Tyr Leu Leu Gly Pro His Ala Ile Asp Asn His Arg Ser Phe His
            20                  25                  30
Asp Lys Tyr Gly Leu Ala
        35          38

<210> 10
<211> 25
<212> PRT
<213> Porcine

<400> 10
Asn Ser Ala Gly Tyr Leu Leu Gly Pro His Ala Ile Asp Asn His Arg
1               5                   10                  15
Ser Phe His Asp Lys Tyr Gly Leu Ala
            20                  25

<210> 11
<211> 34
<212> PRT
<213> Porcine

<220>
<221> unsure

<222> 28
<223> Xaa at the 28th position is unidentified.

<400> 11
Ala Pro Val His Arg Gly Arg Gly Gly Trp Thr Leu Asn Ser Ala Gly
1               5                   10                  15
Tyr Leu Leu Gly Pro Val Leu His Pro Pro Ser Xaa Ala Glu Gly Gly
            20                  25                  30
Gly Lys
    34

<210> 12
<211> 32
<212> PRT
<213> Porcine

<220>
<221> unsure
<222> 28
<223> Xaa at the 28th position is unidentified.

<400> 12
Ala Pro Val His Arg Gly Arg Gly Gly Trp Thr Leu Asn Ser Ala Gly
1               5                   10                  15
Tyr Leu Leu Gly Pro Val Leu His Pro Pro Ser Xaa Ala Glu Gly Gly
            20                  25                  30      32

<210> 13
<211> 9

<212> PRT

<213> Porcine

<400> 13

Ala Pro Val His Arg Gly Arg Gly Gly

1 5 9

<210> 14

<211> 6

<212> PRT

<213> Porcine

<400> 14

Thr Leu Asn Ser Ala Gly

1 5 6

<210> 15

<211> 27

<212> PRT

<213> Porcine

<220>

<221> unsure

<222> 11

<223> Xaa at the 11th position is unidentified.

<400> 15

Leu Leu Gly Pro Val Leu His Pro Pro Ser Xaa Ala Glu Gly Gly Gly

1               5                   10                  15

Lys Gly Lys Thr Ala Leu Gly Ile Leu Asp Leu
20 25 27

<210> 16
<211> 11
<212> PRT
<213> Porcine

<220>
<221> unsure
<222> 1
<223> Xaa at the 1st position is unidentified.

<400> 16
Xaa Ala Ile Asp Gly Leu Pro Tyr Pro Gln Ser
1 5 10 11

<210> 17
<211> 44
<212> PRT
<213> Porcine

<220>
<221> unsure
<222> 28
<223> Xaa at the 28th position is unidentified.

<400> 17
Ala Pro Val His Arg Gly Arg Gly Gly Trp Thr Leu Asn Ser Ala Gly

1                   5                   10                  15
Tyr Leu Leu Gly Pro Val Leu His Pro Pro Ser Xaa Ala Glu Gly Gly
            20                  25                  30
Gly Lys Gly Lys Thr Ala Leu Gly Ile Leu Asp Leu
        35                  40              44

<210> 18
<211> 27
<212> DNA
<213> Artificial Sequence

<220>
<223> primer 1

<400> 18
gtcgacatga atggctccgg cagccag   27

<210> 19
<211> 32
<212> DNA
<213> Artificial Sequence

<220>
<223> primer 2

<400> 19
actagtttaa caagccggat ccagggttct ac   32

<210> 20

<211> 23
<212> DNA
<213> Artificial Sequence

<220>
<223> degenerate primer pGAL4-7F

<220>
<221> misc_feature
<222> 6
<223> n is any base.

<220>
<221> misc_feature
<222> 9
<223> n is inosine.

<220>
<221> misc_feature
<222> 12
<223> n is any base.

<220>
<221> misc_feature
<222> 15
<223> n is inosine.

<400> 20
caymgnggnm gnggnggstg gac   23

<210> 21

<211> 20

<212> DNA

<213> Artificial Sequence

<220>

<223> degenerate primer pGAL9-3F

<220>

<221> misc_feature

<222> 9

<223> n is any base.

<220>

<221> misc_feature

<222> 12

<223> n is any base.

<400> 21

gghtggacnc tnaayagygc  20

<210> 22

<211> 23

<212> DNA

<213> Artificial Sequence

<220>

<223> degenerate primer pGAL34-1R

<220>

<221> misc_feature

<222> 3

<223> n is inosine.

<220>

<221> misc_feature

<222> 6

<223> n is any base.

<220>

<221> misc_feature

<222> 9

<223> n is inosine.

<220>

<221> misc_feature

<222> 12

<223> n is any base.

<220>

<221> misc_feature

<222> 18

<223> n is inosine.

<400> 22

atnccnagng cngtyttncc ytt   23

<210> 23

<211> 98

<212> DNA

<213> Porcine

<400> 23

ggctggactt taaatagtgc tggttacctc ctgggtcccg tactccatcc gccctccagg 60

gctgaaggag gcgggaaggg caaaacagcc ctgggcat 98

<210> 24

<211> 98

<212> DNA

<213> Porcine

<400> 24

ggttggactt tgaacagtgc tggttacctc ctgggtcccg tactccatcc gccctccagg 60

gctgaaggag gcgggaaggg caaaaccgcc ctaggcat 98

<210> 25

<211> 20

<212> DNA

<213> Artificial Sequence

<220>

<223> degenerate primer pGAL9-3F

<220>

<221> misc_feature

<222> 9

<223> n is any base.

<220>
<221> misc_feature
<222> 12
<223> n is any base.

<400> 25
gghtggacnc tnaayagygc  20

<210> 26
<211> 23
<212> DNA
<213> Artificial Sequence

<220>
<223> degenerate primer pGAL34-8R

<400> 26
atdccbaggg cdgttttgcc ctt  23

<210> 27
<211> 974
<212> DNA
<213> Porcine

<400> 27
ttcagcctca agcacccatc cctccagccc tcagatggct ctgactgtcc ctctgatcgt    60
tcttgcagtc ctgctcagcc tgatggagtc tccagcctct gctccggtcc acagggggcg    120

aggaggctgg accctcaaca gtgctggtta cctcctgggt cccgtactcc atccgccctc 180

cagggctgaa ggaggcggga aggggaagac agccctcggg atcctggacc tgtggaaggc 240

cattgatggg ctcccctatc cccagtctca gttggcctcc aagaggagtc tgggggagac 300

tttcgccaaa ccagactctg gagtaacatt tgttggagtt cctgacgtgg tgccgtggaa 360

acgaatccga ccaggaacta cgaggtttca gatctaggca agctctgcaa gaacgttcca 420

aaggagaaag atgccttgcc gtcatatatg cctccaaact tccgctccaa acttcccccc 480

cgtctccaga tcctcctgaa accctaggta gacaccctct actgagactg ggagcctgaa 540

agtaaatccc caaatcccag gtagaaaatg ggagcatttt gaagaattat tctcaaaagt 600

ccccggactg tgccaggttt cactgatccc cccctccccc ttggactaag tgtaaagcga 660

tgtaaaccaa ctcaagaata attctgaaac cattcaggag atccggagag gaatcgggaa 720

atactcctgc agtgcattta aagtaactgg gtcctatgca acatgagcca ttggatcata 780

caatattgat atcccttcta acacggaggt tctagggtgt ctcagctgga aaagattctt 840

cagagtagca tgcttgcctt accccatcct tctcacccca ccccgagcct cctccagcag 900

aaaggacgag aggccatctg gagcagagca gagagaataa atattccctt tcaaagaaaa 960

aaaaaaaaaa aaaa 974

<210> 28

<211> 1007

<212> DNA

<213> Porcine

<400> 28

ggaacgagct ggggagagct gccgactgca ggcagccttc ttcagcctca agcacccatc 60

cctccagccc tcagatggct ctgactgtcc ctctgatcgt tcttgcagtc ctgctcagcc 120

tgatggagtc tccagcctct gctccggtcc acagggggcg aggaggctgg accctcaaca 180

gtgctggtta cctcctgggt cccgtactcc atccgccctc cagggctgaa ggaggcggga 240

aggggaagac agccctcggg atcctggacc tgtggaaggc cattgatggg ctcccctatc 300

cccagtctca gttggcctcc aagaggagtc tggggagact ttcgccaaac cagactctgg 360

agtaacattt gttggagttc ctgacgtggt gccgtggaaa cgaatccgac caggaactac 420

gaggtttcag atctaggcaa gctctgcaag aacgttccaa aggagaaaga tgccttgtcg 480
tcatatatgc ctccaaactt ccgctccaaa cttccccccc gtccccagat cctcctgaaa 540
ccctaggtag acaccctcta ctgagactgg gagcctgaaa gtaaatcccc aaatcccagg 600
tagaaaatgg ggagcatttg aagaattatt ctcaaaagtc cccggactgt gccaggtttc 660
actgatcccc cccccccccc tccttggact aagtgtaaag cgatgtaaac caactcaaga 720
ataattctga aaccattcag gagatccgga gaggaatcgg gaaatactcc tgcagtgcat 780
ttaaagtaac tgggtcctat gcaacatgag ccattggatc atacaatatt gatatccctt 840
ctaacacgga ggttctaggg tgtctcagct ggaaaagatt cttcagagta gcatgcttgc 900
cttaccccat ccttctcacc ccaccccgag cctcctccag cagaaaggac gagaggccat 960
ctggagcaga gcagagagaa taaatattcc ctttcaaaga aaaaaaa 1007

<210> 29
<211> 120
<212> PRT
<213> Porcine

<400> 29
Met Ala Leu Thr Val Pro Leu Ile Val Leu Ala Val Leu Leu Ser Leu
1               5                   10                  15
Met Glu Ser Pro Ala Ser Ala Pro Val His Arg Gly Arg Gly Gly Trp
            20                  25                  30
Thr Leu Asn Ser Ala Gly Tyr Leu Leu Gly Pro Val Leu His Pro Pro
        35                  40                  45
Ser Arg Ala Glu Gly Gly Gly Lys Gly Lys Thr Ala Leu Gly Ile Leu
    50                  55                  60
Asp Leu Trp Lys Ala Ile Asp Gly Leu Pro Tyr Pro Gln Ser Gln Leu
65                  70                  75                  80
Ala Ser Lys Arg Ser Leu Gly Glu Thr Phe Ala Lys Pro Asp Ser Gly
                85                  90                  95

Val Thr Phe Val Gly Val Pro Asp Val Val Pro Trp Lys Arg Ile Arg
            100                 105                 110
Pro Gly Thr Thr Arg Phe Gln Ile
        115                 120

<210> 30
<211> 96
<212> PRT
<213> Porcine

<400> 30
Met Ala Leu Thr Val Pro Leu Ile Val Leu Ala Val Leu Leu Ser Leu
1               5                   10                  15
Met Glu Ser Pro Ala Ser Ala Pro Val His Arg Gly Arg Gly Gly Trp
            20                  25                  30
Thr Leu Asn Ser Ala Gly Tyr Leu Leu Gly Pro Val Leu His Pro Pro
        35                  40                  45
Ser Arg Ala Glu Gly Gly Gly Lys Gly Lys Thr Ala Leu Gly Ile Leu
    50                  55                  60
Asp Leu Trp Lys Ala Ile Asp Gly Leu Pro Tyr Pro Gln Ser Gln Leu
65                  70                  75                  80
Ala Ser Lys Arg Ser Leu Gly Arg Leu Ser Pro Asn Gln Thr Leu Glu
85 90 95 96

<210> 31
<211> 60
<212> PRT
<213> Porcine

<400> 31

Ala Pro Val His Arg Gly Arg Gly Gly Trp Thr Leu Asn Ser Ala Gly

1               5                   10                  15

Tyr Leu Leu Gly Pro Val Leu His Pro Pro Ser Arg Ala Glu Gly Gly

            20                  25                  30

Gly Lys Gly Lys Thr Ala Leu Gly Ile Leu Asp Leu Trp Lys Ala Ile

        35                  40                  45

Asp Gly Leu Pro Tyr Pro Gln Ser Gln Leu Ala Ser

    50                  55                  60

<210> 32

<211> 180

<212> DNA

<213> Porcine

<400> 32

gctccggtcc acagggggcg aggaggctgg accctcaaca gtgctggtta cctcctgggt    60

cccgtactcc atccgccctc cagggctgaa ggaggcggga aggggaagac agccctcggg   120

atcctggacc tgtggaaggc cattgatggg ctcccctatc cccagtctca gttggcctcc   180

<210> 33

<211> 60

<212> PRT

<213> Rat

<400> 33

Ala Pro Ala His Arg Gly Arg Gly Gly Trp Thr Leu Asn Ser Ala Gly

1               5                   10                  15

Tyr Leu Leu Gly Pro Val Leu His Leu Ser Ser Lys Ala Asn Gln Gly

20                  25                  30

Arg Lys Thr Asp Ser Ala Leu Glu Ile Leu Asp Leu Trp Lys Ala Ile

        35                  40                  45

Asp Gly Leu Pro Tyr Ser Arg Ser Pro Arg Met Thr

    50                  55                  60

<210> 34

<211> 60

<212> PRT

<213> Human

<400> 34

Ala Pro Ala His Arg Gly Arg Gly Gly Trp Thr Leu Asn Ser Ala Gly

1               5                   10                  15

Tyr Leu Leu Gly Pro Val Leu His Leu Pro Gln Met Gly Asp Gln Asp

                20                  25                  30

Gly Lys Arg Glu Thr Ala Leu Glu Ile Leu Asp Leu Trp Lys Ala Ile

        35                  40                  45

Asp Gly Leu Pro Tyr Ser His Pro Pro Gln Pro Ser

    50                  55                  60

<210> 35

<211> 9

<212> PRT

<213> Human, Rat

<400> 35

Ala Pro Ala His Arg Gly Arg Gly Gly

1               5               9

<210> 36

<211> 21

<212> PRT

<213> Human, Rat

<400> 36

Ala Pro Ala His Arg Gly Arg Gly Gly Trp Thr Leu Asn Ser Ala Gly

1               5                   10                  15

Tyr Leu Leu Gly Pro

            20  21

<210> 37

<211> 123

<212> PRT

<213> Rat

<400> 37

Met Pro Cys Phe Ser Ser Ser Arg Met Ala Cys Ser Lys His Leu Val

 1               5                   10                  15

Leu Phe Leu Thr Ile Leu Leu Ser Leu Ala Glu Thr Pro Asp Ser Ala

            20                  25                  30

Pro Ala His Arg Gly Arg Gly Gly Trp Thr Leu Asn Ser Ala Gly Tyr

        35                  40                  45

Leu Leu Gly Pro Val Leu His Leu Ser Ser Lys Ala Asn Gln Gly Arg

    50                  55                  60

Lys Thr Asp Ser Ala Leu Glu Ile Leu Asp Leu Trp Lys Ala Ile Asp

 65                  70                  75                  80

Gly Leu Pro Tyr Ser Arg Ser Pro Arg Met Thr Lys Arg Ser Met Gly

                85                  90                  95
Glu Thr Phe Val Lys Pro Arg Thr Gly Asp Leu Arg Ile Val Asp Lys
            100                 105                 110
Asn Val Pro Asp Glu Glu Ala Thr Leu Asn Leu
        115                 120         123

<210> 38
<211> 116
<212> PRT
<213> Human

<400> 38
Met Ala Pro Pro Ser Val Pro Leu Val Leu Leu Leu Val Leu Leu Leu
1               5                   10                  15
Ser Leu Ala Glu Thr Pro Ala Ser Ala Pro Ala His Arg Gly Arg Gly
            20                  25                  30
Gly Trp Thr Leu Asn Ser Ala Gly Tyr Leu Leu Gly Pro Val Leu His
        35                  40                  45
Leu Pro Gln Met Gly Asp Gln Asp Gly Lys Arg Glu Thr Ala Leu Glu
    50                  55                  60
Ile Leu Asp Leu Trp Lys Ala Ile Asp Gly Leu Pro Tyr Ser His Pro
65                  70                  75                  80
Pro Gln Pro Ser Lys Arg Asn Val Met Glu Thr Phe Ala Lys Pro Glu
                85                  90                  95
Ile Gly Asp Leu Gly Met Leu Ser Met Lys Ile Pro Lys Glu Glu Asp
            100                 105                 110
Val Leu Lys Ser
        115 116

<210> 39

<211> 180

<212> DNA

<213> Rat

<400> 39

gcacctgctc acaggggacg aggaggctgg accctcaata gtgctggtta cctcctgggt 60

cctgtcctcc acctttcctc aaaggccaac cagggcagga agacagactc agctcttgag 120

atcctagacc tgtggaaggc catagatggg ctcccttatt cccgctctcc aaggatgacc 180

<210> 40

<211> 180

<212> DNA

<213> Human

<400> 40

gcacctgccc accggggacg aggaggctgg accctcaata gtgctggcta ccttctgggt 60

cccgtcctcc accttcccca aatgggtgac caagacggaa agagggagac agcccttgag 120

atcctagacc tgtggaaggc catcgatggg ctcccctact cccaccctcc acagccctcc 180

<210> 41

<211> 695

<212> DNA

<213> Rat

<400> 41

aggacaactg ggattacaga tgtgcatccc tgcaaccggc tgccacacaa gttctgggat 60

ctgaactcct ggcctcaaac ttgccagcat tccttagctg tatgccgtgc ttttccagtt 120

ccaggatggc ctgctccaag catctggtcc tcttcctcac catcttgcta agcctcgcag 180

aaacaccaga ctctgcacct gctcacaggg gacgaggagg ctggaccctc aatagtgctg 240
gttacctcct gggtcctgtc ctccaccttt cctcaaaggc caaccagggc aggaagacag 300
actcagctct tgagatccta gacctgtgga aggccataga tgggctccct tattcccgct 360
ctccaaggat gaccaaaagg tcaatgggag aaacgtttgt caagccgagg actggagatc 420
tgcgcatagt ggacaagaat gttccggatg aagaagccac cctgaactta tagagagtta 480
gccctagctc actcctacgt ttccagctca ccgcctctcc ccccaccccc gcccccagaa 540
gcgctctgaa caccctttct aagtctaaga ccttacaact atattcccta atctcactaa 600
gacatgttgt gatatttaaa gagttattct gcccagctcc gaaaaaaaaa aaaaaaaaaa 660
aagagttatt ctgacgtaaa aaaaaaaaaa aaaaa 695

<210> 42
<211> 473
<212> DNA
<213> Human

<400> 42
gaggagccag agagagctgc ggagagctgc cagctgcacc gggcgtgttc cgcagctgta 60
ggcacctgtc gtcctgcctt cgatggctcc tccctccgtc cccctggtcc tcctcctcgt 120
cctcttgctg agcctggcag agactccagc atccgcacct gcccaccggg gacgaggagg 180
ctggaccctc aatagtgctg gctaccttct gggtcccgtc ctccaccttc cccaaatggg 240
tgaccaagac ggaaagaggg agacagccct tgagatccta gacctgtgga aggccatcga 300
tgggctcccc tactcccacc ctccacagcc ctccaagagg aatgtgatgg agacgtttgc 360
caaaccagag attggagatc tgggcatgct cagcatgaaa attcccaagg aggaagatgt 420
cctgaagtca tagatgtctt caaatccctg ttcctatcct tcttctccag ctc 473

<210> 43
<211> 30
<212> PRT
<213> Porcine

<400> 43

Ala Pro Val His Arg Gly Arg Gly Gly Trp Thr Leu Asn Ser Ala Gly

1               5                   10                  15

Tyr Leu Leu Gly Pro Val Leu His Pro Pro Ser Arg Ala Glu

            20                  25                  30

<210> 44

<211> 10

<212> PRT

<213> Porcine

<400> 44

Cys Ala Pro Ala His Arg Gly Arg Gly Gly

1               5                   10

<210> 45

<211> 356

<212> DNA

<213> Rat

<400> 45

atggctctga ctgtccctct gatcgttctt gcagtcctgc tcagcctgat ggagtctcca    60

gcctctgctc cggtccacag ggggcgagga ggctggaccc tcaacagtgc tggttacctc   120

ctgggtcccg tactccatcc gccctccagg gctgaaggag gcgggaaggg gaagacagcc   180

ctcgggatcc tggacctgtg gaaggccatt gatgggctcc cctatcccca gtctcagttg   240

gcctccaaga ggagtctggg ggagactttc gccaaaccag actctggagt aacatttgtt   300

ggagttcctg acgtggtgcc gtggaaacga atccgaccag gaactacgag gtttca       356

<210> 46

<211> 29

<212> DNA

<213> Artificial Sequence

<220>

<223> primer pGAL1-1F

<400> 46

atggctctga ctgtccctct gatcgttct 29

<210> 47

<211> 29

<212> DNA

<213> Artificial Sequence

<220>

<223> primer pGAL88-1R

<400> 47

tgaaacctcg tagttcctgg tcggattcg 29

<210> 48

<211> 28

<212> DNA

<213> Artificial Sequence

<220>

<223> primer F/R120

<400> 48
aggctggacc ctcaatagtg ctggttac                                          28

<210> 49
<211> 28
<212> DNA
<213> Artificial Sequence

<220>
<223> primer R/R120

<400> 49
ccatctatgg ccttccacag gtctagga                                          28

<210> 50
<211> 126
<212> DNA
<213> Human

<400> 50
aggctggacc ctcaatagtg ctggttacct tctgggtccc gtcctccacc ttccccaaat  60
gggtgaccaa gacggaaaga gggagacagc ccttgagatc ctagacctgt ggaaggccat 120
agatgg                                                                 126

<210> 51
<211> 28
<212> DNA
<213> Artificial Sequence

<220>
<223> primer 1F/H120

<400> 51
caaatgggtg accaagacgg aaagaggg 28

<210> 52
<211> 28
<212> DNA
<213> Artificial Sequence

<220>
<223> primer 1R/H120

<400> 52
ggtctaggat ctcaagggct gtctccct 28

<210> 53
<211> 26
<212> DNA
<213> Artificial Sequence

<220>
<223> primer 1F/H470

<400> 53
gaggagccag agagagctgc ggagag 26

<210> 54
<211> 28
<212> DNA
<213> Artificial Sequence

<220>
<223> primer 1R/H470

<400> 54
gagctggaga agaaggatag gaacaggg                                          28

<210> 55
<211> 23
<212> DNA
<213> Artificial Sequence

<220>
<223> primer 1

<400> 55
agcatatggc tccggtccac agg                                               23

<210> 56
<211> 27
<212> DNA
<213> Artificial Sequence

<220>
<223> primer 2

<400> 56
ctggatcctc aggaggccaa ctgagac 27

<210> 57
<211> 21
<212> DNA
<213> Artificial Sequence

<220>
<223> primer 3

<400> 57
cagccctcgg catcctggac c 21

<210> 58
<211> 21
<212> DNA
<213> Artificial Sequence

<220>
<223> primer 4

<400> 58
ggtccaggat gccgagggct g 21

<210> 59
<211> 21
<212> DNA

<213> Artificial Sequence

<220>
<223> synthetic DNA

<400> 59
ctagacatat gccagcattg c                                                21

<210> 60
<211> 21
<212> DNA
<213> Artificial Sequence

<220>
<223> synthetic DNA

<400> 60
tcgggcaatg ctggcatatg t                                                21

<210> 61
<211> 29
<212> PRT
<213> Rat

<400> 61
Gly Trp Thr Leu Asn Ser Ala Gly Tyr Leu Leu Gly Pro His Ala Ile
 1               5                  10                  15

Asp Asn His Arg Ser Phe Ser Asp Lys His Gly Leu Thr

20                    25                    29

<210> 62
<211> 60
<212> PRT
<213> Mouse

<400> 62
Ala Pro Ala His Arg Gly Arg Gly Gly Trp Thr Leu Asn Ser Ala Gly
 1               5                   10                  15
Tyr Leu Leu Gly Pro Val Leu Pro Val Ser Ser Lys Ala Asp Gln Gly
            20                  25                  30
Arg Lys Arg Asp Ser Ala Leu Glu Ile Leu Asp Leu Trp Lys Ile Ile
        35                  40                  45
Asp Gly Leu Pro Tyr Ser His Ser Pro Arg Met Thr
    50                  55                  60

<210> 63
<211> 117
<212> PRT
<213> Mouse

<400> 63
Met Ala Cys Ser Val His Leu Val Leu Phe Leu Thr Ile Leu Leu Ser
 1               5                   10                  15
Leu Ala Glu Thr Pro Glu Ser Ala Pro Ala His Arg Gly Arg Gly Gly
            20                  25                  30
Trp Thr Leu Asn Ser Ala Gly Tyr Leu Leu Gly Pro Val Leu Pro Val
        35                  40                  45

Ser Ser Lys Ala Asp Gln Gly Arg Lys Arg Asp Ser Ala Leu Glu Ile
    50                  55                  60
Leu Asp Leu Trp Lys Ile Ile Asp Gly Leu Pro Tyr Ser His Ser Pro
65                  70                  75                  80
Arg Met Thr Lys Arg Thr Met Gly Glu Thr Phe Val lys Ala Asn Thr
                85                  90                  95
Gly Asp Met His Ile Leu Asp Lys Asn Val Pro Lys Glu Glu Ala Thr
            100                 105                 110
Leu Asp Ser Glu Ser
115 117

<210> 64
<211> 180
<212> DNA
<213> Mouse

<400> 64
gcacctgctc acaggggacg aggaggctgg accctcaata gtgctggtta cctcctgggt 60
cctgtcctcc ccgtttcctc caaggccgac cagggcagga agagagactc agctcttgag 120
atcctagacc tgtggaagat cattgatgga cttccttatt cccactctcc aaggatgacc 180

<210> 65
<211> 28
<212> DNA
<213> Artificial Sequence

<220>
<223> Primer to amplify mouse GALR2 ligand

<400> 65

aggctggacc ctcaatagtg ctggttac     28

<210> 66

<211> 28

<212> DNA

<213> Artificial Sequence

<220>

<223> Primer to amplify mouse GALR2 ligand

<400> 66

ccatctatgg ccttccacag gtctagga     28

<210> 67

<211> 126

<212> DNA

<213> Mouse

<400> 67

aggctggacc ctcaatagtg ctggttacct cctgggtcct gtcctccccg tttcctccaa  60

ggccgaccag ggcaggaaga gagactcagc tcttgagatc ctagacctgt ggaaggccat 120

agatgg                                                            126

<210> 68

<211> 28

<212> DNA

<213> Artificial Sequence

<220>
<223> Primer for 5'RACE to amplify mouse GALR2 ligand

<400> 68
tccaaggccg accagggcag gaagagag     28

<210> 69
<211> 28
<212> DNA
<213> Artificial Sequence

<220>
<223> Primer for 3' RACE to amplify mouse GALR2 ligand

<400> 69
ggtctaggat ctcaagagct gagtctct     28

<210> 70
<211> 28
<212> DNA
<213> Artificial Sequence

<220>
<223> Primer to amplify mouse GALR2 ligand

<400> 70
gaaagacagt ctcaaatcaa ggacctgc     28

<210> 71

<211> 28

<212> DNA

<213> Artificial Sequence

<220>

<223> Primer to amplify mouse GALR2 ligand

<400> 71

tgagctggaa acgaaggagg agtgaggt     28

<210> 72

<211> 351

<212> DNA

<213> Rat

<400> 72

atggcctgct ccgtacatct ggtcctcttc ctcaccatct tgctgagcct ggcagaaaca  60

ccggaatctg cacctgctca caggggacga ggaggctgga ccctcaatag tgctggttac 120

ctcctgggtc ctgtcctccc cgtttcctcc aaggccgacc agggcaggaa gagagactca 180

gctcttgaga tcctagacct gtggaagatc attgatggac ttccttattc ccactctcca 240

aggatgacca aaaggacaat gggagaaacg tttgtcaaag cgaatactgg agatatgcac 300

atactggaca agaatgttcc caaggaagaa gccaccctgg actcagagag t          351

## Description

### TECHNICAL FIELD

The present invention relates to a use of a physiologically active peptide. More specifically, the present invention relates to an agent regulating the luteinizing hormone (LH) secretion and an agent regulating the luteinizing hormone-releasing hormone (LHRH) secretion, which comprise a polypeptide ligand of a G-protein coupled receptor protein (referred to as the receptor) or a salt thereof or a DNA encoding the polypeptide.

### BACKGROUND ART

Many hormones and neurotransmitters regulate biological functions through specific receptors on cell membranes. A number of these receptors are coupled with guanine nucleotide-binding proteins (referred to as G-proteins) and transmit signals into cells through activation of the G-proteins.

Recently, we identified a peptide ligand of Galanin receptor subtype GALR2 (J. Biol. Chem. 272, 24612, 1997; FEBS Letter 471, 225, 1997) from pig hypothalamus (J. Biol. Chem. 274, 37041, 1999). This receptor had been one of so-called orphan G-protein coupled receptors or G-protein coupled receptors whose ligands were unknown. This peptide and Galanin have a common 13-amino acid sequence, and thus the peptide is referred to as Galanin-like peptide (GALP). However, it has been entirely unknown whether GALP has an activity of regulating the LH secretion or LHRH secretion.

As described above, GALP has the 13-amino acid sequence in common with Galanin, but it has been desired to find a new physiological function of GALP, which is different from Galanin function and to develop a useful pharmaceutical.

### SUMMARY OF THE INVENTION

To attain the said object, we intensively studied the change of blood levels of pituitary hormones after administration of GALP or Galanin into rat cerebral ventricles. As the result, it was found that the blood luteinizing hormone (LH) level was increased in the GALP-administered group (5 nmol/rat) and no other pituitary hormone levels were changed. Furthermore, Galanin did not induce an increase in the blood LH level as GALP did. Until now, no peptide factor has been known, which can increase the blood LH level through administration thereof into cerebral ventricles. In addition, in Zucker fatty rat having abnormal Leptin receptor, GALP induced an significant increase in the blood LH level even in the does of 1 nmol/rat, but in Zucker lean rat as a control animal, there was no change in the blood LH level, indicating that Zucker fatty rat has a raised responsiveness to GALP.

A further our study based on these findings led to the present invention.

The invention provides:
(1) An agent regulating the luteinizing hormone (LH) secretion, which comprises GALP or a salt thereof or a DNA encoding GALP.
(2) The agent regulating the LH secretion according to (1), wherein said GALP is a peptide comprising the same or substantially the same amino acid sequence as the sequence shown by SEQ ID NO: 35.
(3) The agent regulating the LH secretion according to (1), wherein said GALP is a peptide comprising the same or substantially the same amino acid sequence as the sequence shown by SEQ ID NO: 31, 33, 34 or 62.
(4) The agent regulating the LH secretion according to (1), wherein said DNA encoding GALP is a DNA comprising a base sequence hybridizable to the base sequence shown by SEQ ID NO: 32, 39, 40 or 64 under high stringent condition.
(5) The agent regulating the LH secretion according to (1), which is an agent enhancing the LH secretion.
(6) The agent regulating the LH secretion according to (1), which is an agent inhibiting the LH secretion.
(7) The agent enhancing the LH secretion according to (5), which is a pharmaceutical agent to prevent and/or treat infertility, menoxenia, dysmenorrhea, amenorrhea, oligomenorrhea, premenstrual syndrome, menopausal disorder, dyspituitarism or obesity.
(8) The agent inhibiting the LH secretion according to (6), which is a pharmaceutical agent to prevent and/or treat prostate cancer, prostate hypertrophy, precocious puberty or LH-producing pituitary gland tumor.
(9) An agent regulating the LH secretion, which comprises an activator of GALP.
(10) An agent regulating the LH secretion, which comprises an inhibitor of GALP.
(11) An agent regulating the luteinizing hormone-releasing hormone (LHRH) secretion, which comprises GALP or a salt thereof or a DNA encoding GALP.
(12) The agent regulating the LHRH secretion according to (11), wherein said GALP is a peptide comprising the same or substantially the same amino acid sequence as the sequence shown by SEQ ID NO: 35.
(13) The agent regulating the LHRH secretion according to (11), wherein said GALP is a peptide comprising the same or substantially the same amino acid sequence as the sequence shown by SEQ ID NO: 31, 33, 34 or 62.
(14) The agent regulating the LHRH secretion according to (11), wherein said DNA encoding GALP is a DNA comprising a base sequence hybridizable to the base sequence shown by SEQ ID NO: 32, 39, 40 or 64 under high stringent condition.
(15) The agent regulating the LHRH secretion according to (11), which is an agent enhancing the LHRH secretion.
(16) The agent regulating the LHRH secretion according to (11), which is an agent inhibiting the LHRH secretion.
(17) The agent enhancing the LHRH secretion according to (15), which is a pharmaceutical agent to prevent and/or treat infertility, menoxenia, dysmenorrhea, amenorrhea, oligomenorrhea, premenstrual syndrome, menopausal disorder, dyspituitarism or obesity.
(18) The agent inhibiting the LHRH secretion according to (16), which is a pharmaceutical agent to prevent and/or treat prostate cancer, prostate hypertrophy, precocious puberty or LHRH-producing pituitary gland tumor.
(19) An agent regulating the LHRH secretion, which comprises an activator of GALP.
(20) An agent regulating the LHRH secretion, which comprises an inhibitor of GALP.
(21) Use of GALP or a salt thereof or a DNA encoding GALP for producing an agent regulating the LH secretion or an agent regulating the LHRH secretion.
(22) A method of preventing or treating disorders of the LH secretion regulation or disorders of the LHRH secretion regulation in a mammal, which comprises administering a pharmacologically effective amount of GALP or a salt thereof or a DNA encoding GALP to said mammal.

### BRIEF DESCRIPTION OF THE DRAWINGS

Fig. 1 shows the detection of galanin receptor-activating effect by [³⁵S]GTPγS binding assay.
Fig. 2 shows the analysis of the sample fractions obtained in Reference Example 2 (2-3) by [³⁵S]GTPγS binding assay using GALR2-expressing cell membrane fractions.
Fig. 3 shows the analysis of the sample fractions obtained in Reference Example 2 (2-3) by Pig Galanin Radioimmunoasssay Kit (Peninsula).
Fig. 4 shows the molecular weight analysis by gel filtration high performance liquid chromatography of the component having GALR2-activating effect ([³⁵S]GTPγS binding promotion activity) obtained in Reference Example 2 (2-4).
Fig. 5 shows the molecular weight analysis of known peptides by gel filtration high performance liquid chromatography as performed in Reference Example 2 (2-4).

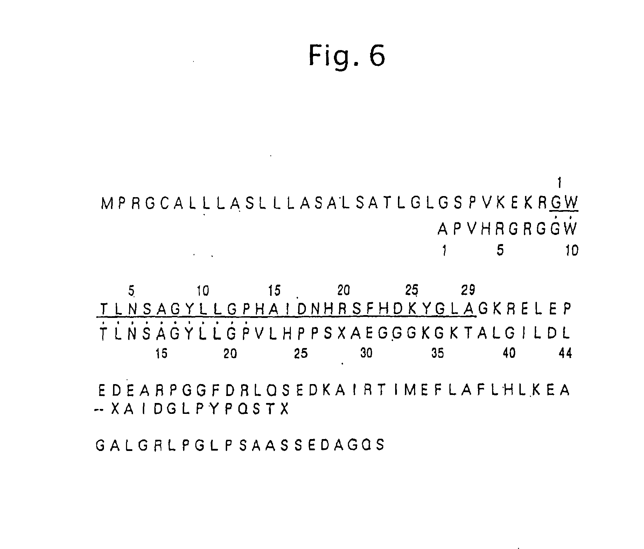

Fig. 6 shows the amino acid sequence comparison of the peptide of the invention to a pig galanin precursor. The amino acid sequence of the pig galanin precursor is described in the upper line and the amino acid sequence of the peptide of the invention in the lower line.
Fig. 7 shows the analysis of antibody titers using HRP-labeled Ala-Pro-Ala-His-Arg-Gly-Arg-Gly-Gly-Cys-NH₂ in Ala-Pro-Ala-His-Arg-Gly-Arg-Gly-Gly-Cys(-NH₂)-KLH complex-immunized mice.
Fig. 8 shows a typical example of the hybridoma screening after cell fusion when Ala-Pro-Ala-His-Arg-Gly-Arg-Gly-Gly-Cys(-NH₂)-KLH complex-immunized mice were used.
Fig. 9 shows the analysis of the reactivity of monoclonal antibody GR2-1N (prepared using Ala-Pro-Ala-His-Arg-Gly-Arg-Gly-Gly-Cys(-NH₂)-KLH complex as an immunogen) against Ala-Pro-Ala-His-Arg-Gly-Arg-Gly-Gly-Cys-NH₂ (an amide form of SEQ ID NO: 44), pig-type ligand peptide (1-60) (SEQ ID NO: 31) and rat galanin (SEQ ID NO: 61) by competitive EIA using HRP-labeled Ala-Pro-Ala-His-Arg-Gly-Arg-Gly-Gly-Cys-NH₂.
Fig. 10 shows the method of preparation of the structural gene for pig-type ligand peptide described in Reference Example 20.
Fig. 11 shows the construction of the fusion protein expression vector described in Reference Example 21.
Fig. 12 shows the construction of the pig-type ligand peptide-expressing strain described in Reference Example 21.
Fig. 13 shows the results of analysis by SDS-polyacrylamide gel electrophoresis described in Reference Example 23 (electrophoregram).
Fig. 14 shows the results of the feeding test performed in Reference Example 24.
Fig. 15 shows changes in blood levels of LH (a), FSH (b), GH (c), TSH (d), ACTH (e) and prolactin (f) 10 min after the administration of rat GALP into the third ventricle of rats. Each level is expressed as measured value±standard error. The symbol ** indicates that there is a significant difference at the significance level of 1% (n=6-7).
Fig. 16 shows changes in blood LH levels when rat GALP and galanin were administered into rat ventricles. In this Figure, -Δ- represents the control; -○- represents the results of rat GALP 1 nmol administration; -●- represents the results of rat GALP 5 nmol administration; and -*- represents the results of rat galanin 5 nmol administration. LH level is expressed as measured value±standard error. The symbol ** indicates that there is a significant difference at the significance level of 1 % (n=5).

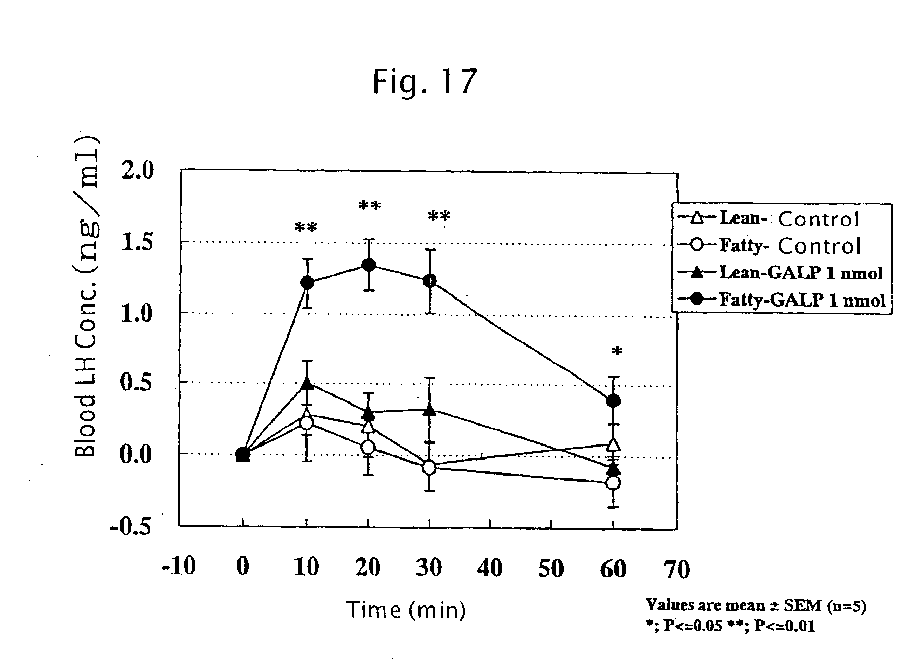

Fig. 17 shows changes in blood LH levels when rat GALP was administered into the ventricles of Zucker fatty rats and Zucker lean rats. In this Figure, -Δ-represents the control in Zucker lean rats; -○- represents the control in Zucker fatty rats; -▲- represents the results of intraventricular administration of rat GALP (1 nmol) to Zucker lean rats; and -●- represents the results of intraventricular administration of rat GALP (1 nmol) to Zucker fatty rats. The symbol ** indicates that there is a significant difference at the significance level of 1 % (n=5). The symbol * indicates that there is a significant difference at the significance level of 5% (n=5).
Fig. 18 compares the amino acid sequences of pig intestine galanin (galanin), pig-type GalR2 ligand (P-GALR2L), rat-type GalR2 ligand (R-GALR2L), human-type GalR2 ligand (H-GALR2L) and the mouse-type GalR2 ligand (M-GALR2L) of the invention.

### BEST MODE FOR CARRYING OUT THE INVENTION

In this specification, the term "substantially the same" is used to express that activities of proteins in question, such as the activity (effect) of regulating the LH secretion, the activity (effect) of regulating the LHRH secretion, the agonist activity to the receptor, i.e. the ligand activity of activating the receptor, the activity of ligand-binding to the receptor are substantially the same as each other.

In some cases, the amino acid modification of a peptide such as substitution, deletion, insertion or addition may not significantly change the physiological or chemical properties of the peptide. In these cases, the peptide having the substitution, deletion, insertion or addition may be considered substantially identical to the peptide having no substitution, deletion, insertion or addition. For example, an amino acid contained in the amino acid sequence may be substituted by substantially the same amino acid which can be selected from the amino acid group that the amino acid to be substituted belongs to. Non-polar (hydrophobic) amino acids include alanine, leucine, isoleucine, valine, proline, phenylalanine, tryptophan, and methionine. Polar (neutral) amino acids include glycine, serine, threonin, cycteine, thyrosine, asparagine, and glutamine. Positively charged (basic) amino acids include arginine, lysine, and histidine. Negatively charged (acidic) amino acids include aspartic acid and glutamic acid.

GALP (which may be referred to as "the polypeptide of the invention"; and "GALP or a salt thereof' may also be referred to as "the polypeptide of the invention") is a peptide capable of being bound to Galanin receptor. It is preferred that GALP is capable of activating Galanin receptor and is a ligand peptide of the receptor, excluding known Galanins. The details of Galanin receptor are described below.

The polypeptide of the invention includes a peptide comprising the same or substantially the same amino acid sequence as the sequence shown by SEQ ID NO: 35 (e.g. the amino acid sequence shown by SEQ ID NO: 13) and having the activity (effect) of regulating the LH secretion or the LHRH secretion, and specifically:
(1) a peptide comprising the same or substantially the same amino acid sequence as the sequence shown by SEQ ID NO: 35 (e.g. the amino acid sequence shown by SEQ ID NO: 13) and comprising the same or substantially the same amino acid sequence as the sequence shown by SEQ ID NO: 11, 12, 15, 16 or 43, and having the activity (effect) of regulating the LH secretion or the LHRH secretion;
(2) the peptide according to (1), which has a molecular weight of 5000 to 10000;
(3) the peptide according to (1), which has a molecular weight of 5000 to 8000;
(4) a peptide having the same or substantially the same amino acid sequence as the sequence shown by SEQ ID NO: 17, and having the activity (effect) of regulating the LH secretion or the LHRH secretion, and having a molecular weight of 5000 to 10000;
(5) a peptide having the same or substantially the same amino acid sequence as the sequence shown by SEQ ID NO: 31, 33, 34 or 62 and having the activity (effect) of regulating the LH secretion or the LHRH secretion; and furthermore,
(6) a peptide comprising the same or substantially the same amino acid sequence as the sequence shown by SEQ ID NO: 36;
(7) the peptide according to (6), which comprises the same or substantially the same amino acid sequence as the sequence shown by SEQ ID NO: 11, 12, 15, 16 or 43;
(8) the peptide according to (7), which has a molecular weight of 5000 to 10000;
(9) the peptide according to (7), which has a molecular weight of 5000 to 8000;
(10) a peptide having the same or substantially the same amino acid sequence as the sequence shown by SEQ ID NO: 31, 33, 34 or 62;
(11) a peptide having the same or substantially the same amino acid sequence as the sequence shown by SEQ ID NO: 17;
(12) a peptide comprising the same or substantially the same amino acid sequence as the sequence shown by SEQ ID NO: 35 (e.g. the amino acid sequence shown by SEQ ID NO: 13) and comprising the same or substantially the same amino acid sequence as the sequence shown by SEQ ID NO: 11, 12, 15, 16 or 43,
(13) the peptide according to (10), (11) or (12), which has a molecular weight of 5000 to 10000.

The polypeptide of the present invention, the method of producing the polypeptide, and the utility of the polypeptide are described in detail below.

The afore-mentioned peptides of the present invention may be derived from any sources without any limitation, for example, from any tissues (e.g. pituitary, pancreas, brain, kidney, liver, genital gland, thyroid, gallbladder, bone marrow, adrenal gland, skin, skeletal muscle, lung, gastrointestinal tract, blood vessel, heart, testis) or any cells of warm-blooded animals (e.g. human, guinea pig, rat, mouse, pig, sheep, bovine, monkey)(preferred are those from, e.g. mouse brain, rat brain, pig brain, bovine brain, pig hypothalamus, bovine hypothalamus, pig lung, bovine lung, bovine stomach, human hypothalamus, pig testis, bovine testis, rat testis, mouse testis, human testis or human lung); or the peptides may be synthesized.

The polypeptide of the invention may be (1) peptides comprising the amino acid sequence shown by SEQ ID NO: 17, 31, 33, 34 or 62 (preferably SEQ ID NO: 31, 33, 34 or 62), and also peptides having an activity substantially identical in property to those of peptides comprising amino acid sequences having about 50 to 99.9 % (preferably 70 to 99.9 %, more preferably 80 to 99.9 %, even more preferably 90 to 99.9 %, most preferably 95 to 99.9 %) homology to the amino acid sequence shown by SEQ ID NO: 17, 31, 33, 34 or 62 (preferably SEQ ID NO: 31, 33, 34 or 62)(excluding Galanin or a precursor thereof defined by SEQ ID NO: 7, 8, 9 or 10).

The "activity substantially identical in property" refers to identicalness in property of, for example, the activity (effect) of regulating the LH secretion, the activity of binding to Galanin receptor, GALR1, GALR2 or GALR3, the activity of activating Galanin receptor, GALR1, GALR2 or GALR3, or activating subsequent signal transduction systems, such as arachidonic acid release, intracellular Ca²⁺ release, intracellular cAMP production inhibition, inositol phosphate production (inhibition), cell membrane potential change, intracellular protein phosphorylation, intracellular pH change. Therefore, quantitative factors such as activity levels and molecular weights of the peptides may be different. These activities can be measured according to a known method or a modified method thereof, for example, the methods described in Reference Example 1 or 17, or Example 1 to 3.

Examples of the polypeptide of the invention include the following (excluding Galanin or a precursor thereof having the amino acid sequence shown by SEQ ID NO: 7, 8, 9 or 10).
(I) A peptide derived from mouse brain, rat brain, pig brain, bovine brain, pig hypothalamus, bovine hypothalamus, pig lung, bovine lung, bovine stomach, human hypothalamus, pig testis, bovine testis, rat testis, mouse testis, human testis or human lung, comprising the same or substantially the same amino acid sequence as the sequence shown by SEQ ID NO: 17, 31, 33, 34 or 62 (preferably SEQ ID NO: 31, 33, 34 or 62) or a partial sequence thereof.
(II) A peptide or a partial peptide thereof, comprising the same or substantially the same amino acid sequence as the sequence shown by SEQ ID NO: 17, 31, 33, 34 or 62 (preferably SEQ ID NO: 31, 33, 34 or 62) or a partial sequence thereof, wherein one or more amino acids are substituted, deleted or added in the said peptide or the partial peptide thereof, is included in the peptide comprising "substantially the same amino acid sequence".

For example, contemplated is a peptide comprising (1) the amino acid sequence shown by SEQ ID NO: 17, 31, 33, 34 or 62 (preferably SEQ ID NO: 31, 33, 34 or 62) or a partial sequence thereof, from which 1 to 7, preferably 1 to 5, more preferably 1 to 3 amino acids are deleted; (2) the amino acid sequence shown by SEQ ID NO: 17, 31, 33, 34 or 62 (preferably SEQ ID NO: 31, 33, 34 or 62) or a partial sequence thereof, to which 1 to 20, preferably 1 to 15, more preferably 1 to 10 amino acids are added (or inserted); (3) the amino acid sequence shown by SEQ ID NO: 17, 31, 33, 34 or 62 (preferably SEQ ID NO: 31, 33, 34 or 62) or a partial sequence thereof, in which 1 to 7, preferably 1 to 5, more preferably 1 to 3 amino acids are substituted with other amino acids.

The term "substantially the same amino acid sequence" refers to an amino acid sequence having 70 to 99.9 %, more preferably 80 to 99.9 %, even more preferably 90 to 99.9 %, most preferably 95 to 99.9 % homology to the reference amino acid sequence.

The polypeptide of the invention has a molecular weight of about 5000 to about 10000 daltons, preferably about 5000 to about 8000 daltons, more preferably about 5500 to about 8000 daltons, even more preferably about 6000 to about 7000.

In the present specification, peptides are shown in a conventional way with placing the N-terminal (amino terminal) on the left side and the C-terminal (carboxyl terminal) on the right side.

The polypeptide of the invention usually has the C-terminal in a form of carboxyl group (-COOH) or carboxylate (-COO⁻). An amide form (-CONH₂) or an ester form (-COOR) is also possible.

Examples of the ester group R include C₁₋₆ alkyl such as methyl, ethyl, n-propyl, isopropyl, n-butyl; C₃₋₈ cycloalkyl such as cyclopentyl, cyclohexyl; C₆₋₁₂ aryl such as phenyl, α-naphthyl; C₇₋₁₄ aralkyl such as phenyl-C₁₋₂ alkyl, e.g. benzyl, phenethyl, and α-naphthyl-C₁₋₂ alkyl, e.g. α-naphthylmethyl; and pivaloyloxymethyl, which is used widely for an oral ester form.

When the polypeptide of the invention contains a carboxyl group or a carboxylate at a position other than the C-terminal, it may be amidated or esterified. Such an amide or ester is also included in the polypeptide of the invention. The ester group may be the same as the above-mentioned C-terminal ester group.

The salt of the polypeptide of the invention includes salts with physiologically acceptable bases (e.g. alkaline metals) or acids (e.g. inorganic acids, organic acids). Physiologically acceptable acid addition salts are particularly preferred. Examples of such salts are salts with inorganic acids (e.g. hydrochloric acid, phosphoric acid, hydrobromic acid, sulfuric acid), and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The polypeptide of the invention can be purified from human or other warm-blooded animal tissues or cells according to a known peptide purification method, or can be produced according to the peptide synthesis method described below.

Alternatively, the polypeptide of the invention can be produced by culturing a transformant comprising the DNA encoding the polypeptide. The DNA encoding the polypeptide can be prepared according to a known cloning method (for example, the method described in Molecular Cloning, 2nd Ed., J. Sambrook et al., Cold Spring Harbor Lab. Press, 1989).

In the cloning method, (1) a transformant comprising the DNA encoding the peptide can be obtained by hybridization using a DNA probe or a DNA primer designed on the basis of the amino acid sequence of the peptide; or (2) a transformant comprising the DNA encoding the peptide can be obtained by PCR using DNA primers designed on the basis of the amino acid sequence of the peptide.

When producing the polypeptide of the invention from human or other warm-blooded animal tissues or cells, these tissues or cells are homogenized and extracted with acids or alcohols, and then the polypeptide can be isolated or purified from the obtained extract through a combination of salting-out, dialysis, gel filtration, and chromatography techniques such as reversed phase chromatography, ion exchange chromatography, affinity chromatography and the like.

As described above, the polypeptide of the invention can be produced by (1) a known peptide synthesis method, or (2) cleaving a peptide comprising the polypeptide of the invention with an appropriate peptidase.

For the peptide synthesis, either solid phase synthesis or liquid phase synthesis may be used. Thus, a partial peptide or amino acids that can construct the polypeptide of the invention can be condensed with the remaining part of the polypeptide. When the product contains protecting groups, these protecting groups are removed to give the desired peptide. Known methods for condensation and elimination of the protecting groups are described in the following 1) to 2):
1) M. Bodanszky & M.A. Ondetti: Peptide Synthesis, Interscience Publishers, New York (1966);
2) Schroeder & Luebke: The Peptide, Academic Press, New York (1965).

After the reaction, the polypeptide of the invention can be purified or isolated by a combination of conventional purification methods such as solvent extraction, distillation, column chromatography, liquid chromatography and recrystallization. When the peptide obtained by the above methods is in a free form, it can be converted into an appropriate salt by a known method; on the other hand, when the peptide is obtained in a salt form, it can be converted into a free form by a known method.

To synthesize an amide form of the polypeptide of the invention, commercially available resins for peptide synthesis, suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin. Using these resins, amino acids in which α-amino groups and functional groups on the side chains are appropriately protected are condensed on the resin in the order of the sequence of the desired peptide according to various known condensation methods. At the end of the reaction, the peptide is excised from the resin and at the same time, the protecting groups are removed. Then, intramolecular disulfide bond-forming reaction is performed in a highly diluted solution to obtain the desired peptide.

For condensation of the protected amino acids described above, a variety of activation reagents for peptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide. For activation by these reagents, the protected amino acids in combination with a racemization inhibitor (e.g. HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents used to activate the protected amino acids or condense with the resin may be chosen from solvents that are known to be usable for peptide condensation reactions. Acid amides such as N,N-dimethylformamide, N,N-dimethylacetamide, N-methylpyrrolidone; halogenated hydrocarbons such as methylene chloride, chloroform; alcohols such as trifluoroethanol; sulfoxides such as dimethylsulfoxide; tertiary amines such as pyridine; ethers such as dioxane, tetrahydrofuran; nitriles such as acetonitrile, propionitrile; esters such as methyl acetate, ethyl acetate; and appropriate mixtures of these solvents are usable. The reaction temperature is appropriately chosen from the range known to be applicable to peptide bond formation and is usually selected in the range of about -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of groups for protecting an amino group in the starting material include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc.

Examples of groups for protecting a carboxyl group include C₁₋₆-alkyl, C₃₋₈-cycloalkyl, C₇₋₁₄-aralkyl as described above as R; and 2-adamantyl, 4-nitrobenzyl, 4-methoxybenzyl, 4-chlorobenzyl, phenacyl; and benzyloxycarbonyl hydrazide, t-butoxycarbonyl hydrazide, trityl hydrazide.

The hydroxyl group of serine and threonine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group.

Examples of groups for protecting the phenolic hydroxyl group of tyrosine include Bzl, Cl₂-Bzl, 2-nitrobenzyl, Br-Z, t-butyl.

Examples of groups for protecting the imidazole moiety of histidine include Tos, 4-methoxy-2,3,6-trimethylbenzenesulfonyl, DNP, benzyloxymethyl, Bum, Boc, Trt, Fmoc.

Examples of the activated carboxyl group in the starting material include the corresponding acid anhydrides, azides, activated esters [esters with alcohols (e.g. pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. Examples of the activated amino group in the starting material include the corresponding phosphoric amide.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of about -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group used for protecting the imidazole of histidine is eliminated by treatment with thiophenol. Formyl group used for protecting the indole of tryptophan is eliminated by the aforesaid acid treatment in the presence of 1,2-ethanedithiol or 1,4-butanedithiol, or by alkali treatment with a dilute sodium hydroxide solution and dilute ammonia.

Protection of functional groups in the starting material, which should not be involved in the reaction, protecting groups, elimination of the protecting groups and activation of functional groups involved in the reaction may be publicly known.

In another method for producing an amide of the polypeptide, the α-carboxyl group of the carboxy terminal amino acid is first amidated, and the peptide chain is then extended to amino group side for a desired length. Thereafter, the peptide in which only the protecting group of the N-terminal α-amino group is eliminated, and the peptide in which only the protecting group of the C-terminal carboxyl group is eliminated are produced. Both the peptides are condensed in a mixture of the solvents described above. The details of the condensation reaction are the same as described above. After the protected peptide obtained by the condensation is purified, all the protecting groups are eliminated by the method described above to give the desired crude peptide. This crude peptide is purified by various known purification means. Lyophilization of the major fraction gives the desired amidated peptide.

To prepare an ester of the polypeptide of the invention, the α-carboxyl group of the carboxy terminal amino acid can be condensed with a desired alcohol to prepare the amino acid ester, which is then processed in a similar way to the preparation of the amidated peptide to give the desired esterified peptide.

The polypeptide of the invention may be any peptide having a function identical to those of peptides comprising the same or substantially the same amino acid sequence as the sequence shown by SEQ ID NO: 17, 31, 33, 34 or 62 (preferably SEQ ID NO: 31, 33, 34 or 62) or a partial sequence thereof (e.g. the activity (effect) of regulating the LH secretion, the activity of GALR1 agonist, GALR2 agonist or GALR3 agonist). These activities (actions) can be measured according to a known method or a modified method thereof, for example, the method described in Reference Example 1 or 17, or Example 1 to 3.

Such a peptide includes a peptide having the same or substantially the same amino acid sequence as the sequence shown by SEQ ID NO: 17, 31, 33, 34 or 62 (preferably SEQ ID NO: 31, 33, 34 or 62) or a partial sequence thereof, wherein 1 to 5 amino acids are deleted, added (inserted) or substituted in the said sequence or the partial sequence thereof.

The precursor of the polypeptide of the invention refers to any peptide coding for the ligand peptide of the invention as a partial peptide (excluding Galanin or a precursor thereof having the amino acid sequence shown by SEQ ID NO: 7, 8, 9 or 10).

Specific examples of the precursor of the polypeptide of the invention include peptides (or polypeptides) comprising the same or substantially the same amino acid sequence as the sequence shown by SEQ ID NO: 29.

The term "substantially the same amino acid sequence" refers to an amino acid sequence having 70 to 99.9 %, more preferably 80 to 99.9 %, even more preferably 90 to 99.9 %, most preferably 95 to 99.9 % homology to the amino acid sequence shown by SEQ ID NO: 29.

Specifically, "substantially the same amino acid sequence" includes (1) the amino acid sequence shown by SEQ ID NO: 29, in which 1 to 7, preferably 1 to 5, more preferably 1 to 3 amino acids are substituted with other amino acids; (2) the amino acid sequence shown by SEQ ID NO: 29, from which 1 to 7, preferably 1 to 5, more preferably 1 to 3 amino acids are deleted; (3) the amino acid sequence shown by SEQ ID NO: 29, to which 1 to 7, preferably 1 to 5, more preferably 1 to 3 amino acids are added (or inserted). Such substitution, deletion or addition (insertion) can be carried out without special limitation at any positions outside of the region coding for the ligand peptide of the invention as a partial peptide.

Specific examples of peptides (or polypeptides) comprising the same or substantially the same amino acid sequence as the sequence shown by SEQ ID NO: 29 include:
(1) peptides (or polypeptides) comprising the same or substantially the same amino acid sequence as the sequence shown by SEQ ID NO: 30;
(2) peptides (or polypeptides) comprising the same or substantially the same amino acid sequence as the sequence shown by SEQ ID NO: 37; or
(3) peptides (or polypeptides) comprising the same or substantially the same amino acid sequence as the sequence shown by SEQ ID NO: 38.

The partial peptide of the invention may contain a single domain among respective domains of the polypeptide of the invention, or a number of these domains together.

In addition, since the partial peptide can be used as an antigen for preparing an anti-ligand peptide antibody, the partial peptide may be (i) a partial peptide of the polypeptide of the invention, such as its N-terminal peptide, C-terminal peptide or a peptide localized between these terminal peptides, to which any one to several (preferably 1 to 3, more preferably 1 or 2) amino acids are added (or inserted); (ii) a partial peptide of the polypeptide of the invention, such as its N-terminal peptide, C-terminal peptide or a peptide localized between these terminal peptides, from which one to several (preferably 1 to 3, more preferably 1 or 2) amino acids are deleted; (iii) a partial peptide of the polypeptide of the invention, such as its N-terminal peptide, C-terminal peptide or a peptide localized between these terminal peptides, in which one to several (preferably 1 to 3, more preferably 1 or 2) constitutional amino acids are substituted with other amino acids.

More specifically, the partial peptide includes:
(i) the peptide consisting of the amino acid sequence of Ala-Pro-Ala-His-Arg-Gly-Arg-Gly-Gly-Cys-NH₂ (the peptide consisting of the amino acid sequence shown by SEQ ID NO: 44 with the C-terminal amidated), which is used in Reference Example 9; as well as
(ii) the peptide consisting of the amino acid sequence shown by SEQ ID NO: 11;
(iii) the peptide consisting of the amino acid sequence shown by SEQ ID NO: 12;
(iv) the peptide consisting of the amino acid sequence shown by SEQ ID NO: 13;
(v) the peptide consisting of the amino acid sequence shown by SEQ ID NO: 14;
(vi) the peptide consisting of the amino acid sequence shown by SEQ ID NO: 15;
(vii) the peptide consisting of the amino acid sequence shown by SEQ ID NO: 16;
(viii) the peptide consisting of the amino acid sequence shown by SEQ ID NO: 17;
(ix) the peptide consisting of the amino acid sequence shown by SEQ ID NO: 35;
(x) the peptide consisting of the amino acid sequence shown by SEQ ID NO: 36; and
(xi) the peptide consisting of the amino acid sequence shown by SEQ ID NO: 43.

The C-terminal of the partial peptide in the present specification may be an amide (-CONH₂) or ester (-COOR), wherein the ester group includes the same examples as described above in the esterified peptide. When the partial peptide contains a carboxyl group or a carboxylate at a position other than the C-terminal, it may be amidated or esterified and such an amide or ester is also included within the partial peptide of the invention. The ester group to be used in this case may be the same as the above-mentioned C-terminal ester group.

The partial peptide of the polypeptide of the invention may have the same activity as that of the polypeptide of the invention (e.g. the activity (effect) of regulating the LH secretion, of regulating the LHRH secretion, of activating Galanin receptor).

Furthermore, the polypeptide of the invention or the partial peptide thereof may constitute a fusion protein with a protein having a well-known function or property.

An example of such a fusion protein is one consisting of a peptide comprising the amino acid sequence of Ala-Pro-Ala-His-Arg-Gly-Arg-Gly-Gly-Cys-NH₂ (the amino acid sequence shown by SEQ ID NO: 44 with the C-terminal amidated), which is used in Reference Example 11, and Keyhole Limpet Hemocyanin (KLH).

The partial peptide of the polypeptide of the invention may take a salt form like the aforementioned salts of the peptide.

The partial peptide of the polypeptide of the invention, an amide, an ester or a salt thereof can be produced according to the peptide synthesis method as described above, or by cleaving the polypeptide of the invention with an appropriate peptidase.

The DNA encoding the polypeptide of the invention may be any DNA comprising a nucleotide sequence encoding (I) a peptide comprising the same or substantially the same amino acid sequence as the sequence shown by SEQ ID NO: 35 (e.g. the amino acid sequence shown by SEQ ID NO: 13) and having the activity (effect) of regulating the LH secretion; or (II) a peptide comprising the same or substantially the same amino acid sequence as the sequence shown by SEQ ID NO: 36.

Further included is any DNA comprising a nucleotide sequence encoding the aforementioned polypeptide of the invention.

In addition, such a DNA may be derived from a genome DNA, a genome DNA library, cDNAs derived from the aforementioned tissues and cells, a cDNA library derived from the aforementioned tissues and cells, or synthetic DNAs. Vectors used for the libraries may be any one of bacteriophage, plasmid, cosmid, phagemid, and the like. In addition, the DNA can be amplified directly by reverse transcriptase polymerase chain reaction (hereinafter abbreviated as RT-PCR) from the RNA fraction prepared from the above-mentioned tissues or cells.

More specifically, used are (1) a DNA comprising a nucleotide sequence encoding a peptide comprising the same or substantially the same amino acid sequence as the sequence shown by SEQ ID NO: 31, 33, 34 or 62; (2) a DNA hybridizable to the nucleotide sequence defined in (1) under high stringent conditions; (3) a DNA not hybridizable to the nucleotide sequence defined in (1) or (2) due to the degeneracy of the genetic code, but encoding a polypeptide having the same amino acid sequence as that defined in (1) and (2).

The hybridization can be carried out according to a known method or a modification thereof. The high stringent condition refers to, for example, a temperature of 42°C, 50% formaldehyde, 4x SSPE (1x SSPE: 150mM NaCl, 10mM NaH₂PO₄·H₂O, 1mM EDTA pH7.4), 5x Denhardt's solution, and 0.1% SDS.

For example, a DNA comprising a nucleotide sequence encoding a peptide comprising the same or substantially the same amino acid sequence as the sequence shown by SEQ ID NO: 31 may be a DNA comprising the nucleotide sequence shown by SEQ ID NO: 32; a DNA comprising a nucleotide sequence encoding a peptide comprising the same or substantially the same amino acid sequence as the sequence shown by SEQ ID NO: 33 may be a DNA comprising the nucleotide sequence shown by SEQ ID NO: 39; a DNA comprising a nucleotide sequence encoding a peptide comprising the same or substantially the same amino acid sequence as the sequence shown by SEQ ID NO: 34 may be a DNA comprising the nucleotide sequence shown by SEQ ID NO: 40; a DNA comprising a nucleotide sequence encoding a peptide comprising the same or substantially the same amino acid sequence as the sequence shown by SEQ ID NO: 62 may be a DNA comprising the nucleotide sequence shown by SEQ ID NO: 64.

The DNA encoding the precursor of the polypeptide of the invention may be any DNA comprising a nucleotide sequence encoding the aforementioned precursor of the polypeptide of the invention, for example, a peptide (polypeptide) comprising the same or substantially the same amino acid sequence as the sequence shown by SEQ ID NO: 29 (e.g. the amino acid sequence shown by SEQ ID NO: 30, 37 or 38).

For example, a DNA comprising a nucleotide sequence encoding a peptide (polypeptide) comprising the same or substantially the same amino acid sequence as the sequence shown by SEQ ID NO: 29 may be a DNA comprising the nucleotide sequence shown by SEQ ID NO: 27; a DNA comprising a nucleotide sequence encoding a peptide comprising the same or substantially the same amino acid sequence as the sequence shown by SEQ ID NO: 30 may be a DNA comprising the nucleotide sequence shown by SEQ ID NO: 28; a DNA comprising a nucleotide sequence encoding a peptide comprising the same or substantially the same amino acid sequence as the sequence shown by SEQ ID NO: 37 may be a DNA comprising the nucleotide sequence shown by SEQ ID NO: 41; a DNA comprising a nucleotide sequence encoding a peptide comprising the same or substantially the same amino acid sequence as the sequence shown by SEQ ID NO: 38 may be a DNA comprising the nucleotide sequence shown by SEQ ID NO: 42.

The DNA encoding the polypeptide of the invention (hereinafter, the polypeptide of the invention, the precursor thereof, or the partial peptide thereof may be simply referred to as "the polypeptide of the invention") can be produced by genetic engineering techniques as shown below. The genetic engineering techniques are described in, for example, "Molecular Cloning" (2nd Ed., J. Sambrook et al., Cold Spring Harbor Lab. Press, 1989). Commercially available libraries and kits may be used according to their attached manufacturer's instructions.

The DNA encoding the polypeptide of the invention can be selected by the hybridization of genome DNAs or a cDNA library with a labeled form of a DNA fragment as a probe, which is prepared on the basis of the amino acid sequence of the peptide or a part thereof.

For cloning of the DNA completely encoding the polypeptide of the invention, the desired DNA may be amplified from genome DNAs or a cDNA library by the known PCR method using synthetic DNA primers having a part of the nucleotide sequence encoding the polypeptide of the invention. Alternatively, the DNA incorporated into an appropriate vector may be selected by hybridization with a labeled form of a DNA fragment or synthetic DNA encoding a part or the entire region of the polypeptide of the invention.

The cloned DNA encoding the polypeptide of the invention can be used as it is for any purpose or if desired, after digestion with a restriction enzyme or after addition of a linker thereto. The DNA may contain ATG as a translation initiation codon at the 5' end and TAA, TGA or TAG as a translation termination codon at the 3' end. These translation initiation and termination codons may also be added by using an appropriate synthetic DNA adapter.

The expression vector for the polypeptide of the invention can be produced, for example, by (a) excising the desired DNA fragment from a DNA (e.g. cDNA) comprising the DNA encoding the polypeptide of the invention, (b) and then inserting the DNA fragment downstream of a promoter in an appropriate vector.

Examples of the vector to be used include plasmids derived form E. coli (e.g. pBR322, pBR325, pUC12, pUC13), plasmids derived from Bacillus subtilis (e.g. pUB110, pTP5, pC194), plasmids derived from yeast (e.g. pSH19, pSH15), bacteriophages such as λ phage, animal viruses such as retrovirus, vaccinia virus, baculovirus.

The promoter to be used in the invention may be any suitable one working in a host to be used for the gene expression.

When animal cells are used as a host to be transformed, SV40 promoter, retrovirus promoter, metallothionein promoter, heat shock promoter, cytomegalovirus promoter, SRα promoter, and the like can be used. When Escherichia bacteria are used as the host, preferred are trp promoter, T7 promoter, 1ac promoter, recA promoter, λPL promoter, lpp promoter; when Bacillus bacteria are used as the host, preferred are SPO1 promoter, SPO2 promoter, penP promoter; when yeasts are used as the host, preferred are PHO5 promoter, PGK promoter, GAP promoter, ADH1 promoter, GAL promoter; when insect cells are used as the host, preferred are polyhedrin promoter, and P10 promoter.

In addition to the foregoing, the expression vector may further optionally contain an enhancer, a splicing signal, a poly A signal, a selection marker, SV40 replication origin (hereinafter abbreviated as SV40ori), and the like. Examples of the selection marker include dihydrofolate reductase (hereinafter abbreviated as dhfr) gene [methotrexate (MTX) resistance], ampicillin resistant gene (hereinafter abbreviated as Amp^{r}), and neomycin resistant gene (hereinafter abbreviated as Neo^{r}, G418 resistance). In particular, when dhfr gene is used as the selection marker in CHO(dhfr⁻) cells, the selection may be made in thymidine-free medium.

If necessary, a signal sequence suitable for a host may be added to the N-terminal of the peptide or the partial peptide thereof. For example, Pho A signal sequence, OmpA signal sequence in Escherichia bacteria as the host; α-amylase signal sequence, subtilisin signal sequence in Bacillus bacteria as the host; Mating Factor α (MFα) signal sequence, invertase signal sequence in yeasts as the host; and insulin signal sequence, α-interferon signal sequence, antibody molecule signal sequence in animal cells as the host can be used respectively.

Using the vector comprising the DNA encoding the peptide as constructed in this way, a transformant can be produced.

For example, Escherichia bacteria, Bacillus bacteria, yeasts, insects, insect cells and animal cells can be used as the host.

Examples of Escherichia bacteria include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)).

Examples of Bacillus bacteria include Bacillus subtilis MI114 (Gene, 24, 255 (1983)), 207-21 (Journal of Biochemistry, 95, 87 (1984)).

Examples of yeasts include Saccharomyces cereviseae AH22, AH22R⁻, NA87-11A, DKD-5D, 20B-12.

Examples of insects include a larva of Bombyx mori (Maeda et al., Nature, 315, 592, 1985).

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea; and for the virus BmNPV, a cell line derived from Bombyx mori (Bombyx mori N cell; BmN cell). Examples of the Sf cell include Sf9 cell (ATCC CRL1711) and Sf21 cell (both cells are described in Vaughn, J. L. et al., In Vitro, 13 213-217, 1977).

Examples of animal cells include monkey COS7 cell, Vero cell, Chinese hamster cell (CHO), dhfr gene-deficient Chinese hamster cell (CHO/dhfr⁻ cell), mouse L cell, mouse 3T3 cell, mouse myeloma cell, human HEK293 cell, human FL cell, 293 cell, C127 cell, BALB3T3 cell, Sp-2/O cell.

Escherichia bacteria can be transformed, for example, by the method described in Proc. Natl. Acad. Sci. U.S.A., 69, 2110 (1972), or Gene, 17, 107 (1982).

Bacillus bacteria can be transformed, for example, by the method described in Molecular & General Genetics, 168, 111 (1979).

Yeasts can be transformed, for example, by the method described in Methods in Enzymology, 194, 182-187 (1991) or Proc. Natl. Acad. Sci. U.S.A., 75, 1929 (1978).

Insect cells or insects can be transformed, for example, according to the method described in Bio/Technology, 6, 47-55 (1988).

Animal cells can be transformed, for example, according to the method described in Virology, 52, 456 (1973).

To introduce the expression vector into cells, for example, lipofection method (Felgner, P.L. et al, Proc. Natl. Acad. Sci. U.S.A. 84, 7413, 1987), calcium phosphate method (Graham, F.L. and van der Eb, A.J., Virology 52, 456-467, 1973), or electroporation method (Nuemann, E. et al., EMBO. J. 1, 841-845, 1982) can be used.

In this way, a transformant, which is transformed with the expression vector comprising the DNA encoding the polypeptide of the invention, can be obtained.

To express the polypeptide of the invention in a stable manner using animal cells, the animal cell clone can be selected, which incorporats the introduced expression vector into the chromosome. To be specific, using the above selection marker as an index, such a transformant can be selected. It is possible to obtain a stable animal cell strain expressing highly the polypeptide of the invention by repeating the clonal selection from animal cells once obtained using the selection marker.

In addition, when using dhfr gene as a selection marker, an animal cell strain showing higher expression can be obtained by culturing transformed cells in gradually increasing concentrations of MTX; and selecting an MTX-resistant cell strain to amplify the DNA encoding the polypeptide along with dhfr gene in the cells.

The polypeptide of the invention can be produced by culturing the above-mentioned transformant under condition allowing the expression of the DNA encoding the polypeptide of the invention; and producing and accumulating the polypeptide of the invention.

When an Escherichia or Bacillus bacterium is used as the host, the transformant can be appropriately cultured in a liquid medium, which contains materials required for the transformant growth such as carbon sources, nitrogen sources, and inorganic materials. Examples of the carbon sources include glucose, dextrin, soluble starch, and sucrose. Examples of the nitrogen sources include inorganic or organic materials such as ammonium salts, nitrates, corn steep liquor, peptone, casein, meat extract, soybean cake, and potato extract. Examples of the inorganic materials include calcium chloride, sodium dihydrogenphosphate, and magnesium chloride. In addition, yeast extracts, vitamins, and growth-stimulating factors may also be added. Desirably, the medium has a pH of about 5 to 8.

A preferred example of the medium for culturing Escherichia bacteria is M9 medium supplemented with glucose and casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to increase the promoter efficiency.

When an Escherichia bacterium is used as the host, the transformant is usually cultured at about 15 to 43 ° C for about 3 to 24 hours. If necessary, the culture may be aerated or agitated.

When a Bacillus bacterium is used as the host, the transformant is cultured generally at about 30 to 40 °C for about 6 to 24 hours. If necessary, the culture can be aerated or agitated.

When a yeast is used as the host, the medium for culturing the transformant may be Burkholder's minimal medium (Bostian, K. L. et al., Proc. Natl. Acad. Sci. U.S.A. 77, 4505, 1980) or SD medium supplemented with 0.5% Casamino acids (Bitter, G. A. et al., Proc, Natl. Acad. Sci. U.S.A. 81,5330, 1984). Preferably, pH of the medium is adjusted to about 5 to 8. In general, the transformant is cultured at about 20 to 35 °C for about 24 to 72 hours. If necessary, the culture can be aerated or agitated.

When an insect cell or insect is used as the host, the medium for culturing the transformant may be Grace's Insect Medium (Grace, T.C.C., Nature 195, 788, 1962) supplemented with an appropriate additive such as 10% inactivated bovine serum. Preferably, pH of the medium is adjusted to about 6.2 to 6.4. Generally, the transformant is cultured at about 27 °C for about 3 to 5 days and, if necessary, the culture can be aerated or agitated.

When an animal cell is used as the host, the medium for culturing the transformant may be MEM medium (Science 122, 501, 1952), DMEM medium (Virology 8, 396, 1959), RPMI 1640 medium (The Journal of the American Medical Association 199, 519, 1967) or 199 medium (Proceeding of the Society for the Biological Medicine 73, 1, 1950), which contain about 5 to 20 % fetal bovine serum. Preferably, pH of the medium is about 6 to 8. The transformant is usually cultured at about 30 to 40 °C for about 15 to 60 hours and, if necessary, the culture can be aerated or agitated.

In particular, when using dhfr gene as a selection marker in CHO (dhfr⁻) cells, preferably used is DMEM medium containing dialyzed fetal bovine serum and almost no thymidine.

The polypeptide of the invention can be isolated or purified from the culture described above by the following procedures.

To extract the polypeptide of the invention from the culture of bacteria or cells, after the culture is completed, the bacteria or cells are collected by a known method and suspended in an appropriate buffer. The bacteria or cells are disrupted by a known method such as ultrasonication, lysozyme treatment and/or freeze-thaw cycling, and then subjected to the centrifugation or filtration to obtain the crude peptide extract. The buffer may contain a protein denaturing agent such as urea or guanidine hydrochloride, or a surfactant such as Triton X-100^{TM}.

In case that the polypeptide of the invention is secreted into the culture broth, after the culture is completed, the supernatant can be separated from bacteria or cells by a known method.

The polypeptide of the invention contained in the supernatant or the extract thus obtained can be purified by an appropriate combination of well-known isolation or purification methods. Such isolation or purification methods include a method utilizing difference in solubility such as salting out, solvent precipitation; a method utilizing difference mainly in molecular weight such as dialysis, ultrafiltration, gel filtration, SDS-polyacrylamide gel electrophoresis; a method utilizing difference in electric charge such as ion exchange chromatography; a method utilizing specific affinity such as affinity chromatography; a method utilizing difference in hydrophobicity such as reversed phase high performance liquid chromatography; a method utilizing difference in isoelectric point such as isoelectrofocusing electrophoresis, chromatofocusing; and the like.

When the polypeptide of the invention thus obtained is in a free form, it can be converted into a salt form by a well-known method or a modification thereof. On the other hand, when the polypeptide is obtained in a salt form, it can be converted into the free form or a different salt form by a well-known method or a modification thereof.

The polypeptide of the invention produced by the transformant can be treated, before or after the purification, with an appropriate protein-modifying enzyme so that the polypeptide can be appropriately modified or partially deleted. Examples of the protein-modifying enzyme include trypsin, chymotrypsin, arginyl endopeptidase, protein kinase, glycosidase and the like.

The thus produced polypeptide of the invention can be detected by an enzyme immunoassay using a specific antibody.

In the following, utilities of the agent regulating the LH secretion comprising the polypeptide of the invention or the DNA encoding the same are described in detail.

As apparent in Examples described later, the polypeptide of the invention and the DNA encoding the same have the activity of increasing the blood LH level in a specific manner (stimulatory activity of the LH secretion), and such a response is enhanced in Zucker fatty rats having abnormality of Leptin receptor (Example 3). Further, Galanin, which has a high homology to the polypeptide of the invention, shows no stimulatory activity of the LH secretion, indicating that the stimulatory activity of the LH secretion is specific to the polypeptide of the invention.

In addition, as shown in Example 3, the polypeptide of the invention plays a part in Leptin-induced increase in the blood LH level.

As above, the polypeptide of the invention and the DNA encoding the same have the stimulatory activity of the LH secretion, and thus can be used as an agent to prevent and/or treat various diseases associated with the failure of the LH secretion (e.g. infertility, menoxenia, dysmenorrhea, amenorrhea, oligomenorrhea, premenstrual syndrome, menopausal disorder, dyspituitarism or obesity). Therefore, the agent regulating the LH secretion comprising the polypeptide of the invention or the DNA encoding the same can be used as an agent enhancing the LH secretion, and thus useful as a pharmaceutical to prevent and/or treat infertility, menoxenia, dysmenorrhea, amenorrhea, oligomenorrhea, premenstrual syndrome, menopausal disorder, dyspituitarism or obesity. Further, the agent regulating the LH secretion comprising the polypeptide of the invention or the DNA encoding the same may be effective as a pharmaceutical to prevent and/or treat various diseases particularly resulted from the abnormality of Leptin receptor (e.g. obesity, infertility).

On the other hand, since the polypeptide of the invention has a high affinity to its receptor protein, a higher does of the polypeptide of the invention or the DNA encoding the same leads to the desensitization of the LH secretion. This means that the polypeptide of the invention or the DNA encoding the same can also show the activity of inhibiting the LH secretion. In this case, the agent regulating the LH secretion comprising the polypeptide of the invention or the DNA encoding the same can be used as an agent inhibiting the LH secretion, and thus useful as a pharmaceutical to prevent and/or treat various diseases associated with the excess LH secretion (e.g. prostate cancer, prostate hypertrophy, precocious puberty or LH-producing pituitary gland tumor).

In the following, utilities of the agent regulating the LHRH secretion comprising the polypeptide of the invention or the DNA encoding the same are described in detail.

The polypeptide of the invention and the DNA encoding the same have the stimulatory activity of the LHRH secretion, and thus can be used as prophylactic and/or therapeutic agents for sex hormone-dependent diseases such as dysmenorrhea, amenorrhea, oligomenorrhea, premenstrual syndrome, menopausal disorder, polycystic ovary syndrome; for diseases such as Alzheimer disease, immunodeficiency; and for infertility. As well, they can be used as prophylactic and/or therapeutic agents for sex hormone-independent and LHRH-sensitive benign or malignant tumors.

The agent regulating the LH secretion or the LHRH secretion comprising the polypeptide of the invention or the DNA encoding the same can be prepared and used in a conventional manner. For example, such an agent can be used orally in a form of tablet, optionally sugar- or enteric-coated, capsule, elixir or microcapsule; or parenterally in a form of injection such as a sterile solution and suspension in water or other pharmaceutically acceptable liquid. These preparations can be produced by mixing the polypeptide of the invention or the DNA encoding the same with a physiologically acceptable carrier, a flavoring agent, an excipient, a vehicle, an antiseptic agent, a stabilizer, a binder, and the like in a unit dosage form required for a generally accepted pharmaceutical preparation. The amount of the active ingredient in the preparation is adjusted appropriately within the specified range.

When the DNA of the invention is used, the DNA can be administered in a conventional manner alone or after being inserted into an appropriate vector such as retrovirus vector, adenovirus vector, and adenovirus-associated virus vector.

Additives miscible in a tablet, a capsule, etc. include a binder such as gelatin, corn starch, tragacanth gum, and gum arabic; an excipient such as crystalline cellulose; a swelling agent such as corn starch, gelatin and alginic acid; a lubricant such as magnesium stearate; a sweetening agent such as sucrose, lactose and saccharin; and a flavoring agent such as peppermint, akamono oil and cherry. When the unit dosage form is a capsule, liquid carriers such as oils and fats may further be used together with the additives described above. An injectable sterile composition may be formulated by conventional procedures, for example, by dissolving or suspending the active ingredient in a vehicle such as water for injection with a naturally occurring vegetable oil such as sesame oil, coconut oil.

Examples of an aqueous medium for injection include a physiological saline and an isotonic solution containing glucose and other auxiliary agents (e.g. D-sorbitol, D-mannitol, sodium chloride), which may be used in combination with an appropriate dissolution aid such as an alcohol (e.g. ethanol), a polyalcohol (e.g. propylene glycol, polyethylene glycol), a nonionic surfactant (e.g. polysorbate 80^{TM}, HCO-50). Examples of the oily medium include sesame oil and soybean oil, which may be used in combination with a dissolution aid such as benzyl benzoate and benzyl alcohol.

The composition may further contain a buffer (e.g. phosphate buffer, sodium acetate buffer), a soothing agent (e.g. benzalkonium chloride, procaine hydrochloride), a stabilizer (e.g. human serum albumin, polyethylene glycol), a preservative (e.g. benzyl alcohol, phenol), an antioxidant, etc. An appropriate ampoule is usually filled with the thus prepared injection liquid.

Since the thus obtained pharmaceutical preparation is safe and low toxic, it can be administered to a mammal (e.g. human, mouse, rat, guinea pig, rabbit, sheep, pig, bovine, cat, dog, monkey).

The dose of the polypeptide of the invention or the DNA encoding the same varies depending on conditions, etc. For example, in oral administration of the peptide to an adult (60 kg body weight), the daily dose is normally about 0.1 to 100 mg, preferably about 1.0 to 50 mg, and more preferably about 1.0 to 20 mg. In parenteral administration (e.g. intravenous injection) of the peptide, the single dose also varies depending on a subject, a target organ, condition, administration mode, etc. For example, the daily dose in an injection form is normally about 0.01 to 30 mg, preferably about 0.1 to 20 mg, and more preferably about 0.1 to 10 mg per an adult (60 kg body weight).

To another animal, the dose per the body weight of the animal, as converted on the basis of the does given above per 60 kg body weight, can be administered.

In the following, the receptor to the polypeptide of the invention (Galanin receptor) is described in detail.

In this specification, Galanin receptor refers to a G-protein coupled receptor protein, which is derived from any tissues (e.g. pituitary, pancreas, brain, kidney, liver, genital gland, thyroid, gallbladder, bone marrow, adrenal gland, skin, skeletal muscle, lung, gastrointestinal tract, blood vessel, heart) or cells of warm-blooded animals (e.g. mammal (e.g. human, rabbit, sheep, goat, rat, mouse, guinea pig, bovine, horse, pig), fowl (e.g. chicken, pigeon, duck, goose, quail)), and which comprises the same or substantially the same amino acid sequence as the sequence shown by SEQ ID NO: 1 as GALR1 (Proc. Natl. Acad. Sci. USA 90, 3845-3849, 1993; J. Mol. Neurosci. 6, 33-41, 1995; FEBS Lett. 411, 225-230, 1997); the sequence shown by SEQ ID NO: 2 as GALR2 (described above); or the sequence shown by SEQ ID NO: 3 as GALR3 (J. Biol. Chem. 272, 31949-31952, 1997).

The receptor protein includes a protein comprising the amino acid sequence shown by SEQ ID NO: 1, 2 or 3; and also a protein comprising an amino acid sequence having about 90 to 99.9% homology to that shown by SEQ ID NO: 1, 2 or 3 and having an activity substantially equivalent in property to that of the protein comprising the amino acid sequence shown by SEQ ID NO: 1, 2 or 3.

The activities of these proteins include the ligand-binding activity (to the polypeptide of the invention), the signal transduction activity and the like. The term "substantially equivalent in property" refers to equivalence of these activities in property. These activities can be measured according to a known method or a modified method thereof, for example, the methods described in Reference Example 1 or 17 or Example 1 to 3. Therefore, quantitative factors such as levels of the ligand-binding activity and the signal transduction activity, and molecular weights of the receptor proteins may be different.

The receptor protein includes variants thereof, wherein the N-terminal Met is protected with a protecting group (e.g. C₁₋₆ alkanoyl such as formyl, acetyl); wherein the N-terminal glutamyl group is pyroglutaminated; wherein the side chain of amino acid in the molecule is protected with a suitable protecting group (e.g. C₁₋₆ alkanoyl such as formyl, acetyl); and conjugated proteins such as glycoprotein having sugar chains.

The receptor protein may take a salt form like the aforementioned salts of the polypeptide of the invention.

The receptor protein or a salt thereof, or a partial peptide thereof can be produced according to a know protein purification method from tissues or cells of a warm-blooded animal; or according to a variation of the afore mentioned method of culturing a transformant comprising the DNA encoding the polypeptide of the invention; or according to the peptide synthesis method as described above.

The partial peptide of the receptor protein may contain a region exposed to outside of the cell membrane among the whole G-protein coupled receptor protein. This is a peptide containing an extracellular region (hydrophilic region) of the G-protein coupled receptor protein, which is predicted by the hydrophobic plot analysis. The peptide containing a hydrophobic region in part can be used as well. In addition, the peptide may contain one of respective domains alone or a number of these domains together.

The partial peptide of the receptor protein may take a salt form like the aforementioned salts of the peptide.

The DNA encoding Galanin receptor protein refers to any DNA comprising a nucleotide sequence encoding Galanin receptor protein comprising the same or substantially the same amino acid sequence as the sequence shown by SEQ ID NO: 1, 2 or 3. Such a DNA may be derived from a genome DNA, a genome DNA library, cDNAs derived from the tissues and cells, a cDNA library derived from the tissues and cells, or synthetic DNAs. Vectors used for the libraries may be any one of bacteriophage, plasmid, cosmid, phagemid, and the like. In addition, the DNA can be amplified directly by RT-PCR from the RNA fraction prepared from the tissues or cells.

Examples of the DNAs encoding Galanin receptors comprising the amino acid sequences shown by SEQ ID NO: 1, 2 and 3 are the DNAs having the nucleotide sequences shown by SEQ ID NO: 4, 5 and 6, respectively.

It is possible to select an activator or inhibitor (antagonist, antibody, antisense polynucleotide, etc) of GALP from test compounds according to the method described in Examples 1 to 3 or a variation thereof (e.g. by comparing the change of the blood LH or LHRH level with the change of the blood LH or LHRH level after addition of a test compound without or with GALP in the process described in Examples 1 to 3 or a variation thereof).

The activator of GALP, obtained by the screening method, has the same stimulatory activity of the LH secretion as the polypeptide of the invention has. Therefore, the agent enhancing the LH secretion comprising the activator is useful as a prophylactic and/or therapeutic agent for infertility, menoxenia, dysmenorrhea, amenorrhea, oligomenorrhea, premenstrual syndrome, menopausal disorder, dyspituitarism or obesity. The agent enhancing the LH secretion can be prepared and used in the same way as the polypeptide of the invention can.

Similarly, the inhibitor of GALP, obtained by the screening method, has the inhibitory activity of the LH secretion. Therefore, the agent inhibiting the LH secretion comprising the inhibitor is useful as a prophylactic and/or therapeutic agent for prostate cancer, prostate hypertrophy, precocious puberty or LH-producing pituitary gland tumor. The agent enhancing the LH secretion can be prepared and used in the same way as the polypeptide of the invention can.

The activator of GALP, obtained by the screening method, has the same stimulatory activity of the LHRH secretion as the polypeptide of the invention has. Therefore, the agent enhancing the LHRH secretion comprising the activator can be used as a prophylactic and/or therapeutic agent for sex hormone-dependent diseases such as dysmenorrhea, amenorrhea, oligomenorrhea, premenstrual syndrome, menopausal disorder, polycystic ovary syndrome; for diseases such as Alzheimer disease, immunodeficiency; and for infertility. As well, it can be used as a prophylactic and/or therapeutic agent for sex hormone-independent and LHRH-sensitive benign or malignant tumors.

Similarly, the inhibitor of GALP, obtained by the screening method, has the inhibitory activity of the LHRH secretion. Therefore, the agent inhibiting the LHRH secretion comprising the inhibitor is useful as prophylactic and/or therapeutic agents for hormone-dependent diseases, in particular, sex hormone-dependent cancers (e.g. prostate cancer, uterine cancer, breast cancer, pituitary tumor), prostate hypertrophy, endometriosis, uterine myoma, precocious puberty, and contraception.

The compound of the invention is very effective even by itself, and can be combined with other medications and treatments for much more effectiveness. The combined medications and treatments include, but not limited to, "a physiologically active peptide capable of regulating the LHRH secretion" and "an appetite suppressant or a physiologically active peptide capable of suppressing appetite" (hereinafter referred to as the combined medication). In addition to such a concomitant use, an admixture formulation having a combination of the compound of the invention and another concomitant medicable compound (various ones as described specifically) can be prepared. Alternatively, the compound of the invention and another medication can be administered at the same time or separately with a given time interval.

Preferred examples of the "physiologically active peptide capable of regulating the LHRH secretion" include LHRH derivatives or salts thereof, which are useful for hormone-dependent diseases, in particular, sex hormone-dependent diseases such as sex hormone-dependent cancers (e.g. prostate cancer, uterine cancer, breast cancer, pituitary tumor), prostate hypertrophy, endometriosis, uterine myoma, precocious puberty, dysmenorrhea, amenorrhea, oligomenorrhea, premenstrual syndrome, polycystic ovary syndrome, and contraception (or infertility if rebound effect after cessation of the drug is used). Further included are LHRH derivatives or salts thereof, which are useful for sex hormone-independent and LHRH-sensitive benign or malignant tumors.

Such LHRH derivatives or salts thereof include peptides described in "Treatment with GnRH analogs: Controversies and perspectives" (The Parthenon Publishing Group Ltd., 1996), JP patent application publication Nos. H3-503165, H3-101695, H7-97334, and H8-259460.

The LHRH derivatives include LHRH agonists and LHRH antagonists. Examples of the LHRH antagonists include a physiologically active peptide represented by the following general formula (I):

X-D2Nal-D4ClPhe-D3Pal-Ser-A-B-Leu-C-Pro-DAlaNH2,

wherein X represents N(4H2-furoyl)Gly or NAc; A represents NMeTyr, Tyr, Aph(Atz) or NMeAph(Atz); B represents DLys(Nic), DCit, DLys(AzaglyNic), DLys(AzaglyFur), DhArg(Et2), DAph(Atz) or DhCi; and C represents Lys(Nisp), Arg or hArg(Et2), or a salt of the peptide.

Examples of the LHRH agonists include a physiologically active peptide represented by the following general formula (II):

5-oxo-Pro-His-Trp-Ser-Tyr-Y-Leu-Arg-Pro-Z,

wherein Y represents DLeu, DAla, DTrp, DSer(tBu), D2Nal or DHis(ImBzl); and Z represents NH-C2H5 or Gly-NH2,
or a salt of the peptide.

Particularly suitable is the peptide wherein Y is DLeu and Z is NH-C2H5 (This is peptide A or leuprorelin represented by
5-oxo-Pro-His-Trp-Ser-Tyr-DLeu-Leu-Arg-Pro-NH-C2H5) or a salt thereof (e.g. acetate form).

These peptides can be produced according to methods described in the aforementioned documents or publications, or variations thereof.

Abbreviations in the general formulas (I) and (II) are as follows.
- N(4H2-furoyl)Gly: : N-tetrahydrofuroylglycine
- NAc: : N-acetyl
- D2Nal: : D-3-(2-naphthyl)alanine
- D4ClPhe: : D-3-(4-chloro)phenylalanine
- D3Pal: : D-3-(3-pyridyl)alanine
- NMeTyr: : N-methyltyrosine
- Aph(Atz): : N-[5'-(3'-amino-1'H-1',2',4'-triazolyl)]phenylalanine
- NMeAph(Atz): : N-methyl-[5'-(3'-amino-1'H-1',2',4'-triazolyl)]phenylalanine
- DLys(Nic): : D-(e-N-nicotinoyl)lysine
- Dcit: : D-citrulline
- DLys(AzaglyNic): : D-(azaglycylnicotinoyl)lysine
- DLys(AzaglyFur): : D-(azaglycylfuranyl)lysine
- DhArg(Et2): : D-(N,N'-diethyl)homoarginine
- DAph(Atz): : D-N-[5'-(3'-amino-1'H-1',2',4'-triazolyl)]phenylalanine
- DhCi: : D-homocitrulline
- Lys(Nisp): : (e-N-isopropyl)lysine
- hArg(Et2): : (N,N'-diethyl)homoarginine

As used herein, the appetite suppressant refers to a drug suppressing appetite through the direct or indirect action on appetite center, and includes centrally acting appetite suppressants and physiologically active peptide-related substances.

As used herein, the centrally acting appetite suppressant refers to a drug suppressing appetite through the action on the alpha-adrenergic receptor, the beta-adrenergic receptor, the dopamine receptor, or the serotonin receptor.

Suitable examples of the centrally acting appetite suppressant include alpha-adrenergic receptor antagonists (e.g. yohimbine), beta-adrenergic receptor agonists (e.g. mazindol, amphetamine, dextroamphetamine, phentermine, benzphetamine, methamphetamine, phendimetrazine, phenmetrazine, diethylpropion, sibutramine, phenylpropanolamine, clobenzorex), dopamine receptor agonists (e.g. ER-230, doprexin), serotonin receptor agonists (e.g. dexfenfluramine, fenfluramine), 5-HT agonists (e.g. (+)norfenfluramine, sertraline), cimetidine, ergoset.

The physiologically active peptide-related substances include physiologically active peptides suppressing appetite through the direct or indirect action on appetite center, analogs of the peptides, agonists or antagonists of the physiologically active peptides.

Suitable examples of the physiologically active peptide-related substances include leptin or analogs thereof, Leptin receptor agonists, agents improving leptin resistance, neuropeptide Y (NPY) antagonists (e.g. NGD-95-1, SR-120819-A, PD-160170, 1229-U-91), cholecystokinin (CCK) agonists (e.g. FPL-15849, GW-5823, GW-7178, GI-248573, AR-R-19021), glucagon-like peptide 1 (GLP-1) or analogs or agonists thereof (e.g. AZM-134), galanin antagonists, glucagon agonists, melanin-concentrating hormone (MCH) antagonists, melanocortin agonists (in particular, melacortin 4 receptor (MC4R) agonists, MC4R/MC3R agonists), enterostatin agonists, tripeptidyl peptidase II inhibitors (e.g. UCL-1397), corticotropin-releasing hormone or analogs or agonists thereof (e.g. urocortin).

Used as the appetite suppressant are preferably the centrally acting appetite suppressant, more preferably beta-adrenergic receptor agonists, and most preferably mazindol.

In the specification and drawings, bases, amino acids, and the like are abbreviated in accordance with the IUPAC-IUB Commission on Biochemical Nomenclature or the conventional usage in the art, as shown below for example. When an amino acid has optical isomers, its L form is selected unless otherwise indicated.
- DNA: : deoxyribonucleic acid
- cDNA: : complementary deoxyribonucleic acid
- A: : adenine
- T: : thymine
- G: : guanine
- C: : cytosine
- Y: : thymine or cytosine
- N: : thymine, cytosine, adenine or guanine
- R: : adenine or guanine
- M: : cytosine or adenine
- W: : thymine or adenine
- S: : cytosine or guanine
- I: : inosine
- H: : adenine, thymine or cytosine
- D: : guanine, adenine or thymine
- B: : guanine, thymine or cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- EIA: : enzyme immuno-assay
- Gly or G: : glycine
- Ala or A: : alanine
- Val or V: : valine
- Leu or L: : leucine
- Ile or I: : isoleucine
- Ser or S: : serine
- Thr or T: : threonine
- Cys or C: : cysteine
- Met or M: : methionine
- Glu or E: : glutamic acid
- Asp or D: : aspartic acid
- Lys or K: : lysine
- Arg or R: : arginine
- His or H: : histidine
- Phe or F: : phenylalanine
- Tyr or Y: : tyrosine
- Trp or W: : tryptophan
- Pro or P: : proline
- Asn or N: : asparagine
- Gln or Q: : glutamine
- pGlu: : pyroglutamic acid
- Me: : methyl
- Et: : ethyl
- Bu: : butyl
- Ph: : phenyl
- TC: : thiazolidine-4(R)-carboxamido

Substituents, protecting groups and reagents used often in this specification are shown by the following codes.
- Tos: : p-toluenesulfonyl
- HONB :: 1-hydroxy-5-norbornene-2,3-dicarboximide
- Bzl: : benzyl
- Cl₂-Bzl: : 2,6-dichlorobenzyl
- Z: : benzyloxycarbonyl
- Br-Z: : 2-bromobenzyl oxycarbonyl
- Cl-Z: : 2-chlorobenzyloxycarbonyl
- Boc: : t-butoxycarbonyl
- HOBt: : 1-hydroxybenztriazole
- DCC: : N,N'-dichlorohexylcarbodiimide
- TFA: : trifluoroacetic acid
- Fmoc: : N-9-fluorenyl methoxycarbonyl
- DNP: : dinitrophenol
- Bum: : t-butoxymethyl
- Trt: : trityl
- PAM: : phenylacetoamidemethyl
- BHA: : benzhydrylamine
- Bom: : benzyloxymethyl
- OcHex: : cyclohexylester
- MeBzl: : 4-methylbenzyl
- CHO: : formyl
- NMP: : N-methylpyrrolidone

SEQ ID NOs in the sequence listing of the specification indicate the following sequences, respectively.

### [SEQ ID NO: 1]

This shows the whole amino acid sequence of rat GALR1 (Galanin receptor type 1).

### [SEQ ID NO: 2]

This shows the whole amino acid sequence of rat GALR2 (Galanin receptor type 2).

### [SEQ ID NO: 3]

This shows the whole amino acid sequence of rat GALR3 (Galanin receptor type 3).

### [SEQ ID NO: 4]

This shows the base sequence of rat GALR1 (Galanin receptor type 1) cDNA.

### [SEQ ID NO: 5]

This shows the base sequence of rat GALR2 (Galanin receptor type 2) cDNA.

### [SEQ ID NO: 6]

This shows the base sequence of rat GALR3 (Galanin receptor type 3) cDNA.

### [SEQ ID NO: 7]

This shows the whole amino acid sequence of pig galanin (29 residues).

### [SEQ ID NO: 8]

This shows the amino acid sequence of pig galanin precuorsor or preprogalain (1-123).

### (SEQ ID NO: 9]

This shows the amino acid sequence of pig galanin precuorsor or preprogalain (24-61).

### [SEQ ID NO: 10]

This shows the amino acid sequence of pig galanin precuorsor or preprogalain (37-61).

### [SEQ ID NO: 11]

This shows the partial amino acid sequence of the pig peptide of the invention (34 residues from N-terminal).

### [SEQ ID NO: 12]

This shows the partial amino acid sequence of the pig peptide of the invention (32 residues from N-terminal).

### [SEQ ID NO: 13]

This shows the partial amino acid sequence of the pig peptide of the invention or a fragment thereof obtained by chymotrypsin digestion (CHY-1)(9 residues from N-terminal).

### [SEQ ID NO: 14]

This shows the partial amino acid sequence of the pig peptide of the invention or a fragment thereof obtained by chymotrypsin digestion (CHY-4)(6 residues).

### [SEQ ID NO: 15]

This shows the partial amino acid sequence of the pig peptide of the invention or a fragment thereof obtained by chymotrypsin digestion (CHY-3)(27 residues).

### [SEQ ID NO: 16]

This shows the partial amino acid sequence of the pig peptide of the invention or a fragment thereof obtained by chymotrypsin digestion (CHY-2)(11 residues).

### [SEQ ID NO: 17]

This shows the partial amino acid sequence of the pig peptide of the invention (44 residues).

### [SEQ ID NO: 18]

This shows the base sequence of a synthetic DNA (primer 1) used for the screening of cDNA encoding rat GALR2.

### [SEQ ID NO: 19]

This shows the base sequence of a synthetic DNA (primer 2) used for the screening of cDNA encoding rat GALR2.

### [SEQ ID NO: 20]

This shows the base sequence of a synthetic DNA (primer pGAL4-7F) used in the Degenerated PCR.

### [SEQ ID NO: 21]

This shows the base sequence of a synthetic DNA (primer pGAL9-3F) used in the Degenerated PCR.

### [SEQ ID NO: 22]

This shows the base sequence of a synthetic DNA (primer pGAL34-1R) used in the Degenerated PCR.

### [SEQ ID NO: 23]

This shows the base sequence of the nested PCR product contained in pCR 100-6.

### [SEQ ID NO: 24]

This shows the base sequence of the nested PCR product contained in pCR100-7.

### [SEQ ID NO: 25]

This shows the base sequence of a synthetic DNA (primer pGAL9-3F) used for preparation of a hybridization probe.

### [SEQ ID NO: 26]

This shows the base sequence of a synthetic DNA (primer pGAL34-8R) used for preparation of a hybridization probe.

### [SEQ ID NO: 27]

This shows the base sequence of the cDNA contained in pGR2PL6 obtained in Reference Example 5.

### [SEQ ID NO: 28]

This shows the base sequence of the cDNA contained in pGR2PL3 obtained in Reference Example 5.

### [SEQ ID NO: 29]

This shows the sequence of the precursor of the peptide of the invention, which is deduced from the base sequence of the cDNA contained in pGR2PL6.

### [SEQ ID NO: 30]

This shows the sequence of the precursor of the peptide of the invention, which is deduced from the base sequence of the cDNA contained in pGR2PL3.

### [SEQ ID NO: 31]

This shows the sequence of the mature protein relating to the peptide of the invention (pig), which is deduced from the base sequence of the cDNA contained in pGR2PL6 and pGR2PL3.

### [SEQ ID NO: 32]

This shows the base sequence of the cDNA encoding the mature protein relating to the peptide of the invention (pig).

### [SEQ ID NO: 33]

This shows the sequence of the mature protein relating to the peptide of the invention (rat).

### [SEQ ID NO: 34]

This shows the sequence of the mature protein relating to the peptide of the invention (human).

### [SEQ ID NO: 35]

This shows the partial amino acid sequence of the mature protein relating to the peptide of the invention (9 residues from N-terminal)(common in rat and human).

### [SEQ ID NO: 36]

This shows the partial amino acid sequence of the mature protein relating to the peptide of the invention (21 residues from N-terminal)(common in rat and human).

### [SEQ ID NO: 37]

This shows the amino acid sequence of the precursor protein of the mature protein relating to the peptide of the invention (rat).

### [SEQ ID NO: 38]

This shows the amino acid sequence of the precursor protein of the mature protein relating to the peptide of the invention (human).

### [SEQ ID NO: 39]

This shows the sequence of the cDNA encoding the mature protein relating to the peptide of the invention (rat).

### [SEQ ID NO: 40]

This shows the sequence of the cDNA encoding the mature protein relating to the peptide of the invention (human).

### [SEQ ID NO: 41]

This shows the sequence of the cDNA encoding the precursor protein of the mature protein relating to the peptide of the invention (rat).

### [SEQ ID NO: 42]

This shows the sequence of the cDNA encoding the precursor protein of the mature protein relating to the peptide of the invention (human).

### [SEQ ID NO: 43]

This shows the partial amino acid sequence of the mature protein relating to the peptide of the invention (pig)(30 residues from N-terminal).

### [SEQ ID NO: 44]

This shows the amino acid sequence of the peptide used as an immunogen in Reference Example 9.

### [SEQ ID NO: 45]

This shows the sequence of the DNA fragment obtained by EcoRI/BglII digestion of the plasmid pGR2PL6, used in Reference Example 18.

### [SEQ ID NO: 46]

This shows the DNA sequence of primer pGAL1-1F used in Reference Example 18.

### [SEQ ID NO: 47]

This shows the DNA sequence of primer pGAL88-1R used in Reference Example 18.

### [SEQ ID NO: 48]

This shows the DNA sequence of primer F/R120 used in Reference Example 19.

### [SEQ ID NO: 49]

This shows the DNA sequence of primer R/R120 used in Reference Example 19.

### [SEQ ID NO: 50]

This shows the sequence of the cDNA obtained by PCR in Reference Example 19.

### [SEQ ID NO: 51]

This shows the DNA sequence of primer 1F/H120 used in Reference Example 19.

### [SEQ ID NO; 52]

This shows the DNA sequence of primer 1R/H120 used in Reference Example 19.

### [SEQ ID NO: 53]

This shows the DNA sequence of primer 1F/H470 used in Reference Example 19.

### [SEQ ID NO: 54]

This shows the DNA sequence of primer 1R/H470 used in Reference Example 19.

### [SEQ ID NO: 55]

This shows the DNA sequence of primer 1 used in Reference Example 20.

### [SEQ ID NO: 56]

This shows the DNA sequence of primer 2 used in Reference Example 20.

### [SEQ ID NO: 57]

This shows the DNA sequence of primer 3 used in Reference Example 20.

### [SEQ ID NO: 58]

This shows the DNA sequence of primer 4 used in Reference Example 20.

### [SEQ ID NO: 59]

This shows the sequence of a synthetic DNA used in Reference Example 21.

### [SEQ ID NO: 60]

This shows the sequence of a synthetic DNA used in Reference Example 21.

### [SEQ ID NO: 61]

This shows the amino acid sequence of rat galanin. (Rat galanin is a peptide having the amino acid sequence of SEQ ID NO: 61 and its C-terminal amidation).

### [SEQ ID NO: 62]

This shows the amino acid sequence of the mature form of the mouse ligand of GALR2 according to the invention.

### [SEQ ID NO: 63]

This shows the amino acid sequence of the precursor of the mouse ligand of GALR2 according to the invention.

### [SEQ ID NO: 64]

This shows the base sequence of the cDNA encoding the mouse ligand of GALR2 according to the invention.

### [SEQ ID NO: 65]

This shows the base sequence of the primer used for cloning of the cDNA encoding the mouse ligand of GALR2.

### [SEQ ID NO: 66]

This shows the base sequence of the primer used for cloning of the cDNA encoding the mouse ligand of GALR2.

### [SEQ ID NO: 67]

This shows the base sequence of the cDNA cloned using primers shown by SEQ ID NOs: 4 and 5.

### [SEQ ID NO: 68]

This shows the base sequence of the primer used in 5'-RACE for cloning of the cDNA encoding the mouse ligand of GALR2.

### [SEQ ID NO: 69]

This shows the base sequence of the primer used in 3'-RACE for cloning of the cDNA encoding the mouse ligand of GALR2.

### [SEQ ID NO: 70]

This shows the base sequence of the primer used in PCR for cloning of the cDNA encoding the mouse ligand of GALR2.

### [SEQ ID NO: 71]

This shows the base sequence of the primer used in PCR for cloning of the cDNA encoding the mouse ligand of GALR2.

### [SEQ ID NO: 72]

This shows the base sequence of the cDNA encoding the precursor of the mouse ligand of GALR2 according to the invention.

The transformant Escherichia coli TOP10/pGR2PL6, obtained in Reference Example 5 is deposited at Institute for Fermentation, Osaka (IFO), located at 2-17-85, Jyuso-Honmati, Yodogawa-ku, Osaka-shi, Osaka 532-8686, Japan, under Accession Number IFO 16201 since August 21, 1998; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6486 since September 4, 1998.

The transformant Escherichia coli TOP10/pGR2HL14, obtained in Reference Example 19 is deposited at Institute for Fermentation, Osaka (IFO), located at 2-17-85, Jyuso-Honmati, Yodogawa-ku, Osaka-shi, Osaka 532-8686, Japan, under Accession Number IFO 16256 since February 5, 1999; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6657 since February 22, 1999.

The transformant Escherichia coli TOP10/pGR2RL4, obtained in Reference Example 18 is deposited at Institute for Fermentation, Osaka (IFO), located at 2-17-85, Jyuso-Honmati, Yodogawa-ku, Osaka-shi, Osaka 532-8686, Japan, under Accession Number IFO 16257 since February 5, 1999; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6658 since February 22, 1999.

The transformant Escherichia coli MM294(DE3)/pTFCGAL, obtained in Reference Example 21 is deposited at Institute for Fermentation, Osaka (IFO), located at 2-17-85, Jyuso-Honmati, Yodogawa-ku, Osaka-shi, Osaka 532-8686, Japan, under Accession Number IFO 16260 since February 26, 1999; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6678 since March 10, 1999.

The transformant Escherichia coli MM294(DE3)/pTB960-11, obtained in Reference Example 21 is deposited at Institute for Fermentation, Osaka (IFO), located at 2-17-85, Jyuso-Honmati, Yodogawa-ku, Osaka-shi, Osaka 532-8686, Japan, under Accession Number IFO 16100 since June 25, 1997; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6388 since June 15, 1998.

The transformant Escherichia coli MM294(DE3)/pTCIId23-MPIF1, obtained in Reference Example 21 is deposited at Institute for Fermentation, Osaka (IFO), located at 2-17-85, Jyuso-Honmati, Yodogawa-ku, Osaka-shi, Osaka 532-8686, Japan, under Accession Number IFO 16212 since October 27, 1998; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6582 since November 24, 1998.

GR2-1N obtained in Reference Example 13 is deposited at Institute for Fermentation, Osaka (IFO), located at 2-17-85, Jyuso-Honmati, Yodogawa-ku, Osaka-shi, Osaka 532-8686, Japan, under Accession Number IFO 50515 since March 11, 1999; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-6682 since March 17, 1999.

The transformant Escherichia coli TOP10/pGR2ML1, obtained in Reference Example 25 is deposited at Institute for Fermentation, Osaka (IFO), located at 2-17-85, Jyuso-Honmati, Yodogawa-ku, Osaka-shi, Osaka 532-8686, Japan, under Accession Number IFO 16361 since February 2, 2000; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Number FERM BP-7092 since March 16, 2000.

### EXAMPLES

The invention is specifically described below with the following Reference Examples and Examples, but it is not limited thereto.

### REFERENCE EXAMPLE 1:

### Detection of Galanin Receptor (GALR1, GALR2) Activating Effect

### (1-1) Construction of Rat-Type Galanin Receptor (GALR1, GALR2) Expressing Cells

In order to obtain the gene encoding rat GALR2, 2 µl of rat hypothalamus cDNA (Clontech), 1 µl each of the following 10 µM primers: Primer 1 (5'-GTCGACATGAATGGCTCCGGCAGCCAG-3', SEQ ID NO: 18) and Primer 2 (5'-ACTAGTTTAACAAGCCGGATCCAGGGTTCTAC-3', SEQ ID NO: 19), 5 µl of 10-fold concentrated buffer (attached to Klen Taq DNA polymerase (Clontech)), 1 µl of 10 mM deoxynucleotide mixture (Invitrogen), 1 µl of Klen Taq DNA polymerase (Clontech) and 39 µl of distilled water for injection (Otsuka Pharmaceutical) were mixed together to prepare a PCR reaction solution. PCR reaction was carried out using Gene Amp PCR System 9700 (Perkin Elmer) under the following conditions: (i) 1 cycle of (95ºC, 5 min), (ii) 3 cycles of (95ºC, 30 sec; 76ºC, 15 sec; 72ºC, 2 min), (iii) 3 cycles of (95ºC, 30 sec; 72ºC, 15 sec; 72ºC 2 min), (iv) 3 cycles of (95ºC, 30 sec; 68ºC, 15 sec; 72ºC, 2 min), (v) 3 cycles of (95ºC, 30 sec; 64ºC, 15 sec; 72ºC, 2 min), (vi) 3 cycles of (95ºC, 30 sec; 60ºC, 15 sec; 72ºC, 2 sec), and (vii) 20 cycles of (95ºC, 30 sec; 56ºC, 15 sec; 72ºC, 2 min). After the PCR reaction, 10 µl of the reaction solution was electrophoresed on 1 % low melting point agarose gel (Sea Plaque GTG; FTN), followed by the confirmation of the PCR product by ethidium bromide staining. The DNA band at approximately 1.2 kbp obtained as the PCR product was recovered from the agarose gel by conventional methods. Briefly, the agarose gel fraction was transferred into a 0.5 ml sampling tube, which was then heated to 70ºC to melt the agarose gel. After equilibration at 40ºC, 0.5 µl of beta agarase (Nippon Gene) was added thereto and reacted for 60 min to degrade the agarose. From the resultant reaction solution, the DNA fragment of interest was recovered by the conventional ethanol precipitation method and then ligated to PCR-Script (Stratagene) plasmid vector. This ligation reaction solution was added to Competent High (JM109; TOYOBO) for transformation. The resultant transformants were cultured overnight on LB agar medium containing 100 µg/ml ampicillin (Wako Purechemical Industries). Colonies formed on the medium were subjected to colony PCR to examine the presence or absence of the insertion of the DNA fragment of interest. Colonies having the insertion of the DNA fragment of interest were cultured overnight in LB medium containing 100 µg/ml ampicillin (Wako Purechemical Industries). Plasmids having the inserted DNA fragment were recovered from the resultant culture broth using Plasmid Mini Kit (Qiagen). Reactions for analyzing nucleotide sequences were carried out using Dye Primer Cycle Sequencing Ready Reaction (ABI). As a result of nucleotide sequence analysis with an automated fluorescence DNA sequencer, it was confirmed that the inserted DNA fragment in the plasmid was a 1119 bp DNA encoding GALR2.

An animal cell expression vector, pAKKO-1.11, was prepared according to the method described in Reference Example 4 in Japanese Unexamined Patent Publication No. 7-304797. The DNA fragment encoding GALR2 was recovered from the above-described plasmid by conventional methods and ligated to this vector pAKKO-1.11. The resultant vector was introduced into *Escherichia coli* DH5α to thereby obtain a transformant. By culturing this transformant, plasmids containing the DNA encoding GALR2 were prepared.

The plasmid was transfected into CHO cells by the following procedures using CellPhect Transfection Kit (Pharmacia) to thereby obtain GALR2 expressing cells of interest. Briefly, 240 µl of Buffer A (attached to CellPhect Transfection Kit) was added to 9.6 mg of the plasmid DNA dissolved in 240 µl of distilled water, agitated and left stationary for 10 min. Then, 480 µl of Buffer B (attached to CellPhect Transfection Kit) was added thereto and agitated vigorously to thereby form liposomes containing the DNA. CHO/dhfr⁻ cells (4x10⁵) (obtained from ATCC) were plated in a 60 mm petri dish and cultured in Ham's F-12 medium (Nihon Pharmaceutical) containing 10% fetal calf serum (Bio Whittaker) at 37ºC under 5% CO₂ for 2 days. Then, 480 µl of the liposome was added dropwise to the cells in the petri dish. These cells were cultured at 37ºC under 5% CO₂ for 6 hr, and washed with serum-free Ham's F-12 medium twice. Then, 3 ml of 15% glycerol was added to the cells in the petri dish to treat the cells for 2 min. The resultant cells were washed again with serum-free Ham's F-12 medium twice and then cultured in Ham's F-12 medium containing 10% fetal calf serum at 37ºC under 5% CO₂ for 15 hr. After trypsin treatment for dispersion, the cells were recovered from the petri dish and plated on 6-well plates at a density of 1.25x10⁴ cells/plate. Cultivation started in Dulbecco's modified Eagle medium (DMEM) (Nihon Pharmaceutical) containing dialyzed 10% fetal calf serum (JRH Biosciences) at 37ºC under 5% CO₂. While transformed CHO cells into which the plasmid has been transferred grow in the medium, non-transferred cells gradually die. Thus, dead cells were removed by exchanging the medium on day 1 and day 2. On day 8 of the cultivation, 20 colonies of the transformed CHO cells grown on the medium (i.e., 20 types of CHO cell clones) were selected. The selected cells (20 types of CHO cell clones) were recovered individually, followed by preparation of membrane fractions from them by the method described later in Section (1-2) of this Reference Example. The amounts of binding of pig ¹²⁵I-galanin (New England Nuclear) to the membrane fractions were determined by a known method (for example, the method described in Examples in EP-0711830A) to thereby select those cell clones with high GALR2 expression. The thus selected cell clones were used in the subsequent experiments.

The cDNA encoding GALR1 was obtained in the same manner as described above in the obtainment of the cDNA encoding GALR2. Briefly, rat GALR1 cDNA consisting of 1486 bp was obtained from a rat brain cDNA library (Clontech) by PCR using Primers 3 and 4. This cDNA was inserted into pUC119 (Takara Shuzo), and the resultant plasmid was designated pRGR2.

This plasmid was double-digested with restriction enzymes EcoRI and Pst I to obtain a cDNA fragment, which was then inserted into an animal cell expression plasmid vector, pcDNA I (Invitrogen), double-digested with restriction enzymes EcoRI and NsiI. The resultant plasmid was designated pRGRPC. This plasmid pRGRPC was double-digested with restriction enzymes Hind III and XbaI to obtain a cDNA fragment, which was then inserted into an animal cell expression plasmid vector, pRc/CMV (Invitrogen), double-digested with restriction enzymes Hind III and XbaI to thereby obtain rat GALR1 cDNA expression plasmid pRGR1.

The rat GALR1 cDNA expression plasmid pRGR1 was introduced into CHO-K1 cells (obtained from ATCC) cultured in Ham's F-12 medium (Nihon Pharmaceutical) by the conventional calcium phosphate method using CellPhect Transfection Kit (Pharmacia). Finally, 500 µl/ml of G-418 resistant 24 clones were isolated using stainless cylinders. Aliquots of the thus isolated 24 clones were plated on 6-well plates and cultured to saturation. Then, a binding experiment to examine the binding of these cells to 100 pM pig ¹²⁵I-galanin (New England Nuclear) was performed (e.g., by the method described in Examples in EP-0711830A) to thereby determine the expression levels of the rat galanin receptor in the 24 clones. As a result, cell clone No. 3 exhibited the highest binding activity to pig ¹²⁵I-galanin, and was believed to have the highest GALR1 expression level. Then, cells of cell clone No. 3 were plated in 96-well microplates at a rate of 1 cell/2 wells by the limiting dilution-culture method and grown from a single cell. Twelve clone cells were isolated from each of the single cell. Using aliquots of these clone cells, a similar binding experiment was carried out. As a result, clone cell No. 3-10 exhibited the highest binding activity to pig ¹²⁵I-galanin, and was believed to have the highest GALR1 expression level. Thus, this clone was used in the subsequent experiments as the GALR1 expressing cell.

### (1-2) Preparation of Membrane Fraction of Galanin Receptor (GALR1, GALR2) Expressing Cell

The GALR2 expressing cells obtained in (1-1) above were cultured to sub-confluence (80-90% confluence) in Dulbecco's modified Eagle medium containing 10% fetal calf serum, glutamine, penicillin and streptomycin. The cultured cells were liberated from the culture vessel by adding thereto 2.7 mM EDTA-containing phosphate buffer [2.7rnM EDTA/Phosphate-buffer saline (138 mM NaCl 2,7 mM KCl, 8 mM Na₂HPO, 1 mM KH₂PO₄)] and suspending the cells. Then, the cells were recovered by remote operation. The cells were homogenized in 10 mM sodium hydrogencarbonate, 5 mM EDTA (pH 7.3) buffer containing a protease inhibitor mixture (final concentrations of individual components are 0.5 mM phenylmethylsulphonyl fluoride, 20 µg/ml leupeptin, 4 µg/ml E-64, 10 µg/ml pepstatin) using a Polytron homogenizer. The resultant homogenate was centrifuged at 2500 rpm for 10 min in a high speed centrifuge (CR26H, RR24A Type Rotor; Hitachi). The resultant supernatant was centrifuged at 30,000 rpm for 1 hr in an ultra-centrifuge (SCP70H, RP42 Type Rotor; Hitachi) to obtain a precipitate. This precipitate was re-suspended in 10 mM sodium hydrogencarbonate, 5 mM EDTA (pH 7.3) buffer containing a protease inhibitor mixture (0.5 mM phenylmethylsulphonyl fluoride, 20 µg/ml leupeptin, 4 µg/ml E-64, 10 µg/ml pepstatin) to thereby prepare a membrane fraction for detecting galanin receptor activating effect (stored at -70ºC).

### (1-3) Detection of Galanin Receptor Activating Effect by [³⁵S]GTPγS Binding Assay

The membrane fraction prepared in (1-2) above was suspended in 50 mM Tris buffer (pH 7.4) containing 1 µM guanosine-5'-diphosphate (GDP), 0.1% bovine serum albumin (BSA), 5 mM MgCl₂ and 150 mM NaCl to give a concentration of 40 µg/ml (for GALR1 membrane fraction) or 32 µg/ml (for GALR2 membrane fraction). The resultant suspension was dispensed into small polypropylene test tubes (Falcon 2053) at 0.2 ml/tube. To the dispensed membrane fraction in each tube, 2 µl of 50 nM [³⁵S]GTPγS (New England Nuclear) and 2 µl of pig galanin as an agonist (at a concentration of 100 µM, 10 µM, 1 µM, 100 nM, 10 nM, 1 nM, 100 pM or 10 pM) were added and reacted at 25ºC for 60 min. To this reaction solution, 1.5 ml of 50 mM Tris buffer (pH 7.4) containing 0.05% 3-[(3-cholamidopropyl)dimethylammonio]-1-propanesulfonate (CHAPS), 0.1% BSA, 5 mM MgCl₂ and 1 mM EDTA was added and filtered with GF/F glass fiber filter paper (Whatman). This filter paper was washed with 1.5 ml of the above-described buffer and dried. Then, the radioactivity was measured with a liquid scintillation counter.

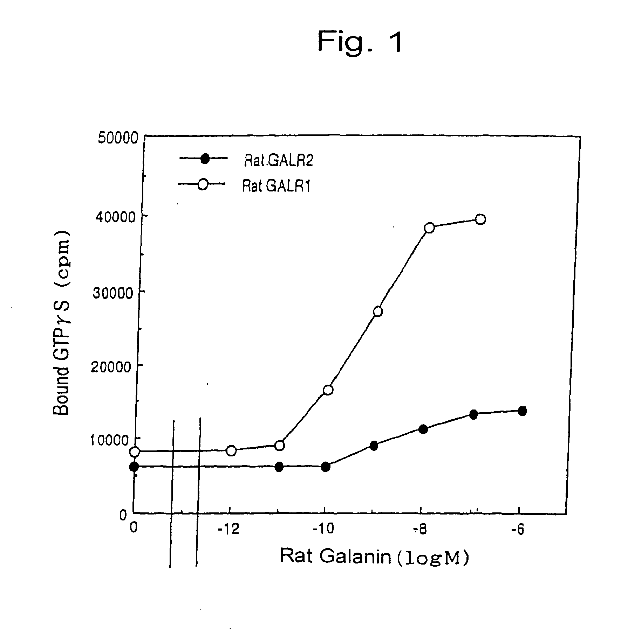

As a result, it was observed that the amount of [³⁵S]GTPγS binding increased in a galanin concentration dependant manner (Fig. 1). Thus, it has become clear that the agonist activity of a galanin receptor can be measured by this method.

The EC₅₀ value of rat galanin (the concentration that gives 50% of the maximum binding amount) was about 3 nM for GALR1 and about 10 nM for GALR2.

### REFERENCE EXAMPLE 2:

### Screening for Compounds that Activate GALR2 (Galanin Receptor Type 2)

### (2-1) Preparation of Pig Hypothalamus Extract

Pig hypothalami were purchased at Tokyo Shibaura Zohki Co., Ltd., and the operations described below were performed within 3 hours to prepare an extract.

First, 35 pig hypothalami (about 500 g) were minced with a kitchen knife, put into a 3000 ml beaker containing 1250 ml of boiling distilled water, and boiled for 10 min. The beaker containing the boiled hypothalami was placed in a water bath and then in an ice bath to lower the temperature to about 4ºC. Subsequently, the hypothalami were homogenized, together with the distilled water used for boiling, for 10 min in a Polytron Homogenizer. To the resultant homogenate, 90 ml of acetic acid was added dropwise to give a final concentration of 1 M and agitated for 1 hr. The homogenate was centrifuged at 10,000 rpm for 30 min in a high speed centrifuge (CR26H, RR10A Type Rotor; Hitachi) to obtain supernatant (1). On the other hand, the precipitate obtained on centrifugation was re-homogenized in a Polytron Homogenizer for 10 min after addition of 2000 ml of 1 M acetic acid. The resultant homogenate was agitated overnight (about 16 hr) with agitation blades, followed by homogenization in a Polytron Homogenizer for 10 min to thereby obtain supernatant (2). Supernatants (1) and (2) were mixed. Two volumes of acetone was added thereto and agitated at 4ºC for 1 hr. Subsequently, the resultant mixture was centrifuged at 10,000 rpm for 15 min in a high speed centrifuge (CR26H, RR10A Type Rotor; Hitachi) to obtain a supernatant. This supernatant was treated with a rotary evaporator to remove acetone and to concentrate to a final volume of 4000 ml. This concentrated solution was centrifuged at 35,000 rpm for 1 hr in an ultra-centrifuge (SCP70H, Hitachi RPZ35T Type Rotor; Hitachi) to obtain a clear supernatant. Each of 1000 ml aliquots of the resultant supernatant was mixed with 500 ml of diethyl ether vigorously in a separating funnel. An aqueous phase was obtained as a result of two-phase separation. This aqueous phase was concentrated up to 1000 ml using a rotary evaporator to thereby obtain a final extract.

### (2-2) Purification of Pig Hypothalamus Extract by Octadodecyl Reversed Phase Chromatography

Silica gel ODS-AM 120-S50 in which octadodecyl groups are fixed (YMC Co., Ltd.) was swollen with methanol, packed in a glass column (5 cm in diameter) to give a volume of 130 ml, and equilibrated with 1 M acetic acid. To this column, the extract (obtained from 500 g of hypothalami) prepared in (2-1) above was fed at a flow rate of 400 ml/hr for adsorption. Subsequently, about 500 ml of 1 M acetic acid, and then about 500 ml of 20% acetonitrile/0.1% trifluoroacetic acid were fed to this column at a flow rate of 400 ml/l to wash the gel. Finally, about 500 ml of 50% acetonitrile/0.1% trifluoroacetic acid was fed to this column at a flow rate of 400 ml/l to elute the crude peptide component of interest. The resultant eluate was concentrated with an evaporator and lyophilized with a lyophilizer (Model 12EL; VirTis).

### (2-3) Purification of Pig Hypothalamus Extract by TSKgel Reversed Phase High Performance Liquid Chromatography

TSKgel ODS80^{TM} reversed phase high performance liquid chromatography column (Tosoh; 22.5 mm x 30 cm) was equilibrated by feeding thereto Solution A (0.1% trifluoroacetic acid/distilled water) at 40ºC at a flow rate of 8 ml/min. The lyophilized product obtained in (2-2) above was dissolved in 40 ml of 1 M acetic acid. This solution was divided into four 10 ml samples, with which chromatographic operations were carried out four times. Briefly, a 10 ml sample of the acetic acid solution of the lyophilized product was fed and adsorbed onto the column, and then elution was performed with a linear gradient from 80% Solution A (0.1% trifluoroacetic acid/distilled water)/20% Solution B (0.1% trifluoroacetic acid/100% acetonitrile) (v/v) to 40% Solution A (0.1% trifluoroacetic acid/distilled water)/60% Solution B (0.1% trifluoroacetic acid/100% acetonitrile) (v/v) at a flow rate of 8 ml/min over 60 min.

The eluate was recovered as serially numbered fractions each consisting of 8 ml. Each fraction was divided into 1 ml samples, which were then concentrated and dried in a vacuum condenser (Savant). Dimethyl sulfoxide (0.03 ml) was added to each of the resultant dried products to dissolve it to thereby prepare an assay sample for measuring GALR1 and GALR2 activating effect.

### (2-4) Isolation and Purification of Compounds that Activate GALR2 (Galanin Receptor Type 2) by [³⁵S]GTPγS Binding Assay

The galanin receptor activating effect of the assay sample obtained in (2-3) above was measured by [³⁵S]GTPγS binding assay using the GALR1 or GALR2 membrane fraction described in (1-3) above.

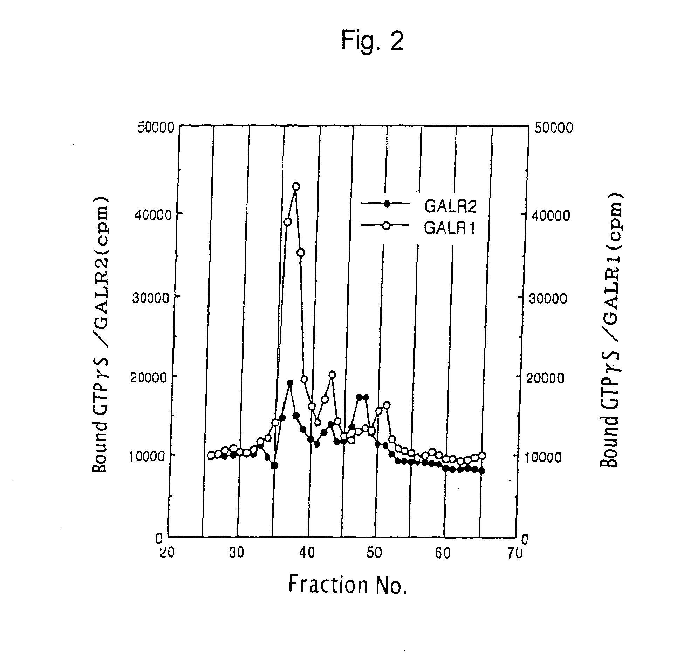

In the [³⁵S]GTPγS binding assay using the GALR1 expressing cell membrane fraction, [³⁵S]GTPγS binding promotion activity was recognized in fractions Nos. 36-39 and 42-43; on the other hand, in the [³⁵S]GTPγS binding assay using the GALR2 expressing cell membrane fraction, [³⁵S]GTPγS binding promotion activity was recognized in fractions Nos. 36-39, 42-43 and 46-49 (Fig. 2).

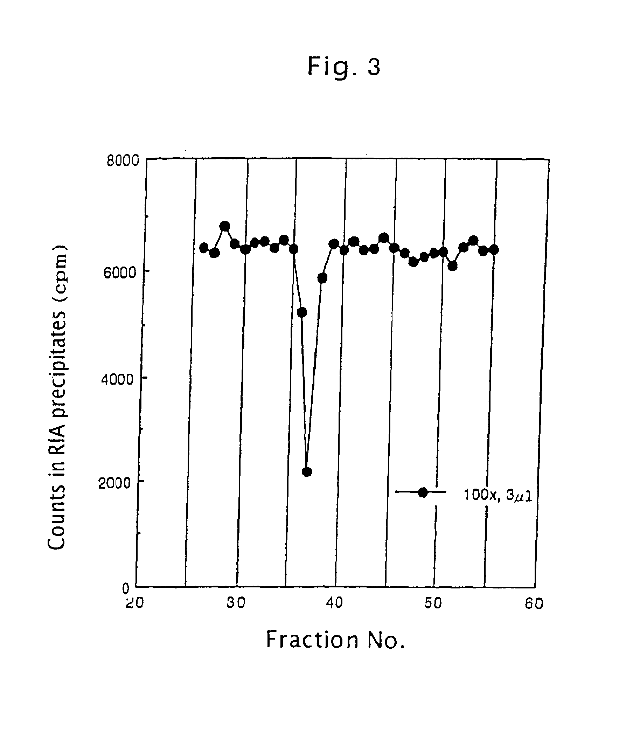

Further, the assay sample was diluted 100-fold with methyl sulfoxide, and 3 ml of this diluted sample was analyzed with Pig Galanin Radioimmunoassay Kit (Peninsula). As a result, galanin immunoactivity was detected in fractions Nos. 36-38 (Fig. 3).

From the above results, it was judged that the component having GALR1 and GALR2 activating effect in fractions Nos. 36-39 is a pig galanin. On the other hand, it was predicted that the component having GALR2 activating effect in fractions Nos. 46-49 is a compound different from galanin.

### (2-5) Measurement of the Molecular Weight of the Compound that Activates GALR2 (Galanin Receptor Type 2)

The molecular weight of the GALR2 activating component (contained in fractions Nos. 46-49) obtained in (2-4) above by conventional gel filtration high performance liquid chromatography.

Briefly, 0.02 ml of a sample mixture composed of fractions Nos. 46-49 was diluted with 0.08 ml of 0.1 % trifluoroacetic acid/distilled water. Then, 0.05 ml of this diluted sample was fed to a G2000SWXL (Tosoh) column for adsorption, and 0.1% trifluoroacetic acid/10% acetonitrile was fed thereto at a flow rate of 0.5 ml/min. The eluate was recovered as 0.25 ml fractions (every 0.5 min). The recovered fractions were concentrated and dried (SpeedVac Plus SC210A; Savant), and dissolved directly in the suspension of the membrane fraction. Subsequently, 2 µl of 50 nM [³⁵S]GTPγS (New England Nuclear) was added thereto, followed by measurement of the amount of [³⁵S]GTPγS binding by the method described in (1-3) above.

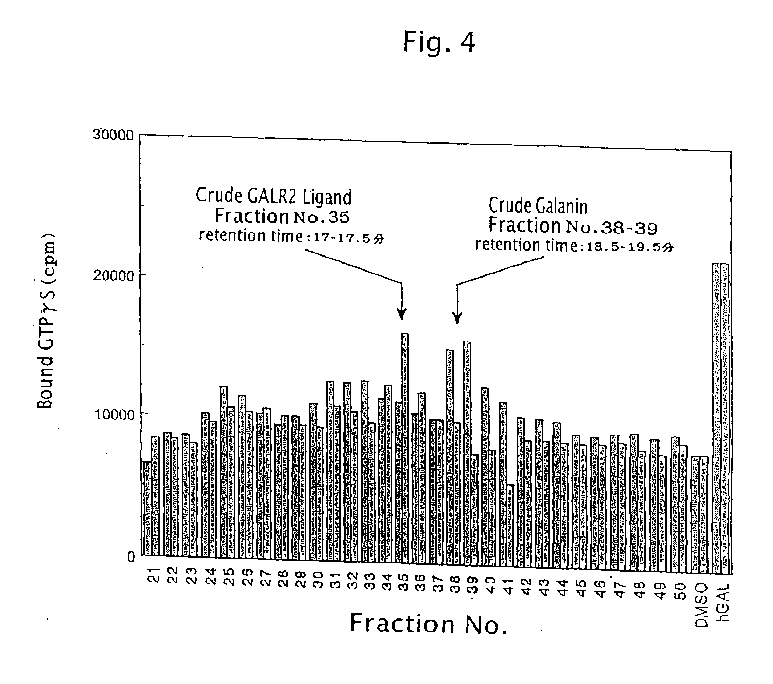

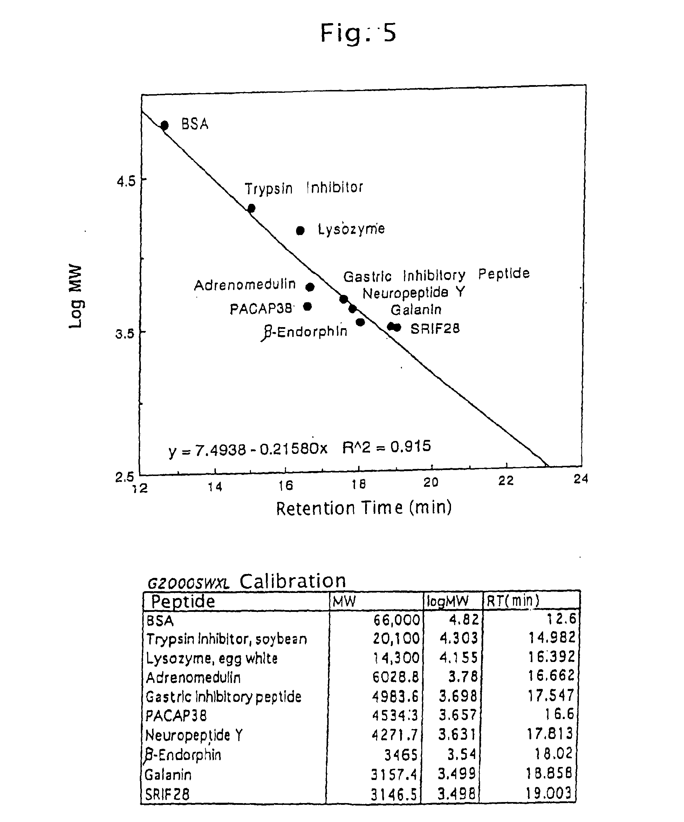

As a result, it was found that the component having GALR2 activating effect ([³⁵S]GTPγS binding promotion activity) was eluted in fraction No. 35 (retention time: 17-17.5 min) (Fig. 4). From the results of comparative analysis of known peptides (adrenomedulin, gastric inhibitory peptide, PACAP38, neuropeptide Y, β-endorphine, galanin, SRIF28 (somatostatin 28), bovine serum albumin, trypsin inhibitor, and lysozyme) under the same conditions (Fig. 5), it was estimated that the molecular weight of the component having GALR2 activating effect in fraction No. 46-49 is from about 5000 to about 7000.

On the other hand, the molecular weight of a known pig galanin (Peptide Institute Inc.) is 3157.4 (retention time: 18.858 min). Therefore, it was suggested that the compound having GALR2 activating effect obtained as fractions Nos. 46-49 is a substance different from the known galanin.

### REFERENCE EXAMPLE 3:

### Purification of the Compound Having GALR2 (Galanin Receptor Type 2) Activating Effect

### (3-1) Preparation of Pig Hypothalamus Extract

In order to prepare a large quantity of hypothalamus extract, the method described in (2-1) above was simplified as described below.

Fifty frozen pig hypothalami (about 1 kg) (Tokyo Shibaura Zohki Co., Ltd.) were sliced into thin sections with a kitchen knife, put into a 5000 ml beaker containing 2500 ml of boiling distilled water, and boiled for 10 min. The beaker containing the boiled hypothalami was placed in a water bath and then in an ice bath to lower the temperature to about 4°C. The hypothalami together with the distilled water used for boiling was homogenized for 10 min in a Polytron Homogenizer. To the resultant homogenate, 150 ml of acetic acid and 8 ml of 6 N HCl were added dropwise to give the final concentrations 1 M and 20 mM, respectively. Then, the homogenate was agitated with agitation blades overnight (about 16 hr). The resultant homogenate was centrifuged at 8000 rpm for 30 min with a high speed centrifuge (CR26H, Hitachi RR10A Type Rotor; Hitachi) to obtain a supernatant, which was filtered with a gauze to remove lipid sections. The above-described operations were performed 4 times to thereby prepare extract from about 4 kg of hypothalami.

### (3-2) Concentration of Pig Hypothalamus Extract and Crude Fractionation

### (3-2-1) Purification by Octadodecyl Reversed Phase Chromatography

Silica gel in which octadodecyl groups are fixed (YMC Co., Ltd.; ODS-AM 120-S50) was swollen with methanol, packed in a glass column (5 cm in diameter) to give a volume of 400 ml, and equilibrated with 1 M acetic acid.

To this column, one half of the extract (from 2 kg of hypothalami) prepared in (3-1) above was fed at a flow rate of 400 ml/hr for adsorption. Subsequently, about 1000 ml of 1 M acetic acid, and then about 1200 ml of 20% acetonitrile/0.1% trifluoroacetic acid were fed to the column at a flow rate of 400 ml/l to wash the gel. Finally, about 2000 ml of 60% acetonitrile/0.1 % trifluoroacetic acid was fed to the column at a flow rate of 400 ml/hr to elute the crude peptide fraction of interest. The resultant eluate was concentrated with an evaporator and lyophilized with a lyophilizer (Model 12EL; VirTis). The above-described operations were performed twice to thereby prepare lyophilized powder of the extract from about 4 kg of hypothalami (200 hypothalami).

### (3-2-2) Purification by SP-Sephadex Ion Exchange Chromatography

SP-Sephadex C25 (Pharmacia Biotech) that had been swollen in 10 mM HCl was packed in a glass column (5 cm in diameter) to give a volume of 120 ml, washed with 100 mM HCl and equilibrated with 1 M acetic acid. The lyophilized powder of hypothalamus extract obtained in (3-2-1) was dissolved in about 800 ml of 1 M acetic acid and fed to the equilibrated SP-Sephadex C25 column at a flow rate of 400 ml/hr for adsorption. About 600 ml of 1 M acetic acid, about 600 ml of 1 M pyridine and about 600 ml of 1 M pyridine/acetic acid (pH 5.0) were fed to the column in order at a flow rate of 400 ml/hr. The eluate was recovered as fractions each consisting of 40 ml. Each fraction was divided into 0.2 ml samples, which were then concentrated and dried in a vacuum condenser (Savant). Each of the resultant dry products was dissolved in 0.04 ml of dimethyl sulfoxide, and used for measuring GALR2 activating effect according to the test method described in (1-3) above.

As a result, it was found that the component of interest having GALR2 activating effect has been eluted by 1 M pyridine/acetic acid (pH 5.0). From this result, it is believed that the component (compound) having GALR2 activating effect is a strongly basic substance.

### (3-2-3) Purification by Sephadex G50 Gel Filtration Chromatography

The fraction of 1 M pyridine/acetic acid (pH 5.0) eluate obtained in (3-2-2) above was concentrated to 100 ml in an evaporator, and fed to Sephadex G50 (Pharmacia Biotech) column (6 cm in diameter, 3000 ml in volume) equilibrated with 1 M acetic acid at a flow rate of 400 ml/hr for adsorption. Then, 1 M acetic acid was fed to the column at a flow rate of 400 ml/hr. The eluate was recovered as serially numbered fractions each consisting of 33 ml. Each fraction was divided into 0.25 ml samples, which were then concentrated and dried in a vacuum condenser (Savant). Each of the resultant dry products was dissolved in 0.03 ml of dimethyl sulfoxide, and used for measuring GALR2 activating effect according to the test method described in (1-3) above.

As a result, it was found that the component of interest having GALR2 activating effect has been eluted mainly into eluate fractions Nos. 66-80. A weak activity was also detected in fractions Nos. 55-65 and 45-54. Fractions Nos. 66-80, 55-65 and 45-54 were mixed together, respectively, and lyophilized.

### (3-3) Purification of the Compound Having GALR2 Activating effect by HPLC

### (3-3-1) Purification by TSKgel ODS80^{TM} Reversed Phase High Performance Liquid Chromatography

TSKgel ODS80™ reversed phase high performance liquid chromatography column (Tosoh; 22.5 mm x 30 cm) was equilibrated by feeding thereto Solution A (0.1% trifluoroacetic acid/distilled water) at 40ºC at a flow rate of 4 ml/min.

Each of the lyophilized products of Sephadex G50 eluate fractions obtained in (3-2-3) above was dissolved in 1 M acetic acid and fed to the column for adsorption. Elution was performed with a linear gradient from 67% Solution A (0.1% trifluoroacetic acid/distilled water)/33% Solution B (0.1% trifluoroacetic acid/60% acetonitrile) (v/v) to 100% Solution B (0.1% trifluoroacetic acid/60% acetonitrile) at a flow rate of 4 ml/min over 120 min. The eluate was recovered.

The eluate was recovered as serially numbered fractions each consisting of 8 ml. The lyophilized product from fractions Nos. 66-80 was divided into 2 samples before application to the chromatography. On the other hand, the lyophilized product from fractions Nos. 55-65 and that from fractions Nos. 45-54 were separated by the chromatography at one time, respectively.

Using 4 µl of the recovered fraction directly, GALR2 activating effect was measured by the test method described in (1-3) above. As a result, mainly three active peaks were observed in any of the chromatographic separations performed. The elution point of the component having the activating effect found in fractions Nos. 35-38 was almost identical to the elution point of the galanin described in Reference Example 2. Further, the component having the activating effect found in fractions Nos. 47-50 has an elution point almost identical to the elution point of the component having GALR2 activating effect described in Reference Example 2 (which does not activate GALR1, and is not detected with the above-mentioned Pig Galanin Radioimmunoassay Kit). Therefore, the inventors intended to purify the component having GALR2 activating effect using fractions Nos. 47-50. These fractions were mixed and lyophilized.

### (3-3-2) Purification by TSKgel CM-2SW Ion Exchange High Performance Liquid Chromatography

TSKgel CM-2SW high performance liquid chromatography column (Tosoh; 0.46 cm x 25 cm) was equilibrated by feeding thereto Solution A (10 mM ammonium formate/40% acetonitrile) at 20ºC at a flow rate of 1 ml/min.

The lyophilized product of the reversed phase high performance liquid chromatography eluate fractions obtained in (3-3-1) above was dissolved in Solution A and fed to the column for adsorption. Elution was performed with a linear gradient from 100% Solution A (10 mM ammonium formate/40% acetonitrile) (v) to 100% Solution B (500 mM ammonium formate/40% acetonitrile) (v) at a flow rate of 1 ml/min over 60 min. The eluate was recovered.

The eluate was recovered as serially numbered fractions each consisting of 0.5 ml. Using 1 µl of the recovered fraction directly, GALR2 activating effect was measured by the test method described in (1-3) above. As a result, a major active peak was detected in fractions Nos. 89-92. These fractions were collected together and used in the subsequent purification.

### (3-3-3) Purification by Diphenyl Reversed Phase High Performance Liquid Chromatography

Diphenyl reversed phase high performance liquid chromatography column (Vydak 219TP54; 0.46 cm x 25 cm) was equilibrated by feeding thereto Solution A (0.1% trifluoroacetic acid/distilled water) at 40ºC at a flow rate of 1 ml/min.

The ion exchange high performance liquid chromatography eluate fractions obtained in (3-3-2) above were fed to the column directly for adsorption. Then, elution was performed as follows. Starting with 100% Solution A (0.1% trifluoroacetic acid/distilled water) (v), the volume of Solution B (0.1% trifluoroacetic acid/60% acetonitrile) was rapidly increased to 50% in 1 min at a flow rate of 1 ml/min; subsequently, the concentration of Solution B was increased to 100% with a linear gradient at a flow rate of 1 ml/min over 60 min. The eluate was recovered.

The eluate was recovered as serially numbered fractions each consisting of 1 ml. Using 1 µl of each of the recovered fractions directly, GALR2 activating effect was measured by the test method described in (1-3) above. As a result, a major active peak was detected in fractions Nos. 24-26. These fractions were collected together and used in the subsequent purification.

### (3-3-4) Purification by Super Phenyl Reversed Phase High Performance Liquid Chromatography

TSKgel Super Phenyl reversed phase high performance liquid chromatography column (Tosoh; 0.46 cm x 10 cm) was equilibrated by feeding thereto Solution A (0.1% trifluoroacetic acid/distilled water) at 40ºC at a flow rate of 1 ml/min.

The diphenyl reversed phase high performance liquid chromatography eluate fractions obtained in (3-3-3) above were fed to the column directly for adsorption. Then, elution was performed as follows. Starting with 100% Solution A (0.1% trifluoroacetic acid/distilled water) (v), the volume of Solution B (0.1% trifluoroacetic acid/60% acetonitrile) was rapidly increased to 45% in 1 min at a flow rate of 1 ml/min; subsequently, the concentration of Solution B was increased to 55% with a linear gradient at a flow rate of 1 ml/min over 80 min. The eluate was recovered.

The eluate was recovered as serially numbered fractions each consisting of 1 ml. Using 1 µl of the recovered fraction directly, GALR2 activating effect was measured by the test method described in (1-3) above. As a result, a major active peak was detected in fractions Nos. 44-45 and Nos. 50-52. Fractions Nos. 44-45 were mixed together and again subjected to the chromatography under the same conditions, followed by recovery of the eluate as 0.5 ml fractions. On the other hand, fraction No. 50, No. 51 and No. 53 were again subjected to the chromatography separately under the same conditions, followed by recovery of the eluate as 0.5 ml fractions.

Using 2 µl of each of the recovered fractions directly, GALR2 activating effect was measured by the test method described in (1-3) above. As a result, an active peak was detected in fractions Nos. 93-95 that were obtained from the second chromatographic separation of fractions Nos. 44-45, and also in fractions Nos. 105-107 that were obtained from the second chromatographic separation of fraction No. 50. A major active peak was detected in fractions Nos. 105-107 that was obtained from the second chromatographic separation of fraction No. 51; and a weak active peak was detected in fractions Nos. 108-109. Further, in fractions Nos. 106-110 obtained by the second chromatographic separation of fraction No. 52, a major active peak was detected broadly.

### (3-3-5) Purification by Super ODS Reversed Phase High Performance Liquid Chromatography

### (3-3-5-1) Purification of the GALR2-Activating Major Component

### (3-3-5-1-1) Chromatography in the Presence of Trifluoroacetic Acid

TSKgel Super ODS reversed phase high performance liquid chromatography column (Tosoh; 0.46 cm x 10 cm) was equilibrated by feeding thereto Solution A (0.1% trifluoroacetic acid/distilled water) at 40ºC at a flow rate of 1 ml/min.

The Super Phenyl reversed phase high performance liquid chromatography eluate fractions Nos. 93-95 obtained in (3-3-4) above were fed to the column directly for adsorption. Elution was performed as follows. Starting with 100% Solution A (0.1% trifluoroacetic acid/distilled water) (v), the volume of Solution B (0.1% trifluoroacetic acid/60% acetonitrile) was increased to 50% with a linear gradient at a flow rate of 1 ml/min in 1 min; subsequently, the concentration of Solution B was increased to 62.5% at a flow rate of 1 ml/min over 84 min. The eluate was recovered.

The eluate was recovered as serially numbered fractions each consisting of 0.5 ml. Using 2 µl of the recovered fraction directly, GALR2 activating effect was measured by the test method described in (1-3) above. As a result, an active peak was detected in fractions Nos. 96-97.

The resultant fractions Nos. 96-97 were mixed together and subjected again to the chromatography under the same conditions, followed by recovery of the eluate as 0.5 ml fractions. Although the peak detected by UV absorption at 210 nm was single, peak shoulders were recognized which suggest the mixing of impurities. Using 2 µl of each of the recovered fractions directly, GALR2 activating effect was measured by the test method described in (1-3) above. As a result, an active peak was detected in fractions Nos. 98-100. These peaks were consistent with the peak (except for the peak shoulder portions) detected by UV absorption at 210 nm. In order to remove the impurities contained in the peak shoulders, further purification was performed according to the method described in (3-3-5-1-2) below.

### (3-3-5-1-2) Chromatography in the Presence of Heptafluorobutyric Acid

TSKgel Super ODS reversed phase high performance liquid chromatography column (Tosoh; 0.46 cm x 10 cm) was equilibrated by feeding thereto Solution A (0.1% heptafluorobutyric acid/distilled water) at 40ºC at a flow rate of 1 ml/min.

Fractions Nos. 98-100 obtained in (3-3-5-1-1) above were fed to the column directly for adsorption. Elution was performed as follows. Starting with 100% Solution A (0.1% heptafluorobutyric acid/distilled water) (v), the volume of Solution B (0.1% heptafluorobutyric acid/100% acetonitrile) was increased rapidly to 35% at a flow rate of 1 ml/min in 1 min; subsequently, the concentration of Solution B was increased to 50% with a linear gradient at a flow rate of 1 ml/min over 60 min. The eluate was recovered.

The eluate was recovered as serially numbered fractions each consisting of 0.5 ml. Using 2 µl of the recovered fraction directly, GALR2 activating effect was measured by the test method described in (1-3) above. As a result, an active peak was detected in fractions Nos. 67-69. This peak was completely consistent with the earlier peak of the separated two UV absorption peaks at 210 nm. Thus, it was judged that the component of interest has been purified to a single peptide. The component having this activating effect is designated the "GALR2 activating major component".

### (3-3-5-2) Purification of GALR2-Activating Sub-component (1)

TSKgel Super ODS reversed phase high performance liquid chromatography column (Tosoh; 0.46 cm x 10 cm) was equilibrated by feeding thereto Solution A (0.1% heptafluorobutyric acid/distilled water) at 40ºC at a flow rate of 1 ml/min.

Super Phenyl reversed phase high performance liquid chromatography eluate fractions Nos. 105-107 (derived from fraction No. 50), fractions Nos. 105-107 (derived from fraction No. 51) and fraction Nos. 106-107 (derived from fraction No. 52) obtained in (3-3-4) above were mixed together and fed to the column directly for adsorption. Elution was performed as follows. Starting with 100% Solution A (0.1% trifluoroacetic acid/distilled water) (v), the volume of Solution B (0.1% trifluoroacetic acid/60% acetonitrile) was increased rapidly to 52.5% at a flow rate of 1 ml/min in 1 min; subsequently, the concentration of Solution B was increased to 65% with a linear gradient at a flow rate of 1 ml/min over 84 min. The eluate was recovered as serially numbered 0.5 ml fractions.

Using 2 µl of the recovered fraction directly, GALR2 activating effect was measured by the test method described in (1-3) above. As a result, an active peak was detected in fractions Nos. 75-76. These fractions were mixed together and subjected again to the same chromatography under the same conditions, followed by recovery of the eluate as serially numbered 0.5 ml fractions. A single peak was detected when the UV absorption at 210 nm of the eluate was examined. Using 3 µl of the recovered fraction directly, GALR2 activating effect was measured by the test method described in (1-3) above. As a result, an active peak was detected in fractions Nos. 76-78. This peak was consistent with the UV absorption peak at 210 nm. Thus, it was judged that the active component has been purified to a single peptide. The component having this activating effect is designated "GALR2 activating sub-component (1)".

### (3-3-5-3) Purification of GALR2 Activating Sub-component (2)

TSKgel Super ODS reversed phase high performance liquid chromatography column (Tosoh; 0.46 cm x 10 cm) was equilibrated by feeding thereto Solution A (0.1% heptafluorobutyric acid/distilled water) at 40ºC at a flow rate of 1 ml/min.

Super Phenyl reversed phase high performance liquid chromatography eluate fractions Nos. 108-109 (derived from fraction No. 51) and fractions Nos. 108-110 (derived from fraction No. 52) obtained in (3-3-4) above were mixed together and fed to the column directly for adsorption. Elution was performed as follows. Starting with 100% Solution A, the volume of Solution B (0.1% trifluoroacetic acid/60% acetonitrile) was increased rapidly to 52.5% at a flow rate of 1 ml/min in I min; subsequently, the concentration of Solution B was increased to 65% with a linear gradient at a flow rate of 1 ml/min over 84 min. The eluate was recovered as serially numbered 0.5 ml fractions.

Using 2 µl of the recovered fraction directly, GALR2 activating effect was measured by the test method described in (1-3) above. As a result, an active peak was detected in fractions Nos. 79-80.

The resultant fractions Nos. 79-80 were mixed together and subjected again to the same chromatography under the same conditions, followed by recovery of the eluate as serially numbered 0.5 ml fractions. A single peak was detected when the UV absorption at 2210 nm of the eluate was examined.

Using 3 µl of the recovered fraction directly, GALR2 activating effect was measured by the test method described in (1-3) above. As a result, an active peak was detected in fractions Nos. 79-81. This peak was consistent with the UV absorption peak at 210 nm. Thus, it was judged that the active component has been purified to a single peptide. The component having this activating effect is designated "GALR2-activating sub-component (2)".

### REFERENCE EXAMPLE 4:

### Amino Acid Sequence Analysis

### (4-1) Analysis of the N-Terminal Amino Acid Sequence of the Major Component Having GALR2 Activating Effect

The GALR2 activating major component in fractions Nos. 67-69 obtained in (3-3-5-1-2) above was dissolved in 20 µl of 0.1% trifluoroacetic acid/30% acetonitrile and added dropwise to a peptide support disk (Beckman), which was then dried with nitrogen gas. This disk was set in a protein sequencer (Model LF3400DT; Beckman), and an automated Edman degradation reaction was performed according to the standard analysis method (Procedure 40) to analyze the amino acid sequence. The PTH amino acids identified in each cycle are shown in Table 1.

**Table 1:**

| Analysis of N-terminal amino acid sequence of GALR2-activating major component | | |
|---|---|---|
| cycle No. | PTH-Amino Acid | Amount (pmol) |
| 1 | A | 131 |
| 2 | P | 89.9 |
| 3 | V | 138 |
| 4 | H | 81.7 |
| 5 | R | 47.3 |
| 6 | G | 92.1 |
| 7 | R | 45.6 |
| 8 | G | 72.9 |
| 9 | G | 78.2 |
| 10 | W | 24.1 |
| 11 | T | 24.0 |
| 12 | L | 51.5 |
| 13 | N | 34.0 |
| 14 | S* | 9.3 |
| 15 | A | 25.4 |
| 16 | G | 26.8 |
| 17 | Y | 24.6 |
| 18 | L | 32.4 |
| 19 | L | 43.5 |
| 20 | G | 24.0 |
| 21 | P | 14.6 |
| 22 | V | 17.2 |
| 23 | L | 26.4 |
| 24 | H | 1.4 |
| 25 | P | 16.1 |
| 26 | P | 22.6 |
| 27 | S* | 2.9 |
| 28 | X | |
| 29 | A | 11.1 |
| 30 | E | 3.8 |
| 31 | G | 13.0 |
| 32 | G | 19.6 |
| 33 | G | 20.4 |
| 34 | K | 4.4 |

| | | |
|---|---|---|
| 1) Raw Data, uncorrected for lag. 2) S*: In addition to PTH-Ser, the degradation product thereof (PTH-dehydroalanin-DTT addition) was detected. 3) At the 28th cycle, no PTH-amino acid was identified. | | |

From Table 1, the amino acid sequence from the N-terminus to the 34th residue of the GALR2-activating major component could be determined as described below ("X" at the 28th residue means that this residue is unidentified). APVHRGRGGWTLNSAGYLLGPVLHPPSXAEGGGK (N-terminal amino acid sequence of GALR2-activating major component: SEQ ID NO: 11)

A partial amino acid sequence consisting of the 13 residues from the 9th residue to the 21st residue (counted from the N-terminus) in the above-described amino acid sequence was identical with the N-terminal amino acid sequence of a known pig galanin. However, the amino acid sequences before and after this partial sequence were not identical with the amino acid sequence of the pig galanin or its precursors. Further, since no known peptides were found that have an amino acid sequence identical to this amino acid sequence, the thus obtained GALR2 activating major component was presumed to be a novel peptide.

### (4-2) Analysis of the N-Terminal Amino Acid Sequence of the Sub-component (1) Having GALR2-Activating Effect

The GALR2-activating sub-component in fractions Nos. 76-78 obtained in (3-3-5-2) above was analyzed in the same manner as described in (4-1) above. The amino acids identified in each cycle are shown in Table 2.

**Table 2:**

| Analysis of N-terminal amino acid sequence of Sub-component (1) | | |
|---|---|---|
| cycle No. | PTH-Amino Acid | Amount (pmol) |
| 1 | A | 50.7 |
| 2 | P | 36.7 |
| 3 | V | 51.3 |
| 4 | H | 31.4 |
| 5 | R | 18.8 |
| 6 | G | 38.9 |
| 7 | R | 17.2 |
| 8 | G | 32.2 |
| 9 | G | 32.5 |
| 10 | W | 9.7 |
| 11 | T | 9.2 |
| 12 | L | 19.0 |
| 13 | N | 13.9 |
| 14 | S* | 3.6 |
| 15 | A | 12.2 |
| 16 | G | 12.0 |
| 17 | Y | 10.9 |
| 18 | L | 15.0 |
| 19 | L | 18.7 |
| 20 | G | 9.9 |
| 21 | P | 6.8 |
| 22 | V | 7.5 |
| 23 | L | 11.4 |
| 24 | H | - ** |
| 25 | P | 7.0 |
| 26 | P | 9.7 |
| 27 | S* | 1.2 |
| 28 | X | |
| 29 | A | 5.3 |
| 30 | E | 2.3 |
| 31 | G | 6.4 |
| 32 | G | 7.4 |
| 1) Raw Data, uncorrected for lag. | | |
| 2) S*: In addition to PTH-Ser, the degradation product thereof (PTH-dehydroalanin-DTT addition) was detected. | | |
| 3) At the 24th cycle, the peak of PTH-His was observed, but below detection limit | | |
| 4) At the 28th cycle, no PTH-amino acid was identified. | | |

From Table 2, the amino acid sequence from the N-terminus to the 32nd residue of the GALR2 activating sub-component (1) could be determined as described below ("X" at the 28th residue means that this residue is unidentified). APVHRGRGGWTLNSAGYLLGPVLHPPSXAEGG (N-terminal amino acid sequence of GALR2-activating sub-component (1): SEQ ID NO: 12)

This amino acid sequence was completely identical with the sequence of the N-terminal 32 residues of the GALR2-activating major component obtained in (4-1) above.

### (4-3) Analysis of the N-Terminal Amino Acid Sequence of the Sub-component (2) Having GALR2-Activating Effect

The GALR2-activating sub-component (2) in fractions Nos. 79-81 obtained in (3-3-5-3) above was dissolved in 20 µl of 0.1% trifluoroacetic acid/30% acetonitrile. To this solution, ammonium bicarbonate (final concentration 1%), TLCK-chymotrypsin (final concentration 10 pmol; Sigma) and distilled water were added to give a 50 µl solution and reacted at 37°C for 1 hr.

Spheri-5 RP-18 reversed phase high performance liquid chromatography column (Brownlee; 2.1 mm x 30 mm) was equilibrated by feeding thereto Solution A (0.1% trifluoroacetic acid) at 25°C at a flow rate of 300 µl/min.

The total volume of the above enzyme reaction solution was fed to the column for adsorption. Then, elution was performed as follows. Starting with 100% Solution A (0.1% trifluoroacetic acid), the volume of Solution B (0.1% trifluoroacetic acid/70% acetonitrile) was increased to 70% at a flow rate of 300 µl/min over 30 min, to thereby recover 4 elution peaks (CHY-1, CHY-2, CHY-3 and CHY-4) exhibiting UV absorption at 210 nm as elution fractions.

The thus recovered 4 fractions (chymotrypsin-digested fragments) were individually concentrated to 50 µl or less under nitrogen gas flow. Then, the N-terminal amino acid sequences were analyzed with protein sequencers in the same manner as described in (4-1) above. For CHY-3, LF3400DT protein sequencer (Beckman) was used. For CHY-1, CHY-2 and CHY-4, 477A protein sequencer (Applied Biosystems) was used.

As a result of the N-terminal amino acid sequence analysis, the following sequences were obtained.

CHY-1: APVHRGRGG (SEQ ID NO: 13)

CHY-2: XAIDGLPYPQS (X is unidentified) (SEQ ID NO: 16)

CHY-3: LLGPVLHPPSXAEGGGKTALGILDL (X is unidentified) (SEQ ID NO: 15)

CHY-4: TLNSAG (SEQ ID NO: 14)

In view of the N-terminal amino acid sequence of the GALR2 activating major component obtained in (4-1) above or that of GALR2 activating sub-component (1) obtained in (4-2) above, it was estimated that GALR2 activating sub-component (2) should have the following N-terminal amino acid sequence:

APVHRGRGGWTLNSAGYLLGPVLHPPSXAEGGGKTALGILDL (SEQ ID NO: 17)

### REFERENCE EXAMPLE 5:

### cDNA Encoding Pig-Type Ligand Peptide (1-60)

### 1) Cloning of a Gene Fragment of Pig GALR2 Activator by Degenerated PCR

Total RNA was extracted from pig whole brain using TRIZOL reagent (Gibco BRL) according to the method described in the manual attached to the reagent. Then, poly(A)⁺ RNA was prepared from the total RNA using an oligo dT cellulose column (mRNA Purification Kit; Pharmacia) according to the method described in the manual attached to the Kit. Subsequently, a first strand DNA was synthesized from the poly(A)⁺ RNA using 3' RACE System for Rapid Amplification of cDNA Ends (Gibco BRL) according to the method described in the manual attached to the System. Further, the following degenerate primers were synthesized which had been designed based on partial amino acid sequences of the pig GALR2 activating component.

pGAL4-7F: 5'-CAYMGNGGIMGNGGIGGSTGGAC-3' (SEQ ID NO: 20)

pGAL9-3F: 5'-GGHTGGACNCTNAAYAGYGC-3' (SEQ ID NO: 21)

pGAL34-1R: 5'-ATICCNAGIGCNGTYTTICCYTT-3' (SEQ ID NO: 22)

Using the first strand cDNA as a template and pGAL4-7F and pGAL34-1R as primers, a first step PCR reaction was performed. The reaction solution for this PCR was prepared by mixing.0.5 µl of Taq polymerase (Takara Shuzo), 10 µl of 10x PCR buffer (attached to the polymerase) (500 mM KCl-100 mM Tris-HCI, pH 8.3), 6 µl of 25 mM MgCl₂, 8 µl of 2.5 mM dNTP mixture, 10 µl each of primer pGAL4-7F and primer pGAL34-1R (each µM), 8 µl of the template cDNA (the first strand cDNA described above) and 47.5 µl of distilled water. The PCR was performed in the following order: (1) initial denaturation (94°C, 30 sec), (2) 32 cycles of (94°C, 20 sec; 55°C, 30 sec; 72°C, 30 sec), and (3) final extension reaction (72°C, 4 min). Thus, a first step PCR product was obtained.

Using the resultant first step PCR product as a template, a second step PCR reaction (nested PCR) was performed. The reaction solution for this PCR was prepared by mixing 0.5 µl of Taq polymerase (Takara Shuzo), 10 µl of 10x PCR buffer (attached to the polymerase) (500 mM KCl-100 mM Tris-HCl, pH 8.3), 6 µl of 25 mM MgCl₂, 8 µl of 2.5 mM dNTP mixture, 10 µl each of primer pGAL9-3F and primer pGAL34-1R (each 10 µM), 5 µl of the template cDNA (the first step PCR product) and 50.5 µl of distilled water. The PCR was performed in the following order: (1) initial denaturation (94°C, 30 sec), (2) 32 cycles of (94°C, 20 sec; 55°C, 30 sec; 72°C, 30 sec), and (3) final extension reaction (72°C, 10 min). Thus, a nested PCR product was obtained.

The nested PCR product was subjected to 0.2% agarose gel electrophoresis. A gel section containing an approx. 100 bp band stained with cyber green was cut out with a razor blade. From this gel section, a DNA fragment (that is the nested PCR product) was recovered with Gene Clean DNA Extraction Kit (BIO 101). This DNA fragment was ligated to plasmid vector pCRII contained in TOPO TA Cloning Kit (Invitrogen) according to the method described in the manual attached to the Kit. The resultant plasmid was purified using QIAwell 8 Ultra Plasmid Purification Kit (Qiagen). A reaction to determine the nucleotide sequence of the nested PCR product in the plasmid was performed using Dye Terminator Cycle Sequencing Kit (Applied Biosystems, Perkin Elmer), followed by determination of the nucleotide sequence with an automated fluorescence DNA sequencer (DNA Sequencer Prism 377; Applied Biosystems, Perkin Elmer). As a result, several plasmids could be obtained which had a 98 bp DNA fragment encoding a partial peptide of a pig GALR2 activator. Since degenerate primers were used in the PCR reactions, the DNAs in these plasmids were different at the portions corresponding to the primers. Described below are the nucleotide sequences of the nested PCR products in the two representative clones pCR100-6 and pCR100-7.

pCR100-6:
GGCTGGACTT TAAATAGTGC TGGTTACCTC CTGGGTCCCG TACTCCATCC GCCCTCCAGG
GCTGAAGGAG GCGGGAAGGG CAAAACAGCC CTGGGCAT (SEQ ID NO: 23)

pCR100-7:
GGTTGGACTT TGAACAGTGC TGGTTACCTC CTGGGTCCCG TACTCCATCC GCCCTCCAGG
GCTGAAGGAG GCGGGAAGGG CAAAACCGCC CTAGGCAT (SEQ ID NO: 24)

### 2) Cloning of cDNA Encoding Pig-Type Ligand Peptide (1-60)

A pig whole brain cDNA phage library was constructed from the above-mentioned poly(A)⁺ RNA from pig whole brain using ZAP-cDNA Gigapack III Gold Cloning Kit (Stratagene) according to the manual attached to the Kit. *Escherichia coli* XL1-Blue MRF' (Stratagene) was infected with the resultant phage (2,200,000 plaque forming units), plated on NZY medium agar plates (number of plates: 120) and cultured at 37°C for 8 hr. From the resultant *E. coli* plaques, the phage was transferred onto Hybond-N⁺ nylon membrane (Amersham). This nylon membrane was soaked in 0.5 N sodium hydroxide solution containing 1.5 M sodium chloride, 0.5 M Tris buffer (pH 7.0) containing 1.5 M sodium chloride, and 2x SSC in this order, and then air-dried.

The resultant nylon membrane was placed in a hybridization buffer (5x SSPE, 5x Denhart's solution, 0.5% SDS, 0.1 mg/ml salmon sperm DNA) and kept at 60°C for 24 hr. Subsequently, the nylon membrane was placed in the hybridization buffer containing a hybridization probe (5x10⁵ cpm/ml) and kept at 60°C for 24 hr. As the hybridization probe, a DNA fragment was used which was amplified using a 1:1 mixture of the above-described plasmids pCR100-6 and pCR100-7 as a template and the following primers pGAL9-3F and pGAL34-8R.

pGAL9-3F: 5'-GGHTGGACNCTNAAYAGYGC-3' (SEQ ID NO: 25)

pGAL34-8R: 5'-ATDCCBAGGGCDGTTTTGCCCTT-3' (SEQ ID NO: 26)

Briefly, the amplification reaction [where the reaction solution was composed of 0.5 ml of Ex Taq (Takara Shuzo), 5 µl of 10x Ex Taq buffer attached thereto (containing 20 mM MgCl₂), 4 µl of 2.5 mM dNTP mixture, 10 µl of [α-³²P]dCTP (6000 Ci/mM), 0.5 µl each of primers pGAL9-3F and pGAL34-8R (each 10 µM), 1 µl of the template cDNA, and 29 µl of distilled water] was performed as follows: after initial denaturation at 96°C for 30 sec, 30 cycles of (94°C, 20 sec; 55°C, 30 sec; 72°C, 30 sec) and final extension reaction at 72°C for 4 min followed. Thus, an amplified DNA fragment was obtained.

The nylon membrane after the hybridization reaction was washed with 0.2x SSC buffer containing 0.1 % SDS at 50°C for 30 min and then subjected to autoradiography using an X-ray film (Kodak; BioMax MS) in the presence of a sensitization screen (exposure conditions: -70°C, 3 days). Positive plaques detected by the autoradiography were isolated, and the phage contained therein was extracted into 5 ml of SM buffer (100 mM NaCI, 8 mM MgSO₄, 0.01 % gelatin, 50 mM Tris-HCl, pH 7.5) containing 0.1 ml of chloroform.

The resultant phage was allowed to infect *E. coli* XL1-Blue MRF' again. The resultant *E. coli* was plated on NZY medium agar plates and cultured at 37°C for 8 hr. From the resultant *E. coli* plaques, phage was transferred onto a nylon membrane (Amershan; Hybond-N⁺), which was then subjected to hybridization in the same manner as described above. A positive single clone was isolated by autoradiography. A series of operations as described above were repeated once to thereby obtain a completely single phage clone.

Subsequently, plasmids were isolated and purified from this phage clone as described below. Briefly, phage solution (1 µl) was mixed with *E. coli* XPORT (50 µl), helper phage (10 µl) and *E. coli* XLOLR (5 µl). The mixture was plated on NYZ agar plates and cultured at 37°C for 8 hr. Small colonies of *E. coli* XLOLR formed in *E. coli* XPORT plaques were picked up with toothpicks, transferred to LB medium agar plates containing ampicillin and tetracycline, and cultured overnight at 37°C. *E. coli* XLOLR was picked up from a single colony and liquid-cultured in LB medium. After completion of the culture, *E. coli* XLOLR cells were harvested. The plasmids in the cells were purified using a plasmid purification kit (Qiagen; QIAwell 8 Ultra).

In order to determine the nucleotide sequence of a cDNA encoding a pig GALR2 activator contained in those plasmids, a reaction was performed using Dye Terminator Cycle Sequencing Kit (Applied Biosystems, Perkin Elmer). The nucleotide sequence of a cDNA encoding the pig GALR2 activator was determined using an automated fluorescence DNA sequencer (DNA Sequencer Prism 377; Applied Biosystems, Perkin Elmer). As a result, plasmid pGR2PL6 having a 974 bp DNA fragment encoding the full-length peptide of a pig GALR2 activator and plasmid pGR2PL3 having a 1007 bp DNA fragment encoding the full-length peptide of a pig GALR2 activator were obtained. Although big differences were observed in the chain length and amino acid sequence of the GALR2 activator precursors encoded by the two DNA sequences, the sequences encoding the mature activators (mature peptides) (that are predicted to be produced through processing) were identical with each other.

The resultant two plasmids were introduced into *E. coli* TOP 10 by a known method to thereby obtain transformants, *Escherichia coli* TOP10/pGR2PL6 and *Escherichia coli* TOP10/pGR2PL.

### REFERENCE EXAMPLE 6:

### Production of Pig-Type Ligand Peptide (1-60):

Ala-Pro-Val-His-Arg-Gly-Arg-Gly-Gly-Trp-Thr-Leu-Asn-Ser-Ala-Gly-Tyr-Leu-Leu
-Gly-Pro-Val-Leu-His-Pro-Pro-Ser-Arg-Ala-Glu-Gly-Gly-Gly-Lys-Gly-Lys-Thr-Ala
-Leu-Gly-Ile-Leu-Asp-Leu-Trp-Lys-Ala-Ile-Asp-Gly-Leu-Pro-Tyr-Pro-Gln-Ser-Gln
-Leu-Ala-Ser (SEQ ID NO: 31)

A protected peptide resin of interest was obtained by placing 0.5 mM of a commercial Boc-Ser(Bzl)l-OCH₂-PAM resin (0.73 mM/g resin) in the reactor of Peptide Synthesizer ABI 430A and introducing thereto Boc-Ala, Boc-Leu, Boc-Gln, Boc-Ser(Bzl), Boc-Pro, Boc-Tyr(Br-Z), Boc-Gly, Boc-Asp(OcHex), Boc-Ile, Boc-Lys(Cl-Z), Boc-Trp(CHO), Boc-Thr(Bzl), Boc-Glu(OcHex), Boc-Arg(Tos), Boc-His(Bom), Boc-Val and Boc-Asn in the order of the desired amino acid sequence according to the Boc-strategy (NMP-HOBt) peptide synthesis method.

The resultant peptide resin (0.105 g), p-cresol (1.1 g) and 1,4-butanedithiol (1.2 ml) were agitated in 10 ml of anhydrous hydrogen fluoride at 0°C for 60 min, followed by vacuum distillation of the hydrogen fluoride. Diethyl ether was added to the residue, and the resultant precipitate was filtered. Then, the precipitate was extracted with 50% aqueous acetic acid solution, followed by removal of the insoluble matter. After sufficient condensation, the extract was loaded onto a Sephadex^{TM} G-25 column (2.0 x 80 cm) filled with 50% aqueous acetic acid solution, followed by development with the same solvent. Major fractions were recovered and loaded on a reversed phase chromatography column (2.6 x 60 cm) packed with LiChroprep™ RP-18. Then, the column was washed with 200 ml of 0.1 % aqueous TFA solution, followed by a linear gradient elution using 300 ml of 0.1 % aqueous TFA solution and 300 ml of 0.1 % TFA/50% aqueous acetonitrile solution. Major fractions were recovered, concentrated and lyophilized to thereby obtain 39 mg of white powder. This powder was further loaded onto an ion exchange chromatography column packed with CM-Cellulofine™, followed by a linear gradient elution using 50 mM to 200 mM ammonium acetate. Major fractions were recovered and lyophilized again to thereby obtain 10 mg of white powder.
(M+H)⁺ by mass spectrometry: 6204.7 (calculated value: 6205.2)
HPLC elution time: 21.8 min
Column conditions:
Column: Wakosil 5C18T (4.6 x 100 mm)
Eluents: Solution A (0.1% aqueous TFA solution) and Solution B (0.1% TFA/acetonitrile). Linear gradient elution from A/B=95/5 to A/B=45/55 (over 25 min).
Flow rate: 1.0 ml/min

### REFERENCE EXAMPLE 7:

### Production of Rat-Type Ligand Peptide (1-60):

Ala-Pro-Ala-His-Arg-Gly-Arg-Gly-Gly-Trp-Thr-Leu-Asn-Ser-Ala-Gly-Tyr-Leu-Leu
-Gly-Pro-Val-Leu-His-Leu-Ser-Ser-Lys-Ala-Asn-Gln-Gly-Arg-Lys-Thr-Asp-Ser-Ala
-Leu-Glu-Ile-Leu-Asp-Leu-Trp-Lys-Ala-Ile-Asp-Gly-Leu-Pro-Tyr-Ser-Arg-Ser-Pro-Arg
-Met-Thr (SEQ ID NO: 33)

and Human-Type Ligand Polypeptide (1-60) :

Ala-Pro-Ala-His-Arg-Gly-Arg-Gly-Gly-Trp-Thr-Leu-Asn-Ser-Ala-Gly-Tyr-Leu-Leu
-Gly-Pro-Val-Leu-His-Leu-Pro-Gln-Met-Gly-Asp-Gln-Asp-Gly-Lys-Arg-Glu-Thr-Ala
-Leu-Glu-Ile-Leu-Asp-Leu-Trp-Lys-Ala-Ile-Asp-Gly-Leu-Pro-Tyr-Ser-His-Pro-Pro-Gln
-Pro-Ser (SEQ ID NO: 34)

These peptides can be obtained by performing synthesis and purification in the same manner as in Reference Example 1 using a commercial Boc-Thr(Bzl)l-OCH₂-PAM resin.

### REFERENCE EXAMPLE 8:

### Production of Antigen Peptide: Ala-Pro-Ala-His-Arg-Gly-Arg-Gly-Gly-Cys-NH₂ (C-Terminal-Amide Form of a Peptide Having the Amino Acid Sequence as Shown in SEQ ID NO: 44) Used in Monoclonal Antibody Production

Using a commercial p-methyl MBH resin (0.77 mM/g resin), Boc-Cys(MeBzl), Boc-Gly, Boc-Arg(Tos), Boc-His(Bom), Boc-Ala, and Boc-Pro were condensed in the order of the desired amino acid sequence and treated with hydrogen fluoride in the same manner as in Reference Example 1. The resultant product was purified on a Sephadex^{TM} G-25 column (2.0 x 80 cm) filled with 50% aqueous acetic acid solution and then lyophilized to thereby obtain 50 mg of white powder.
(M+H)⁺ by mass spectrometry: 980.5 (calculated value: 980.5)
HPLC elution time: 5.2 min
Column conditions:
Column: Wakosil 5C18T (4.6 x 100 mm)
Eluents: Solution A (0.1% aqueous TFA solution) and Solution B (0.1% TFA/acetonitrile). Linear gradient elution increasing the concentration of Solution B from 0% to 50% (over 25 min).
Flow rate: 1.0 ml/min

### REFERENCE EXAMPLE 9:

### Preparation of Immunogen Comprising Ala-Pro-Ala-His-Arg-Gly-Arg-Gly-Gly (SEQ ID NO: 35)

A complex of the peptide represented by Ala-Pro-Ala-His-Arg-Gly-Arg-Gly-Gly-Cys-NH₂ (a C-terminal-amide form of a peptide having the amino acid sequence as shown in SEQ ID NO: 44) obtained in Reference Example 8 and keyhole limpet hemocyanin (KLH) was created as an immunogen. Briefly, 20 mg of KLH was dissolved in 1.4 ml of 0.1 mM phosphate buffer (pH 6.5), mixed with 100 µl of DMF solution containing 2.2 mg (8 µmol) of N-(γ-maleimidobutyryloxy)succinimide (GMBS) and reacted at room temperature for 40 min, followed by fractionation on a Sephadex G-25 column. Then, 15 mg of the thus prepared KLH into which maleimide groups were introduced and 1.6 mg of the peptide represented by Ala-Pro-Ala-His-Arg-Gly-Arg-Gly-Gly-Cys-NH₂ (a C-terminal-amide form of a peptide having the amino acid sequence as shown in SEQ ID NO: 44) were mixed and reacted at 4°C for two days. After the reaction, the reaction solution was dialyzed against physiological saline at 4°C for two days.

### REFERENCE EXAMPLE 10:

### Immunization

Six to eight-week old BALB/c female mice were immunized by subcutaneous injection of the immunogen described in Reference Example 9 above, i.e.
Ala-Pro-Ala-His-Arg-Gly-Arg-Gly-Gly-Cys(-NH₂)-KLH complex (about 30 µg/mouse) together with complete Freund's adjuvant. Subsequently, the same dose of the immunogen was administered together with incomplete Freund's adjuvant twice in every 3 weeks as a booster.

### REFERENCE EXAMPLE 11:

### Preparation of Enzyme-Labeled Antigen

### Preparation of horseradish peroxidase (HRP)-labeled peptide represented by Ala-Pro-Ala-His-Arg-Gly-Arg-Gly-Gly-Cys-NH₂ (an amide form of SEQ ID NO: 44)

The peptide represented by Ala-Pro-Ala-His-Arg-Gly-Arg-Gly-Gly-Cys-NH₂ (a C-terminal-amide form of a peptide having the amino acid sequence as shown in SEQ ID NO: 44) obtained in Reference Example 8 and HRP (for enzyme immunoassay; Boehringer Mannheim) were cross-linked to thereby prepare a labeled product for use in enzyme immunoassay (EIA). Briefly, 6 mg (150 nmol) of HRP was dissolved in 0.95 ml of 0.1 M phosphate buffer (pH 6.5) and mixed with 50 µl of DMF solution containing 0.42 mg (1.5 µmol) of GMBS and reacted at room temperature for 30 min, followed by fractionation on a Sephadex G-25 column. Then, 4.2 mg (105 nmol) of the thus prepared HRP into which maleimide groups were introduced and 0.31 mg (315 nmol) of the peptide represented by Ala-Pro-Ala-His-Arg-Gly-Arg-Gly-Gly-Cys-NH₂ (a C-terminal-amide form of a peptide having the amino acid sequence as shown in SEQ ID NO: 44) were mixed and reacted at 4°C for one day. After the reaction, the reaction solution was fractionated using an Ultrogel AcA44 (LKB-Pharmacia) column to thereby obtain HRP-labeled Ala-Pro-Ala-His-Arg-Gly-Arg-Gly-Gly-Cys-NH₂.

### REFERENCE EXAMPLE 12:

### Measurement of Antibody Titers in Antisera of Mice Immunized with Ala-Pro-Ala-His-Arg-Gly-Arg-Gly-Gly-Cys-NH₂-KHL Complex

Antibody titers in antisera of mice immunized with the Ala-Pro-Ala-His-Arg-Gly-Arg-Gly-Gly-Cys-NH₂-KHL complex obtained in Reference Example 9 were measured as described below. Briefly, in order to prepare anti-mouse immunoglobulin antibody-bound microplates, 0.1 M carbonate buffer (pH 9.6) containing 100 µg/ml anti-mouse immunoglobulin antibody (IgG fraction: Cappel) was added to 96-well microplates (100 µl/well) and left at 4°C for 24 hr. Subsequently, the plates were washed with phosphate-buffered saline (PBS, pH 7.4), and then 25% Block Ace (Snow Brand Products)-containing PBS was added thereto (300 µl/well) to block the excessive binding sites in the wells. The plates were treated at 4°C for at least 24 hr.

To each well of the thus prepared anti-mouse immunoglobulin-bound plate, 50 µl of Buffer C [0.02 M phosphate buffer (pH 7.0) containing 1 % BSA, 0.4 M NaCl and 2 mM EDTA] and 10 µl of an antiserum against the Ala-Pro-Ala-His-Arg-Gly-Arg-GIy-Gly-Cys-NH₂-KHL complex diluted with Buffer C were added and reacted at 4°C for 16 hr. After washing the plate with PBS, 100 µl of the HRP-labeled Ala-Pro-Ala-His-Arg-Gly-Arg-Gly-Gly-Cys-NH₂ (diluted 500-fold with Buffer C) prepared in Reference Example 11 was added to each well and reacted at room temperature for one day. After washing the plate with PBS, 100 µl of TMB microwell peroxidase substrate system (Kirkegaard & Perry Lab, Inc.; purchased from Funakoshi) was added to each well and reacted at room temperature for 10 min to thereby measure the enzyme activity on the solid phase. The reaction was terminated by adding 100 µl of 1 M phosphoric acid. Then, absorption at 450 nm was measured with a plate reader (Biochromatic; Dainippon Pharmaceutical).

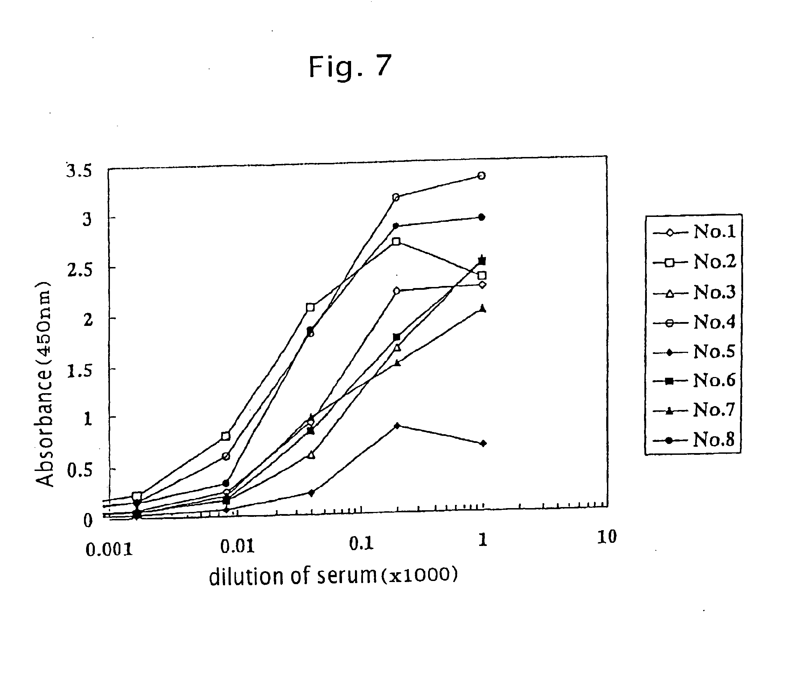

The results are shown in Fig. 7. Increase in antibody titer against the Ala-Pro-Ala-His-Arg-Gly-Arg-Gly-Gly-Cys-NH₂-KHL complex was observed in all the 8 mice immunized.

### REFERENCE EXAMPLE 13:

### Preparation of Monoclonal Antibodies to Rat-Type Ligand Peptide (1-60) (SEQ ID NO: 33)

Final immunization was performed by intravenously injecting 200-300 µg of the immunogen (dissolved in 0.25-0.3 ml of physiological saline) into mice that had exhibited a relatively high antibody titer.

Three to four days after the final immunization, the spleen was removed from each mouse, pressed with a stainless mesh, filtered, and suspended in Eagle's minimum essential medium (MEM) to thereby prepare a spleen cell suspension. As a cell for cell fusion, BALB/c mouse-derived myeloma cell P3-X63.Ag8.U1 (P3U1) was used [Current Topics in Microbiology and Immunology, 81: 1 (1978)]. Cell fusion was performed based on the original method [Nature, 256: 495 (1975)]. Briefly, spleen cells and P3U1 cells were washed separately with serum-free MEM three times and mixed so that the ratio of spleen cells:P3U1 cells is 10:1 in number. The mixture was centrifuged at 800 rpm for 15 min to precipitate cells. The supernatant was sufficiently removed, and the precipitate was loosened rightly. To this precipitate, 0.3 ml of 45% polyethylene glycol (PEG) 6000 was added, and cell fusion was performed by leaving the precipitate stationary in a warm water bath at 37°C for 7 min. After the cell fusion, 2 ml of MEM was added to the cells every minute. When a total 15 ml of MEM was added, the cells were centrifuged at 600 rpm for 15 min to remove the supernatant. The resultant cell pellet was suspended in 10% fetal calf serum-containing GIT medium (Wako Purechemical) (GIT-10% FCS) to give a concentration of 2x10⁵ P3U1 cells/ml and plated in 192 wells of 24-well multi-dishes (Linbro) at 1 ml/well. The plated cells were cultured in a 5% carbon dioxide incubator at 37°C. After 24 hours, HAT (hypoxanthine 1x10⁻⁴ M, aminopterin 4x10⁻⁷ M, thymidine 6x10⁻³ M)-containing GIT-10% FCS medium (HAT medium) was added to each well (1 ml/well to thereby start HAT selection culture. On day 3, 6 and 9 of the cultivation, 1 ml of the old medium was discarded and 1 ml of HAT medium was added. The growth of hybridomas was recognized on day 9 to 14 of the cultivation. When the color of the culture broth changed into yellow (approx. 1x10⁶ cells/ml), the supernatant was recovered, followed by measurement of the antibody titer according to the method described in Reference Example 5.

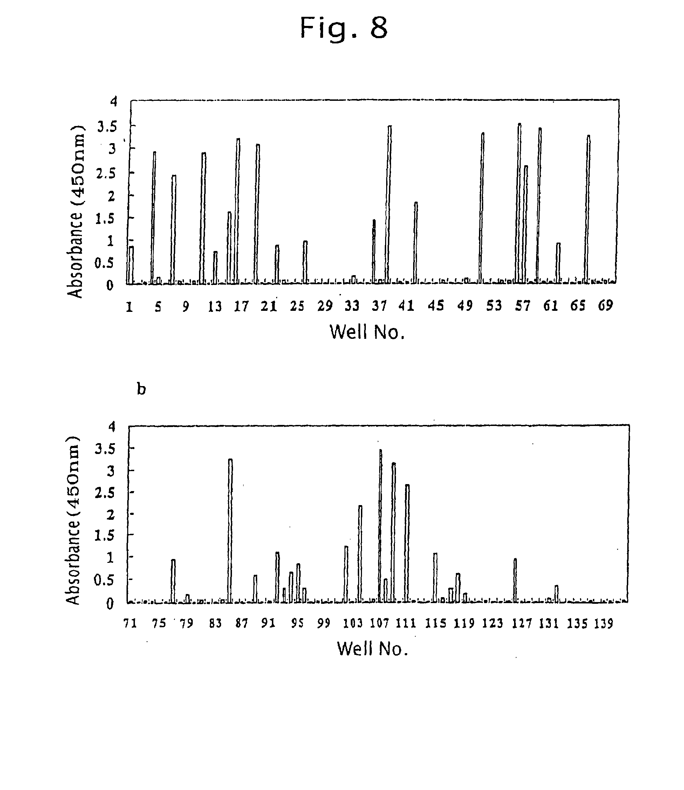

As a typical example of screening of hybridomas derived from the Ala-Pro-Ala-His-Arg-Gly-Arg-Gly-Gly-Cys(-NH₂)-KLH-immunized mice, the results obtained from mouse No. 8 (see Fig. 7) are shown in Fig. 8.

Subsequently, these hybridomas exhibiting high antibody titers were subjected to cloning by the limiting dilution-culture method. In the cloning, BALB/c mouse thymus-derived cells were added as a feeder cell (5x10⁵ cells/well). After the cloning, the hybridoma was injected intraperitoneally into BALB/c mice (1-3 x 10⁶ cells/mouse) into which 0.5 ml of mineral oil was pre-injected intraperitoneally. After 6 to 20 days, antibody-containing abdominal dropsy was recovered from each mouse.

Monoclonal antibodies were purified from the resultant abdominal dropsy using a protein A column. Briefly, 6-20 ml of abdominal dropsy was diluted with an equal volume of a binding buffer (1.5 M glycine buffer, pH 9.0, containing 3.5 M NaCI and 0.05% NaN₃) and loaded onto a recombinant protein A-agarose (Repligen) column pre-equilibrated with the binding buffer, followed by elution of specific antibody with an elution buffer (0.1 M acetate buffer, pH 3.0, containing 0.05% NaN₃). The eluate was dialyzed against PBS at 4°C for two days, filtered for sterilization with a 0.22 µm filter (Millipore), and stored at 4°C or -80°C. For the determination of the class/sub-class of monoclonal antibody, enzyme-linked immunosorbent assay (ELIZA) was performed using a purified monoclonal antibody-bound solid phase. Briefly, 0.1 M carbonate buffer (pH 9.6) containing 2 µg/ml antibody was added to 96-well microplates at 100 µl/well and left at 4°C for 24 hr. According to the method described in Reference Example 12, excessive binding sites in the wells were blocked with Block Ace. Then, the class and sub-class of the antibody bound to the solid phase were examined ELISA using an isotype typing kit (Mouse-Typer^{TM} Sub-Isotyping Kit; Biorad).

### REFERENCE EXAMPLE 14:

### Competitive Enzyme Immunoassay (Competitive EIA)

The reaction specificity of those monoclonal antibodies prepared with the Ala-Pro-Ala-His-Arg-Gly-Arg-Gly-Gly-Cys(-NH₂)-KLH complex as an immunogen was examined as described below. First, the antibody titers of individual monoclonal antibody solutions were examined by the method described in Reference Example 12. As an antibody concentration to be used in competitive EIA, the antibody concentration at which the amount of binding of labeled product will be about 50% of the amount of saturation binding was determined (approx. 30-50 ng/ml). Subsequently, 50 µl of an antibody solution diluted with Buffer C to the predetermined concentration; 50 µl of Buffer C containing Ala-Pro-Ala-His-Arg-Gly-Arg-Gly-Gly-Cys-NH₂ (an amide form of SEQ ID NO: 44), a pig-type ligand peptide (1-60) (SEQ ID NO: 31) or a rat-type galanin (SEQ ID NO: 61) with 0.05% CHAPS; and 50 µl of the HRP-labeled Ala-Pro-Ala-His-Arg-Gly-Arg-Gly-Gly-Cys-NH₂ described in Reference Example 11 (diluted 250-fold with Buffer C) were added to the anti-mouse immunoglobulin antibody-bound microplate described in Reference Example 12 and reacted at 4°C for 16 hr. After the reaction, the plate was washed with PBS, and the enzyme activity on the solid phase was measured according to the method described in Reference Example 12. The results are shown in Table 3.

**Table 3:**

| Reactivity of Monoclonal Antibody to Ala-Pro-Ala-His-Arg-Gly-Arg-Gly-Gly-Cys-NH₂ (an amide form of SEQ ID NO: 44) | | | | | |
|---|---|---|---|---|---|
| Reactivity 1) | | | | | |
| Hybridoma No. | SEQ ID NO: 44 | Pig Ligand (SEQ ID NO:31) | Rat Galanin (SEQ ID NO:61) | Class/Subclass | Note |
| 1 | + | - | - | | |
| 2 | + | + | - | IgGM, K | |
| 3 | + | ± | - | IgG1,K | |
| 4 | + | + | - | IgG2b, K | GR2-1N |
| 5 | + | ± | - | | |
| 6 | + | + | - | IgG2b, K | |
| 7 | + | - | - | | |
| 8 | + | - | - | | |
| 1) In the presence of 100 nM immunogen (SEQ ID NO: 44, 31, 61) + : (B/B₀) < 0.50 ± : 0.50 ≤ (B/B₀) < 0.80 - : 0.8 ≤ (B/B₀) wherein B: Amount of HRP-labeled SEQ ID NO: 44 bound in the presence of the immunogen; B₀: Amount of HRP-labeled SEQ ID NO: 44 bound in the absence of the immunogen. | | | | | |

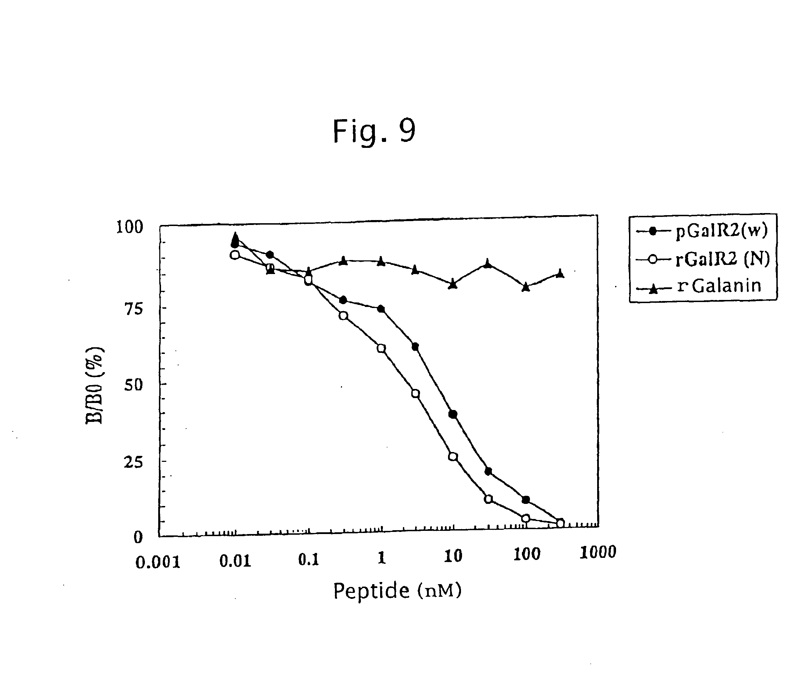

As a typical example, the results of competitive EIA on the monoclonal antibody GR2-1N (IgG_{2b}, K) that exhibited the highest reactivity with the pig-type ligand peptide (1-60) (SEQ ID NO: 31) are shown in Fig. 9. As seen from this Figure, GR2-1N has a similar degree of reactivity with Cys-Ala-Pro-Ala-His-Arg-Gly-Arg-Gly-Gly (SEQ ID NO: 44) and the pig-type ligand peptide (1-60) (SEQ ID NO: 31), but it does not react with the rat-type galanin (SEQ ID NO: 61). From the standard curve of the pig-type ligand peptide (1-60) (SEQ ID NO: 31) of GR2-1N, it was found that the pig-type ligand peptide (1-60) (SEQ ID NO: 31) concentration that gives (B/B₀)=0.5 is 6 nM, 4.95 ng/well and that the detection sensitivity is about 0.1 nM [(B/B₀)=0.8]. Therefore, competitive EI using GR2-1N is capable of measuring specifically the rat-type ligand peptide (SEQ ID NO: 33), the pig-type ligand peptide (1-60)(SEQ ID NO: 31) or the human-type ligand peptide (SEQ ID NO: 44), each having Ala-Pro-Ala-His-Arg-Gly-Arg-Gly-Gly, up to about 0.1 nM without cross-reaction with SEQ ID NO: 61 (rat galanin).

### REFERENCE EXAMPLE 15:

### Preparation of Pig-Type Ligand Peptide (1-34) (SEQ ID NO: 11)

In 250 µl of 1% ammonium bicarbonate solution containing 16% acetonitrile, 5 nmol of the pig-type ligand peptide (1-60) and 50 pmol of lysyl endopeptidase (Wako Purechemical) were retained at 37°C for 3.5 hr.

A Spheri-5 RP-18 reversed phase high performance liquid chromatography column (Brownlee; 2.1 mm x 30 mm) is pre-equilibrated at 25°C by feeding Solution A (0.1% trifluoroacetic acid) at a flow rate of 300 µl/min. The above enzyme reaction solution is loaded onto the column. Then, while keeping a flow rate of 300 µl/min, the concentration of Solution B (0.1% trifluoroacetic acid/70% acetonitrile) is increased to 70% over 30 min. The eluate was monitored with absorption at 210 nm, and peaks were fractioned and recovered manually.

The peak fraction eluted at 20.2 min gave a value of 3466.5 for M+H⁺ as a result of mass spectrometry. Its amino acid analysis also confirmed that this peak is a fragment ligand peptide (1-34) of the pig-type ligand peptide (1-60).

### REFERENCE EXAMPLE 16:

### Preparation of Pig-Type Ligand Peptide (1-30) (SEQ ID NO: 43)

In 250 µl of 1% ammonium bicarbonate solution containing 8% acetonitrile, 5 nmol of the pig-type ligand peptide (1-60) and 50 pmol of endoproteinase Glu-C (Boehringer Mannheim) were retained at 37°C for 6 hr and then at 25°C for 13 hr.

This enzyme reaction solution was separated under the same conditions as used for the pig-type ligand peptide (1-34).

The peak fraction eluted at 19.8 min gave a value of 3167.2 for M+H⁺ as a result of mass spectrometry. Its amino acid analysis also confirmed that this peak is a fragment ligand peptide (1-30) of the pig-type ligand peptide (1-60).

### REFERENCE EXAMPLE 17:

### Galanin Receptor Binding Experiment Using [¹²⁵I]Rat Galanin

The membrane fraction prepared earlier in (1-2) above was suspended in 50 mM Tris buffer (pH 7.3) containing 0.1% bovine serum albumin (BSA), 5 mM MgCl₂, 0.5 mM phenylmethylsulfonyl fluoride, 20 µg/ml leupeptin, 10 µg/ml pepstatin and 80 µg/ml E-64, to give a concentration of 13 µg/ml (for the GALR1 membrane fraction) or 15 µg/ml (for the GALR2 membrane fraction). The resultant suspension was added to polypropylene small test tubes (Falcon 2053) at 0.1 ml/tube. To the membrane fraction in each tube, 4 µl of 5 nM [¹²⁵I]rat galanin (New England Nuclear) and 1 µl of the synthesized peptide of the invention [pig-type ligand peptide (1-60) (SEQ ID NO: 31)], a partial digest of this synthesized peptide [pig-type ligand peptide (1-30) (SEQ ID NO: 43) or pig-type ligand peptide (1-34) (SEQ ID NO: 11)] or a rat galanin peptide (SEQ ID NO: 61) (Peptide Institute) at a varied concentration (i.e. 100 µM to 100 pM in dimethyl sulfoxide solution) were added and reacted at 25°C for 60 min. To the resultant reaction solution, 1.5 ml of 50 mM Tris buffer (pH 7.4) containing 0.05% 3-[(3-cholamidopropyl)dimethylammonio]-1-propanesulfonate (CHAPS), 0.1% BSA, 5 mM MgCl₂ and 1 mM EDTA was added and filtered with GF/F glass fiber filter paper (Whatman). This filter paper was washed with 1.5 ml of the same buffer, and then the radioactivity was measured with a gamma counter.

The amount of binding obtained when 1 µl of 100 µM rat galanin was added was taken as the non-specific binding amount (NSB); and the amount of binding obtained when 1 µl of dimethyl sulfoxide was added was taken as the maximum binding amount (B₀). Then, the concentration of peptide that decreases the maximum specific binding amount (B₀ - NSB) to 50% (IC₅₀) was determined (Table 4).

**Table 4:**

| | IC₅₀ (nM) for Rat GALR 1 | IC₅₀ (nM) for Rat GALR2 |
|---|---|---|
| SEQ ID NO: 31 pig (1-60) | 4.1 | 1.5 |
| SEQ ID NO: 43 pig (1-30) | 4.3 | 1.4 |
| SEQ ID NO: 11 pig (1-34) | not determined | 1.4 |
| SEQ ID NO: 61 Rat Galanin | 0.25 | 0.71 |

### REFERENCE EXAMPLE 18:

### Cloning of cDNA Encoding Rat-Type Ligand Peptide (1-60)

Total RNA was prepared from rat hypothalamus using TRIZOL reagent (Gibco BRL) in the same manner as described in Reference Example 5. Then, poly(A)⁺ RNA was purified from the total RNA using an oligo dT cellulose column (mRNA Purification Kit; Pharmacia). Subsequently, a rat hypothalamus cDNA phage library was constructed from the poly(A)⁺ RNA using ZAP-cDNA Gigapack III Gold Cloning Kit (Stratagene). The resultant phage (2,200,000 plaque forming units) was allowed to infect *Escherichia coli* XL1-Blue MRF' (Stratagene), which was then plated on NZY medium agar plates (number of plates: 130) and cultured at 37°C for 8 hr. From the resultant *E. coli* plaques, the phage was transferred onto Hybond N⁺ nylon membrane (Amersham). This nylon membrane was soaked in 0.5 N sodium hydroxide solution containing 1.5 M sodium chloride, 0.5 M Tris buffer (pH 7.0) containing 1.5 M sodium chloride, and 2x SSC in this order, and then air-dried.

The resultant nylon membrane was placed in a hybridization buffer (5x SSPE, 5x Denhart's solution, 0.1% SDS, 0.1 mg/ml salmon sperm DNA) and retained at 60°C for 24 hr. Subsequently, the nylon membrane was placed in the hybridization buffer containing a hybridization probe (2.8x10⁶ cpm/ml) and retained at 60°C for 18 hr. As the hybridization probe, a DNA fragment (365 bp) was used which was amplified using a EcoRI/BglII digest of plasmid pGR2PL6 (SEQ ID NO: 45) as a template and the primers pGAL1-1F and pGAL88-1R described below. The amplification reaction solution was prepared by mixing 0.5 µl of Ex Taq (Takara Shuzo), 10 µl of 10x PCR buffer attached thereto (500 mM KCl, 25 mM MgCl₂, 100 mM Tris-HCI, pH 8.3), 8 µl of 2.5 mM dNTP mixture, 25 µl of [α-³²P]dCTP (6000 Ci/mmol), 1 µl each of primers pGAL9-3F and pGAL34-8R (each 10 µM), 1 µl of the template cDNA and 54 µl of distilled water. The amplification was performed as follows: after initial denaturation at 94°C for 30 sec, 32 cycles of (94°C, 20 sec; 60°C, 30 sec; 72°C, 30 sec) and final extension reaction at 72°C for 4 min followed.

pGAL1-1F: 5'-ATGGCTCTGACTGTCCCTCTGATCGTTCT -3' (SEQ ID NO: 46)

pGAL88-1R: 5'-TGAAACCTCGTAGTTCCTGGTCGGATTCG -3' (SEQ ID NO: 47)

The nylon membrane after the hybridization reaction was washed with 0.2x SSC buffer containing 0.1% SDS at 50°C and then subjected to autoradiography using an X-ray film (Kodak; BioMax MS) in the presence of a sensitization screen (exposure conditions: -70°C, 3 days). Positive plaques detected by the autoradiography were pulled out, and the phage contained therein was extracted into 5 ml of SM buffer (100 mM NaCl, 8 mM MgSO₄, 0.01% gelatin, 50 mM Tris-HCl, pH 7.5) containing 0.1 ml of chloroform.

The resultant phage was again allowed to infect *E. coli* XL1-Blue MRF', which was then plated on NZY agar plates and cultured at 37°C for 8 hr. From the resultant *E. coli* plaques, phage was transferred onto a nylon membrane (Amershan; Hybond N⁺), followed by hybridization in the same manner as described above. Positive single clones were selected by autoradiography and pulled out by capillary. A series of operations as described above were repeated once to thereby obtain completely single phage clones.

Subsequently, plasmids were isolated from the cloned phage as described below. Briefly, phage solution (1 µl) was mixed with *E. coli* XPORT (50 µl), helper phage (10 µl) and *E. coli* XLOLR (5 µl). The mixture was plated on NYZ agar plates and cultured at 37°C for 8 hr. Small colonies of *E. coli* XLOLR formed in *E. coli* XPORT plaques were picked up with toothpicks, transferred to LB medium agar plates containing ampicillin and tetracycline, and cultured overnight at 37°C. *E. coli* XLOLR was picked up from a single colony and liquid-cultured in LB medium. After completion of the culture, *E. coli* XLOLR cells were harvested. The plasmid in the cells was purified using a plasmid purification kit (Qiagen; QIAgen 8Well Ultra).

The resultant plasmid was subjected to a sequencing reaction using Dye Terminator Cycle Sequencing Kit (Applied Biosystems, Perkin Elmer). The sequencing reaction product was analyzed with DNA Sequencer Prism 377 (Applied Biosystems, Perkin Elmer). As a result, plasmid pGR2R could be obtained which has a 974 bp DNA fragment (SEQ ID NO: 39) encoding the rat-type ligand full-length peptide (1-60). The *E. coli* transformed with this plasmid was designated *Escherichia coli* TOP10/pGR2RL4.

### REFERENCE EXAMPLE 19:

### Cloning of Human-Type Peptide Ligand (1-60)

A human brain cDNA library was constructed from human brain poly(A)⁺ RNA (Clontech) using ZAP-cDNA Synthesis Kit (Stratagene). With the resultant cDNA as a template, a PCR reaction was performed using the following primers F/R120 and R/R120.

F/R120: 5'-AGGCTGGACCCTCAATAGTGCTGGTTAC-3' (SEQ ID NO: 48)

R/R120: 5'-CCATCTATGGCCTTCCACAGGTCTAGGA-3' (SEQ ID NO: 49)

The reaction solution was prepared by mixing 0.5 µl of Taq (Takara Shuzo), 5 µl of 10x PCR buffer attached thereto (500 mM KCl, 25 mM MgCl₂, 100 mM Tris-HCl, pH 8.3), 4 µl of 2.5 mM dNTP mixture, 3 µl of 25 mM MgCl₂, 0.25 ml each of primers F/R120 and R/R120 (each 100 µM), 1 µl of the template cDNA and 36 µl of distilled water. The reaction was performed as follows: after initial denaturation at 94°C for 30 sec, 35 cycles of (94°C, 30 sec; 60°C, 30 sec; 72°C, 60 sec) and final extension reaction at 72°C for 10 min followed. The resultant DNA fragment was cloned using TOPO TA Cloning Kit (Invitrogen) according to the method described in the attached manual. The thus cloned DNA fragment was analyzed by the method described in Reference Example 5 to thereby obtain the sequence of SEQ ID NO: 50.

AGGCTGGACC CTCAATAGTG CTGGTTACCT TCTGGGTCCC GTCCTCCACC TTCCCCAAAT 60
GGGTGACCAA GACGGAAAGA GGGAGACAGC CCTTGAGATC CTAGACCTGT GGAAGGCCAT 120
AGATGG 126
(SEQ ID NO: 50)

From the resultant sequence, the following primers 1F/H120 (SEQ ID NO: 51) and 1R/H12 (SEQ ID NO: 52) were prepared and used in 5' RACE and 3'RACE experiments.

1F/H120: 5'-CAAATGGGTGACCAAGACGGAAAGAGGG-3' (SEQ ID NO: 51)

1R/H120: 5'-GGTCTAGGATCTCAAGGGCTGTCTCCCT-3' (SEQ ID NO: 52)

PCR reaction solutions for 5' RACE and 3'RACE were prepared by mixing 0.5 µl of Taq (Takara Shuzo), 5 µl of 10x PCR buffer attached thereto (500 mM KCI, 25 mM MgCl₂, 100 mM Tris-HCI, pH 8.3), 4 µl of 2.5 mM dNTP mixture, 3 µl of 25 mM MgCl₂, 1 ml of 10 µM primer F/R120 (for 3'RACE) or 10 µM primer R/R120 (for 5'RACE), 1 µl of 10 µM primer AP1 (this primer is contained in Clontech's Marathon-Ready cDNA Kit), 5 µl of template cDNA (Clontech; Marathon-Ready cDNA Kit, Human Hypothalamus) and 31 µl of distilled water. The reaction conditions were as follows: after initial denaturation at 94°C for 60 sec, 5 cycles of (94°C, 30 sec; 72°C, 120 sec), 5 cycles of (94°C, 30 sec; 70°C, 120 sec), 25 cycles of (94°C, 30 sec; 68°C, 120 sec) and final extension reaction at 68°C for 10 min were performed.

Subsequently, using the resultant reaction solution as a template, nested PCR was performed. The reaction solution for this PCR was prepared by mixing 0.5 µl of Taq (Takara Shuzo), 5 µl of 10x PCR buffer attached thereto (500 mM KCl, 25 mM MgCl₂, 100 mM Tris-HCl, pH 8.3), 4 µl of 2.5 mM dNTP mixture, 3 µl of 25 mM MgCl₂, 1 µl of 10 µM primer 1F/H120 (for 3'RACE) or 10 µM primer 1R/H120 (for 5'RACE), 1 µl of 10 µM primer AP2 (this primer is contained in Clontech's Marathon-Ready cDNA Kit), 5 µl of template DNA (50-fold dilution of the above PCR reaction solution) and 31 µl of distilled water. The reaction conditions were as follows: after initial denaturation at 94°C for 60 sec, 5 cycles of (94°C, 30 sec; 72°C, 120 sec), 5 cycles of (94°C, 30 sec; 70°C, 120 sec), 25 cycles of (94°C, 30 sec; 68°C, 120 sec) and final extension reaction at 68°C for 10 min were performed.

The resultant DNA fragment was cloned using TOPO TA Cloning Kit (Invitrogen) according to the method described in the attached manual. The thus cloned DNA fragment was analyzed by the method described in Reference Example 5 (in the section addressed to the cloning of pig cDNA) to thereby obtain sequence information on the 5' end and the 3' end. Based on this information, primers 1F/H470 and 1R/H450 were prepared.

1F/H470: 5'-GAGGAGCCAGAGAGAGCTGCGGAGAG-3' (SEQ ID NO: 53)

1R/H470: 5'-GAGCTGGAGAAGAAGGATAGGAACAGGG-3' (SEQ ID NO: 54)

A PCR reaction was performed using primers F/H470 and R/H450 and the human brain cDNA library as a template. The reaction solution was prepared by mixing 0.5 µl of Pfu turbo DNA polymerase (Stratagene), 5 µl of 10x PCR buffer attached thereto (500 mM KCl, 25 mM MgCl₂, 100 mM Tris-HCI, pH 8.3), 4 µl of 2.5 mM dNTP mixture, 2.5 ml each of 10 µM primer F/H470 and 10 µM primer R/H450, 0.5 µl of human whole brain cDNA as a template and 35 µl of distilled water. The reaction conditions were as follows: after initial denaturation at 94°C for 30 sec, 35 cycles of (94°C, 30 sec; 70°C, 5 min) and final extension reaction at 72°C for 10 min were performed. The resultant DNA fragment was cloned into pPCR-Script Amp SK(⁺) Vector (Stratagene) according to the method described in the attached manual. The cloned DNA sequence was analyzed by the method described in Reference Example 5 (in the section addressed to the cloning of pig cDNA). As a result, plasmid pGR2HL14 could be obtained which has a 473 bp DNA fragment (SEQ ID NO: 40) encoding the human-type ligand full-length peptide (1-60). The *E. coli* transformed with this plasmid was designated *Escherichia coli* TOP10/pGR2HL4.

### REFERENCE EXAMPLE 20:

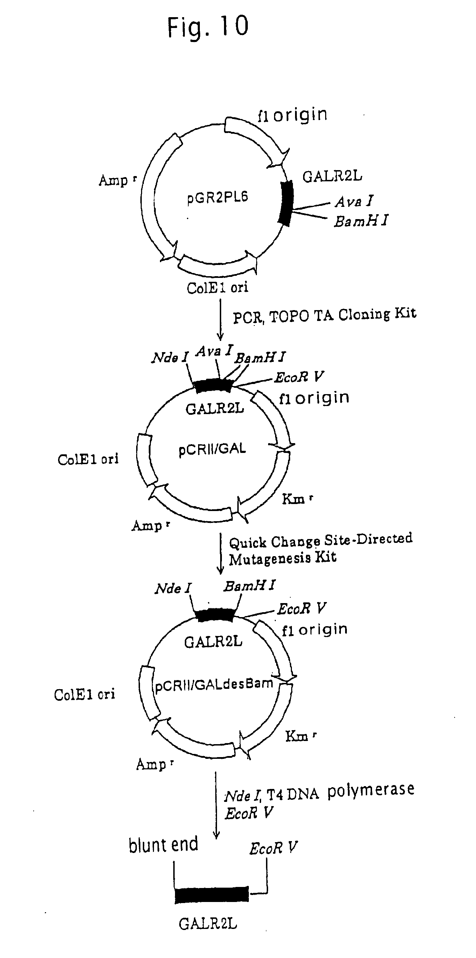

### Preparation of Structural Gene of Pig-Type Ligand Peptide (1-60) (see Fig. 10)

The structural gene for the (1-60) peptide of the invention was amplified from plasmid pGR2PL6 obtained in Reference Example 5 by PCR using Primer 1: 5'-AGCATATGGCTCCGGTCCACAGG-3' (SEQ ID NO: 55; Kikotech) located immediately upstream of the structural gene and having an NdeI restriction site and Met, and Primer 2: 5'-CTGGATCCTCAGGAGGCCAACTGAGAC-3' (SEQ ID NO: 56; Greiner Japan) located immediately downstream of the structural gene and having a termination codon and a BamHI restriction site. This gene was ligated to pCRII-TOPO vector using TOPO TA Cloning Kit (Invitrogen) to thereby prepare pCRII/GAL. This plasmid was transformed into TOP10 One Shot competent cells, which were then plated on 50 µg/ml ampicillin-containing LB agar medium (1% peptone, 0.5% yeast extract, 0.5% sodium chloride, 2% agar) coated with X-gal(5-bromo-4-chloro-3-indolyl-β-D-galactose). The cells were cultured at 37°C for one day, followed by selection of transformants using tetracycline resistance and β galactosidase activity as indicators. The transformant was cultured in 50 µg/ml ampicillin-containing LB medium (1% peptone, 0.5% yeast extract, 0.5% sodium chloride) overnight, followed by recovery of the plasmid using QIAprep8 Miniprep Kit (Qiagen). Further, plasmid pCRII/GALdesBam in which the AvaI recognition site located adjacent to the BamHI site in the structural gene of the pig-type ligand peptide (1-60) of the invention is deleted was created by site-directed mutagenesis (Quick Change; Stratagene) using Primer 3: 5'-CAGCCCTCGGCATCCTGGACC-3' (SEQ ID NO: 57; Greiner Japan) and Primer 4: 5'-GGTCCAGGATGCCGAGGGCTG-3' (SEQ ID NO: 58; Greiner Japan). The nucleotide sequence of structural gene moiety of the pig-type ligand peptide (1-60) of the invention was confirmed with Model 377 DNA sequencer (Applied Biosystems). The nucleotide sequence-confirmed pCRII/GALdesBam (2 µg) was digested with NdeI and blunt-ended with T4 DNA polymerase (DNA Blunting Kit; Takara Shuzo). After further digestion with EcoRV, the digest was subjected to 3% agarose gel electrophoresis. An approx. 200 bp DNA fragment was extracted from the gel using QIAquick Gel Extraction Kit (Qiagen) and dissolved in 25 µl of TE buffer (10 mM Tris-HCl (pH 8.0), 1 mM EDTA).

### REFERENCE EXAMPLE 21:

### Preparation of Pig-Type Ligand Peptide (1-60) Expression Plasmid

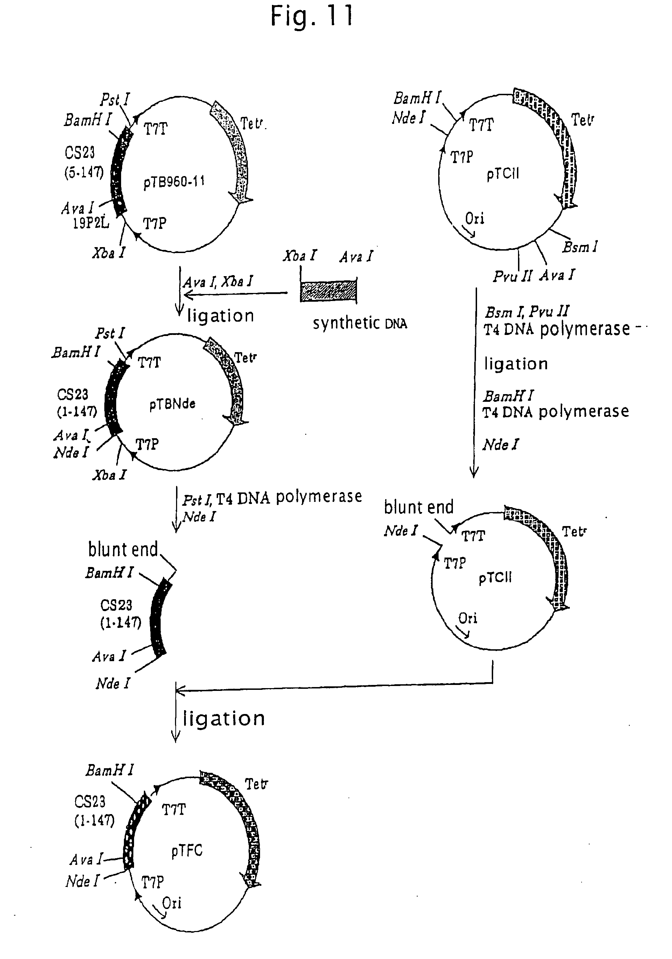

### a) Preparation of Fusion Protein Expression Vector (see Fig. 11)

Plasmid pTB960-11 (1 µg) retained in a transformant *Escherichia coli* MM294(DE3)/pTB960-11 was digested with XbaI and AvaI, and subjected to 1% agarose gel electrophoresis. An approx. 4.4 kb DNA fragment was extracted from the gel using QIAquick Gel Extraction Kit (Qiagen) and dissolved in 50 µl of TE buffer. Synthetic DNAs 5'-CTAGACATATGCCAGCATTGC-3'(SEQ ID NO: 59) and 5'-TCGGGCAATGCTGGCATATGT-3' (SEQ ID NO: 60) (both produced by Greiner Japan) (1 µg each), which were designed to insert a deleted portion of hFGF mutein CS23 structural gene, an NdeI restriction site immediately upstream thereof and a start coon, were reacted in 100 µl of a phosphorylation reaction solution [50mM Tris-HCl (pH7.6), 10mM MgCl₂, 5mM dithiothreitol, 0.1mM spermidine, 0.1 mM EDTA, 1 mM ATP, 10 units of T4 polynucleotide kinase (Nippon Gene)] at 37°C for 1 hr to thereby phosphorylate their 5'end. After the completion of the reaction, phenol/chloroform extraction and ethanol precipitation were carried out. The resultant precipitate was dissolved in 50 µl of TE buffer. This solution was retained at 80°C for 10 min and then gradually cooled at room temperature for annealing. The XbaI-AvaI DNA fragment obtained above and this annealing solution were ligated using Takara DNA Ligation Kit (Takara Shuzo). Briefly, 3 µl of distilled water and 5 µl of ligation solution I were added to 1 µl of the XbaI-AvaI DNA fragment and 1 µl of the annealing solution and reacted at 16°C for 30 min. Using 10 µl of this ligation solution, *E. coli* JM109 competent cells (Toyobo) were transformed and plated on 10 µg/ml tetracycline-containing LB agar medium. Then, tetracycline resistant colonies growing on the medium were selected. The resultant transformant was cultured overnight in LB medium, followed by preparation of the plasmid using QIAprep8 Miniprep Kit (Qiagen). The resultant plasmid was digested with NdeI and subjected to 1 % agarose gel electrophoresis, to thereby confirm that an Ndel restriction site was newly generated. This plasmid was designated pTBNde.

Subsequently, 1 µg of plasmid pTBNde was digested with PstI, blunt-ended with T4 DNA polymerase (DNA Blunting Kit; Takara Shuzo), and subjected to phenol/chloroform extraction and ethanol precipitation. The resultant precipitate was dissolved in 10 µl of TE buffer, digested with NdeI, and then subjected to 3% agarose gel electrophoresis. An approx. 470 bp DNA fragment was extracted from the gel using QIAquick Gel Extraction Kit (Qiagen) and dissolved in 25 µl of TE buffer. Plasmid pTCII (1 µg) retained in a transformant *Escherichia coli* MM294(DE3)/pTCIId23-MPIF1 was digested with BsmI and PvuII, and then subjected to subjected to 1% agarose gel electrophoresis. An approx. 3.7 kbp DNA fragment was extracted from the gel using QIAquick Gel Extraction Kit and dissolved in 25 µl of TE *buffer.* This DNA fragment was blunt-ended with T4 DNA polymerase using DNA Blunting Kit and self-ligated. The ligation solution was extracted with phenol/chloroform and subjected to ethanol precipitation. The resultant precipitate was dissolved in 10 µl of TE buffer. This solution was digested with BamHI, blunt-ended with T4 DNA polymerase, digested further with NdeI, and subjected to 1 % agarose gel electrophoresis. An approx. 3.7 kbp DNA fragment was extracted from the gel using QIAquick Gel Extraction Kit and dissolved in 50 µl of TE buffer. To 4 µl of the approx. 470 bp NdeI-blunt DNA fragment prepared above and 1 µl of this approx. 3.7 kbp NdeI-blunt DNA fragment, 5 µl of ligation solution I was added and reacted at 16°C for 30 min. Using 10 µl of the resultant ligation solution, *E. coli* JM109 competent cells (Toyobo) were transformed and plated on 10 µg/ml tetracycline-containing LB agar medium. Then, tetracycline resistant colonies growing on the medium were selected. The resultant transformant was cultured overnight in LB medium. The plasmid in the transformant was recovered using QIAprep8 Miniprep Kit to thereby obtain a fusion protein expression vector pTFC.

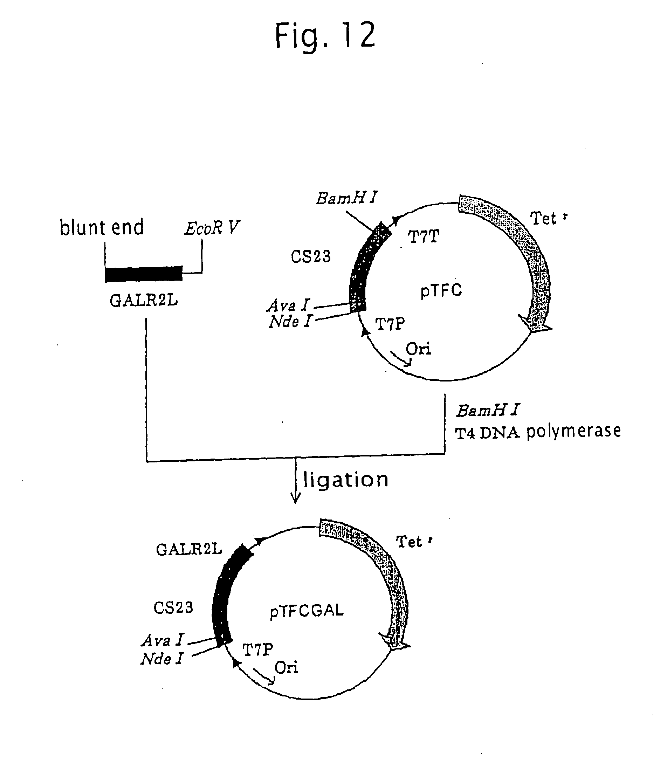

### b) Construction of Pig-Type Ligand Peptide (1-60) Expressing Strain (see Fig. 12)

The fusion protein expression vector pTFC (1 µg) was digested with BamHI, blunt-ended with T4 DNA polymerase (DNA Blunting Kit; Takara Shuzo), and subjected to 1% agarose gel electrophoresis. An approx. 4.2 kbp DNA fragment was extracted from the gel using QIAquick Gel Extraction Kit and dissolved in 50 µl of TE buffer. This DNA fragment and the structural gene of the polypeptide of the invention prepared in Reference Example 20 were ligated using Takara Ligation Kit Ver2 (Takara Shuzo). Briefly, 1 µl of the BamHI-digested and blunt-ended pTFC solution and 4 µl of a solution of the structural gene of the polypeptide of the invention were mixed. Ligation solution I (5 µl) was added to the mixture and reacted at 16° for 30 min. Using 10 µl of the resultant ligation solution, *E. coli* JM109 competent cells (Toyobo) were transformed, plated on 10 µg/ml tetracycline-containing LB agar medium and cultured at 37°C for one day. Then, tetracycline resistant colonies growing on the medium were selected.

The resultant transformant was cultured overnight in LB medium. The plasmid in the transformant was recovered using QIAprep8 Miniprep Kit to thereby obtain the polypeptide of the invention-expression plasmid pTFCGAL. The nucleotide sequence of the structural gene moiety encoding the fusion protein in this plasmid was confirmed using Applied Biosystems Model 377 DNA sequencer. This expression plasmid pTFCGAL was transformed into *E. coli* MM294(DE3), which was then plated on 10 µg/ml tetracycline-containing LB agar medium and cultured at 37°C for one day, followed by selection of tetracycline resistant transformants. From the transformant, the polypeptide of the invention-expressing strain MM294(DE3)/pTFCGAL was obtained.

### REFERENCE EXAMPLE 22:

### Cultivation of the Pig-Type Ligand Peptide (1-60) Expressing Strain

The polypeptide of the invention-expressing strain MM294(DE3)/pTFCGAL obtained in Reference Example 21 was cultured in 1 L of 5 mg/ml tetracycline-containing LB medium at 37°C for 16 hr under shaking. The resultant culture broth was transferred into a 50 L-fermenter containing 20 L of primary fermentation medium (1.68% sodium monohydrogenphosphate, 0.3% potassium dihydrogenphosphate, 0.1 % ammonium chloride, 0.05% sodium chloride, 0.05% magnesium sulfate, 0.0005% thiamine sulfate, 1.5% glucose, 1.0% casamino acid, 1.0% yeast extract) and cultured at 37°C under aeration at a gas flow of 20 L/min and agitation at 200 rpm. When the turbidity of the culture broth reached about 1200 Klett Units, isopropyl-β-D-thiogalactopyranoside (IPTG) was added to give a final concentration of 23.8 mg/L. Thirty minutes and 150 minutes after the addition of IPTG, 0.75% glucose was added. The cultivation was continued up to 10 hours after the start of the cultivation. The culture broth was separated by centrifugation at 5000 rpm for 30 min to thereby obtain about 830 g of cells.

### REFERENCE EXAMPLE 23:

### Purification of Pig-Type Ligand Peptide (1-60)

The cells (200 g) obtained in Reference Example 21 were suspended in 600 ml of 50 mM phosphate buffer (pH 7.0) containing 150 mM sodium chloride, 0.1 mM p-amidino-phenyl-methane-sulfonyl fluoride (APMSF) and 0.1 mM EDTA, sonicated (Branson Sonifier Model 450), and centrifuged at 10000 rpm for 10 min. The supernatant was discarded to thereby obtain an inclusion body. This inclusion body was suspended in 60 nil of distilled water and dissolved by adding thereto 140 ml of formic acid. To this solution, 2 g of cyanogen bromide was added and reacted at room temperature for 24 hr in order to cut CS23 from the pig-type ligand peptide (1-60) of the invention in the fusion protein at the C-terminal peptide bond of the Met linking these peptides. After the completion of the reaction, the solution was dialyzed against distilled water overnight and centrifuged (10000 rpm, 10 min). The supernatant was dialyzed against 50 mM Tris-HCl (pH 7.5) overnight and, after adjustment of its pH to 6.0 with HCl, centrifuged at 10000 rpm for 10 min to thereby obtain about 400 ml of supernatant. This supernatant was fed to a CM-TOYOPERAL 650 M column (3.0 cm ID x 10 cm L; Tosoh) pre-equilibrated with 50 mM sodium acetate buffer (pH 6.0) at a flow rate of 10 ml/min for adsorption. Then, the column was washed sufficiently with the same buffer used for the equilibration. Elution was performed with a linear gradient of 0-100% Solution B (50 mM sodium acetate buffer + 1 M sodium chloride, pH 6.0) to thereby obtain fractions containing the pig-type ligand peptide (1-60) of the invention. One-third (1/3) volume of these fractions was fed to a C4P-50 column (2.15 cm ID x 30 cm L; Showa Denko) pre-equilibrated with 0.1% trifluoroacetic acid at a flow rate of 5 ml/min for adsorption. The adsorbate was eluted with a linear gradient of 33-43 % Solution B (80% acetonitrile/0.1 % trifluoroacetic acid). For the remaining 2/3 volumes, the same operations were performed. Those fractions containing the pig-type ligand peptide (1-60) of the invention were thus recovered, and lyophilized to thereby obtain 67 mg of a purified product of the pig-type ligand peptide (1-60) of the invention.

### a) Analysis by SDS-Polyacrylamide Gel Electrophoresis

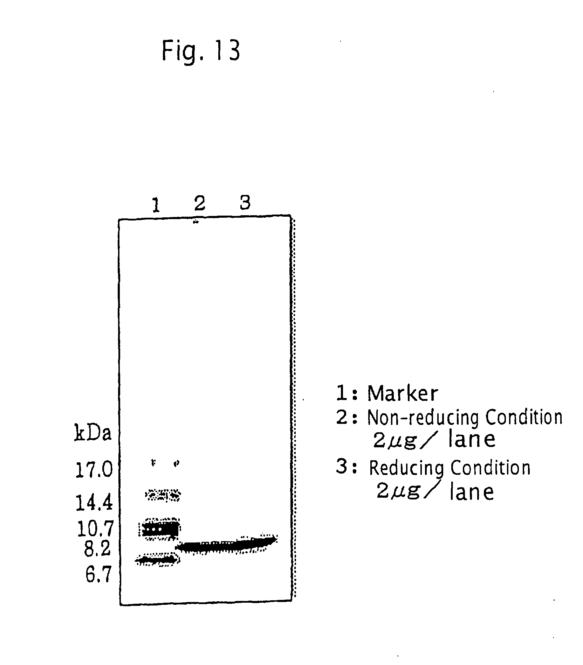

The purified product of the pig-type ligand peptide (1-60) of the invention was dissolved in 0.0625 Tris-HCl (pH 6.8) containing 2% SDS, 10% glycerol, 5% 2-mercaptoethanol and 0.001% bromophenol blue [Leammli, U.K., Nature 227:680 (1979)], boiled for 3 min, and electrophoresed on Multigel 15/25 (Daiichi Pure Chemicals). The resultant gel was stained with Rapid CBB KANTO (Kanto Kagaku). As a result, a single band was observed (see Fig. 13).

### b) Analysis of the Amino Acid Composition

The purified product of the pig-type ligand peptide (1-60) of the invention was hydrolyzed in vapor phase with 1% phenol-containing 6N HCl at 110°C for 24 and 48 hr. Then, the amino acid composition was determined with an amino acid analyzer (Hitachi L-8500A Amino Acid Analyzer). As shown in Table 5, the analysis results were consistent with the amino acid composition predicted from the cDNA sequence.

**Table 5:**

| Amino Acid | Residues per mol | Predicted from the nucleotide sequence |
|---|---|---|
| Asp | 3.0 | 3 |
| Thr | 2.0 | 2 |
| Ser | 3.7 | 4 |
| Glu | 3.0 | 3 |
| Pro | 5.8 | 6 |
| Gly | 10.6 | 11 |
| Ala | 5.8 | 6 |
| Val | 1.9 | 2 |
| Ile | 1.9 | 2 |
| Leu | 9.0 | 9 |
| Tyr | 1.9 | 2 |
| Lys | 2.9 | 3 |
| His | 1.9 | 2 |
| Trp | 1.3 | 2 |
| Arg | 2.7 | 3 |
| For Thr and Ser, data were extrapolated at 0 hr. | | |
| For Val and Ile, data obtained by the 48 hr hydrolysis were used. | | |

### c) Analysis of the N-Terminal Amino Acid Sequence

The N-terminal amino acid sequence was determined with a vapor-phase protein sequencer (Applied Biosystems; Model 477A). As shown in Table 6, the determined N-terminal amino acid sequence was consistent with the amino acid composition predicted from the cDNA sequence.

**Table 6:**

| Base No. | Detected PTH¹⁾-Amino Acid (pmol) | Amino Acid predicted from the nucleotide sequence |
|---|---|---|
| 1 | Ala (823) | Ala |
| 2 | Pro (676) | Pro |
| 3 | Val(719) | Val |
| 4 | His (355) | His |
| 5 | Arg (418) | Arg |
| 6 | Gly (548) | Gly |
| 7 | Arg (339) | Arg |
| 8 | Gly (470) | Gly |
| 9 | Gly (575) | Gly |
| 10 | Trp(189) | Trp |
| Analysis was carried out using 1 nmol of the sample. 1) phenylthiohydantoin | | |

### d) Analysis of the C-Terminal Amino Acids

The purified product of the pig-type ligand peptide (1-60) of the invention was hydrolyzed in vapor phase with anhydrous hydrazine at 100°C for 3.5 hr. Then, the C-terminal amino acids were determined with an amino acid analyzer (Hitachi L-8500A Amino Acid Analyzer). As shown in Table 7, the analysis results were consistent with the amino acids predicted from the cDNA sequence.

**Table 7:**

| C-terminal Amino Acid | Recovery (%) |
|---|---|
| Ser | 61.5 |
| Gas phase hydrazinolysis (100°C, 3.5 hr) | |

### REFERENCE EXAMPLE 24:

### Feeding Test

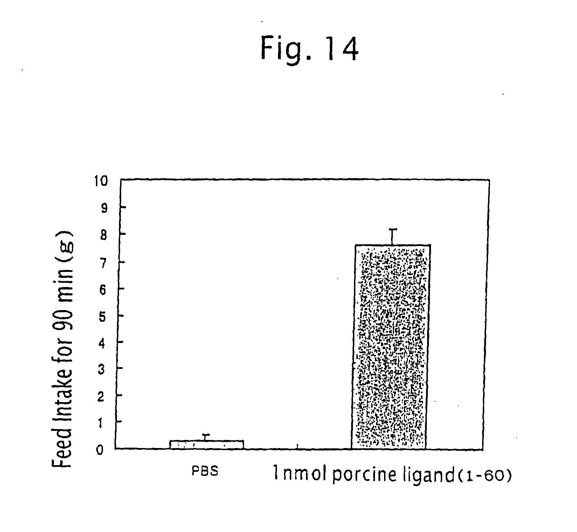

A guide cannula was inserted into the third ventricle (AP: -7.1, L: 0.0, H: 2.2 mm) of male Wistar rats (8 week old) anesthetized with pentobarbital. A feeding experiment was carried out after the rats were given one week for recovery from surgery. Briefly, the rats were kept under 12 hr light/12 hr dark cycles (light: 8:00-20:00), and the feeding experiment started at 16:00. A microinjection cannula was installed in each rat under no anesthetization nor restraints. The pig-type peptide (1-60) (SEQ ID NO: 31) dissolved in PBS or PBS alone was administered through the cannula for 4 min at 2.5 µl/min. One minute after the completion of the administration, the microinjection cannula was removed, and the rats were allowed to eat the standard feed freely. The amount of feed taken by the rat within 90 min after the completion of the administration was calculated and taken as the amount of feeding (Fig. 14).

### REFERENCE EXAMPLE 25:

### Cloning of cDNA Encoding Mouse-Type Ligand Peptide

A PCR reaction was performed using Mouse Testis Marathon-Ready cDNA (derived from BALB/c mouse; Clontech) as a template and primers F/R120 and R/R120 (described in WO 99/48920).

F/R120: 5'-AGGCTGGACCCTCAATAGTGCTGGTTAC-3' (SEQ ID NO: 65)

R/R120: 5'-CCATCTATGGCCTTCCACAGGTCTAGGA-3' (SEQ ID NO: 66)

The PCR reaction solution was prepared by mixing 0.5 µl of Ex Taq (Takara Shuzo), 5 µl of 10x PCR buffer attached, 4 µl of 2.5 mM dNTP mixture, 25 µl of [α-³²P]dCTP (6000 Ci/mmol), 0.5 µl each of primers F/R120 and R/R120 (each 10 µM), 1 µl of the template cDNA and 34.5 µl of distilled water. The reaction conditions were as follows: after initial denaturation at 94°C for 30 sec, 35 cycles of (94°C, 30 sec; 62°C, 30 sec; 72°C, 60 sec) and final extension reaction at 72°C for 10 min were performed. The resultant DNA fragment was cloned using TOPO TA Cloning Kit (Invitrogen) according to the method described in the attached manual. The sequence of the cloned DNA was analyzed with ABI 377 DNA Sequencer to thereby obtain the sequence of SEQ ID NO: 67.

AGGCTGGACC CTCAATAGTG CTGGTTACCT CCTGGGTCCT GTCCTCCCCG TTTCCTCCAA 60
GGCCGACCAG GGCAGGAAGA GAGACTCAGC TCTTGAGATC CTAGACCTGT GGAAGGCCAT 120
AGATGG 126
(SEQ ID NO: 67)

From the resultant sequence, primers 1F/M120 (SEQ ID NO: 68) and 1R/M120 (SEQ ID NO: 69) were prepared and used in the 5' RACE and 3'RACE experiments described below.

1F/M120: 5'-TCCAAGGCCGACCAGGGCAGGAAGAGAG -3' (SEQ ID NO: 68)

1R/M120: 5'-GGTCTAGGATCTCAAGAGCTGAGTCTCT-3' (SEQ ID NO: 69)

PCR reaction solutions for 5' RACE and 3'RACE were prepared by mixing 0.5 µl of Taq (Takara Shuzo), 5 µl of 10x PCR buffer attached thereto (500 mM KCI, 25 mM MgCl₂, 100 mM Tris-HCl, pH 8.3), 4 µl of 2.5 mM dNTP mixture, 3 µl of 25 mM MgCl₂, 1 µl of 10 µM primer F/R120 (for 3'RACE) or 10 µM primer R/R120 (for 5'RACE), 1 µl of 10 µM primer AP1 (this primer is contained in Clontech's Marathon-Ready cDNA Kit); 5 µl of template cDNA (Clontech; mouse testis Marathon-Ready cDNA) and 31 µl of distilled water. The reaction conditions were as follows: after initial denaturation at 94°C for 60 sec, 5 cycles of (94°C, 30 sec; 72°C, 120 sec), 5 cycles of (94°C, 30 sec; 70°C, 120 sec), 25 cycles of (94°C, 30 sec; 68°C, 120 sec) and final extension reaction at 68°C for 10 min were performed.

Subsequently, using the resultant reaction solution as a template, nested PCR was performed. The reaction solution for this PCR was prepared by mixing 0.5 µl of Taq (Takara Shuzo), 5 µl of 10x PCR buffer attached thereto (500 mM KCl, 25 mM MgCl₂, 100 mM Tris-HCl, pH 8.3), 4 µl of 2.5 mM dNTP mixture, 3 µl of 25 mM MgCl₂, 1 µl of 10 µM primer 1F/M120 (for 3'RACE) or 10 µM primer 1R/M120 (for 5'RACE), 1 µl of 10 µM primer AP2 (this primer is contained in Clontech's Marathon-Ready cDNA Kit), 5 µl of template DNA (50-fold dilution of the above PCR reaction solution) and 31 µl of distilled water. The reaction conditions were as follows: after initial denaturation at 94°C for 60 sec, 5 cycles of (94°C, 30 sec; 72°C, 120 sec), 5 cycles of (94°C, 30 sec; 70°C, 120 sec), 25 cycles of (94°C, 30 sec; 68°C, 120 sec) and final extension reaction at 68°C for 10 min were performed.

The resultant DNA fragment was cloned using TOPO TA Cloning Kit (Invitrogen) according to the method described in the attached manual. The thus cloned DNA fragment was analyzed to thereby obtain sequence information on the 5' end and the 3' end. Based on this information, primers 1F/M650 and 1R/M650 were prepared.

1F/H650: 5'-GAAAGACAGTCTCAAATCAAGGACCTGC-3' (SEQ ID NO: 70)

1R/H650: 5'-TGAGCTGGAAACGAAGGAGGAGTGAGGT-3' (SEQ ID NO: 71)

A single-stranded DNA was synthesized from BALB/c mouse-derived testis poly(A)⁺ RNA (Nippon Gene) using Superscript II reverse transcriptase (Gibco BRL) by conventional methods. Using this DNA as a template and primers F/M650 and R/M650, a PCR reaction was performed. The reaction solution was prepared by mixing 1 µl of Pfu DNA polymerase (Stratagene), 5 µl of 10x PCR buffer attached, 4 µl of 2.5 mM dNTP mixture, 2.5 µl each of 10 µM primer F/M650 and 10 µM primer R/M650, 1 µl of the template DNA and 34.5 µl of distilled water. The reaction conditions were as follows: after initial denaturation at 94°C for 30 sec, 30 cycles of (94°C, 30 sec; 67°C, 30 sec; 72°C, 4 min) and final extension reaction at 72°C for 10 min were performed. The resultant DNA fragment was cloned into pCRII Blunt TOTO vector (Invitrogen) according to the method described in the attached manual. The cloned DNA sequence was sequenced by conventional methods. Thus, plasmid pGR2ML1 having a 180 bp DNA fragment (SEQ ID NO: 64) encoding the mouse GALR2 ligand full-length peptide was obtained. *E. coli* TOP10 transformed with this plasmid was designated TOP10/ pGR2ML1. The amino acid sequence deduced from this 180 bp nucleotide sequence is shown in SEQ ID NO: 63. The mature moiety thereof is shown in SEQ ID NO: 62.

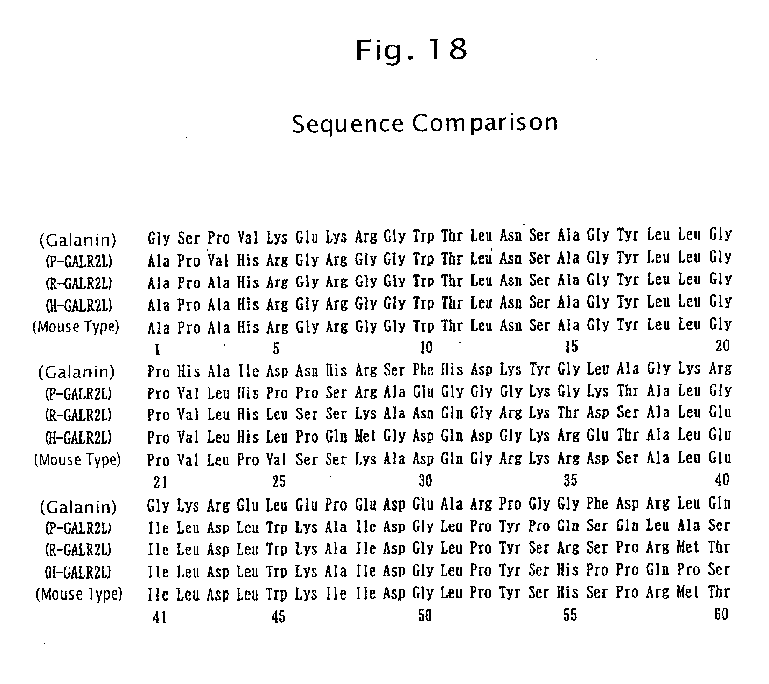

When the amino acid sequence of the mature moiety was compared with the amino acid sequences of pig-type intestine galanin (galanin), pig-type GalR2 ligand (P-GALR2L). rat-type GalR2 ligand (R-GALR2L) and human-type GalR2 ligand (H-GALR2L), the amino acid sequence of the mature moiety exhibited a high homology to the amino acid sequences of pig-type GalR2 ligand (P-GALR2L), rat-type GalR2 ligand (R-GALR2L) and human-type GalR2 ligand (H-GALR2L) (see Fig. 18).

### EXAMPLE 1

Effects of GALP administration into the third ventricle on pituitary hormone levels in plasma were examined. Briefly, adult male Wistar rats (body weight: 350-380 g at the time of surgery) were anesthetized by intraperitoneal administration of 50 mg/kg pentobarbital and fixed in a stereotaxic apparatus for rat brain. The incisor bar was set 3.3 mm below the interaural line. The skull surface was exposed, and a hole was drilled in the skull with a dental drill in order to implant a guide cannula AG-8 (inside dia. 0.4 mm, outside dia. 0.5 mm; Eicom) in the third ventricle. Further, four anchor screws were embedded around the hole. A stainless guide cannula (AG-12) was inserted so that its tip was located above the third ventricle. The stereotaxic coordinates were set as follows according to the atlas of Paxinos and Watson (1998): AP: -0.8 mm, L: 0.0 mm, H: -0.54 mm from bregma. The guide cannula was fixed onto the skull with instant glue, dental cement and the anchor screws. A stainless dummy cannula AD-12 (outside dia. 0.35 mm; Eicom) was inserted into the guide cannula and fixed with a cap nut (Eicom). After the surgery, the rats were kept separately in individual cages.

The rats were allowed about one week for recovery after the implantation of the guide cannula. Then, the cap nut and the dummy cannula installed on the rat skull were removed. Instead of them, a stainless microinjection cannula (inside dia. 0.17 mm, outside dia. 0.35 mm; Eicom) connected with a Teflon tube (length 50 cm, inside dia. 0.1 mm, outside dia. 0.35 mm; Eicom) was inserted into the guide cannula. The length of the microinjection cannula was adjusted in advance so that 1 mm of it tip was exposed from the guide cannula. One end of the Teflon tube was connected with a microsyringe pump. Then, a total 10 µl of phosphate buffered saline (PBS) (pH 7.2) or rat GALP (a peptide consisting of the amino acid sequence as shown in SEQ ID NO: 33; 0.5 nmol, 5 nmol) dissolved in PBS was injected into the lateral ventricle at a flow rate of 5 µl/min. Five minutes after the completion of the injection, the microinjection cannula was removed, and a dummy cannula was fixed again with a cap nut. Ten minutes after the completion of the injection, the rats were decapitated and the blood was collected. In order to prevent blood coagulation, 300 µl of 300 KIU/ml aprotinin solution containing 3 mg/ml EDTA was added in advance into 50 ml tubes (Coming). The collected blood was centrifuged at 2,500 rpm for 25 min in a high-speed refrigerated centrifuge (HIMAC 5DL; Hitachi) to recover the supernatant (plasma). Growth hormone (GH), thyroid-stimulating hormone (TSH), follicle-stimulating hormone (FSH), LH, prolactin and adorenocorticotropic hormone (ACTH) contained in the plasma were quantified by radioimmunoassay (GH, TSH, FSH, LH and prolactin; Amersham) (ACTH; Mitsubishi Kagaku).

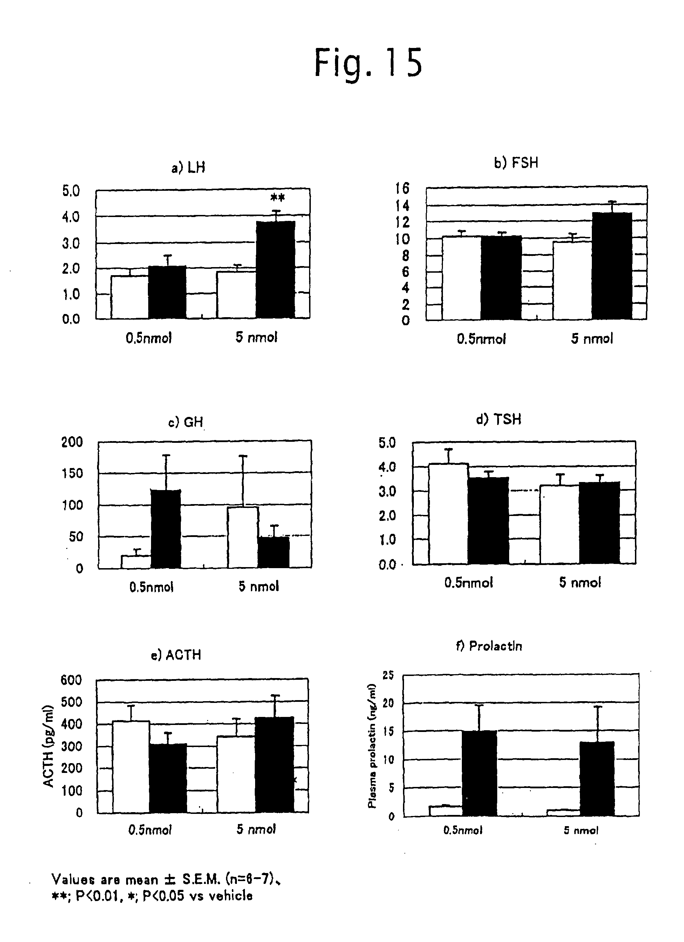

As shown in Fig. 15, a significant increase was observed only in blood LH level in a concentration dependent manner in rat GALP administration groups compared to the control group.

### EXAMPLE 2

A guide cannula was implanted in adult male Wistar rats treated in the same manner as described in Example 1. The rats were allowed about one week for recovery from the surgery. Then, they received another surgery so that blood samples can be taken from them moving freely. Briefly, the guide cannula-implanted rat was anesthetized by intraperitoneal administration of 5 mg/kg pentobarbital and fixed on an anatomical pad in face-up position. Then, the left jugular vein was exposed. A polyethylene tube SP35 (inside dia. 0.5 mm, outside dia. 0.9 mm; Natsume Seisakusho) was cut to give an about 30 cm length, filled with 200 U/ml heparin-containing physiological saline, inserted into the jugular vein about 4.5 cm, and fixed. The other end of the tube was passed subcutaneously through dorsal skin and exposed from the neck (dorsal).

After one night from the surgery, 300 µl of blood was collected from each rat using a 1 ml injection syringe for tuberculin and a 25-gauge injection needle (both Terumo Corp.) prior to the administration of rat GALP (a peptide consisting of the amino acid sequence as shown in SEQ ID NO: 33). In order to prevent blood coagulation, 10 µl of 300 KIU/ml aprotinin solution containing 3 mg/ml EDTA was added in advance into the injection syringe. The cap nut and the dummy cannula installed on the rat skull were removed. Instead of them, a stainless microinjection cannula (inside dia. 0.17 mm, outside dia. 0.35 mm; Eicom) connected with a Teflon tube (length 50 cm, inside dia. 0.1 mm, outside dia. 0.35 mm; Eicom) was inserted into the guide cannula. The length of the microinjection cannula was adjusted in advance so that 1 mm of it tip was exposed from the guide cannula. One end of the Teflon tube was connected with a microsyringe pump. Then, a total 10 µl of phosphate buffered saline (PBS) (pH 7.2), rat GALP (0.5 nmol, 5 nmol) dissolved in PBS, or rat galanin (5 nmol) dissolved in PBS was injected into the third ventricle at a flow rate of 5 µl/min. Five minutes after the completion of the injection, the microinjection cannula was removed, and a dummy cannula was fixed again with a cap nut. A 300 µl blood sample was collected at 0, 10, 20, 30 and 60 min after the start of the intraventricular administration. The collected blood was centrifuged at 13,000 rpm for 5 min in a high-speed refrigerated microcentrifuge (MR-150; Tomy Seiko) to recover the supernatant (plasma). The LH levels in the plasma were measured by radioimmunoassay (Amersham).

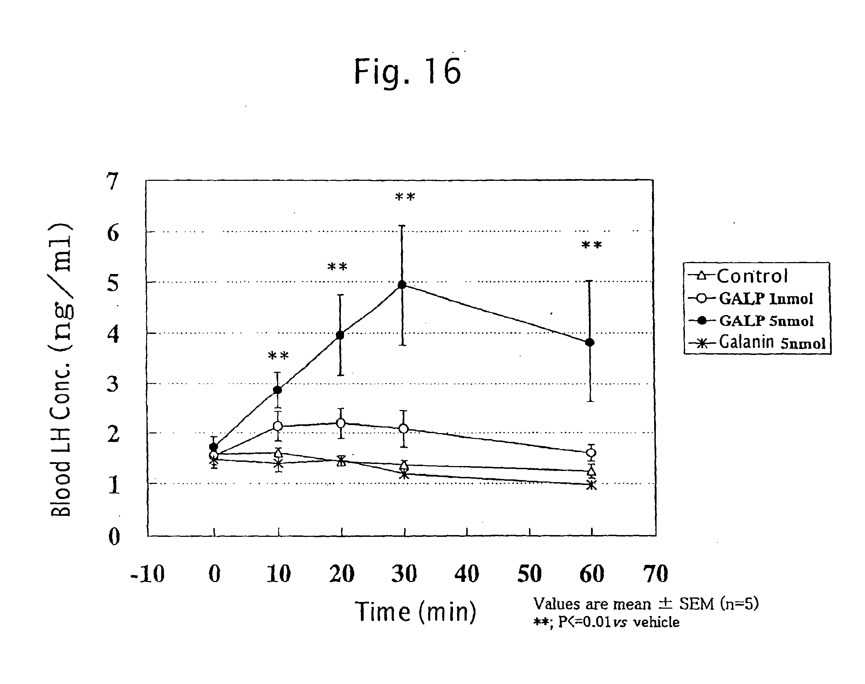

As shown in Fig. 16, a tendency to rise was observed in GALP 1 nmol administration group; and a significant rise was observed at any time of the blood collection in GALP 5 nmol administration group with a peak at 30 min after the administration, when compared with the control group. On the other hand, no change was observed in blood LH levels in galanin administration groups.

### EXAMPLE 3

Effects of GALP administration into the third ventricle on LH levels in plasma were examined using Zucker obese rats having an abnormality of Leptin receptor. Briefly, adult male Zucker obese rats (body weights at the time of surgery: fatty: 410-430 g; lean: 290-310 g) were anesthetized by intraperitoneal administration of 50 mg/kg pentobarbital and fixed in a stereotaxic apparatus for rat brain. The incisor bar was set 3.3 mm below the interaural line. The skull surface was exposed, and a hole was drilled in the skull with a dental drill in order to implant a guide cannula AG-8 (inside dia. 0.4 mm, outside dia. 0.5 mm; Eicom) in the third ventricle. Further, four anchor screws were embedded around the hole. A stainless guide cannula (AG-12) was inserted so that its tip was located above the third ventricle. The stereotaxic coordinates were set as follows according to the atlas of Paxinos and Watson (1998): for Zucker fatty, AP: -1.00 mm, L: 0.0 mm, H: 5.6 mm from bregma, and for Zucker lean, AP: -0.9 mm, L: 0.0 mm, H: 5.4 mm from bregma. The guide cannula was fixed onto the skull with instant glue, dental cement and the anchor screws. A stainless dummy cannula AD-12 (outside dia. 0.35 mm; Eicom) was inserted into the guide cannula and fixed with a cap nut (Eicom). After the surgery, the rats were kept separately in individual cages.

The rats were allowed about one week for recovery after the implantation of the guide cannula. Then, they received another surgery in the same manner as in the preceding Example so that blood samples can be taken from them moving freely. After one night from the surgery, 300 µl of blood was collected from each rat using a 1 ml injection syringe for tuberculin and a 25-gauge injection needle (both Terumo Corp.) prior to GALP administration. In order to prevent blood coagulation, 10 µl of 300 KIU/ml aprotinin solution containing 3 mg/ml EDTA was added in advance into the injection syringe. The cap nut and the dummy cannula installed on the rat skull were removed. Instead of them, a stainless microinjection cannula (inside dia. 0.17 mm, outside dia. 0.35 mm; Eicom) connected with a Teflon tube (length 50 cm, inside dia. 0.1 mm, outside dia. 0.35 mm; Eicom) was inserted into the guide cannula. The length of the microinjection cannula was adjusted in advance so that 1 mm of it tip was exposed from the guide cannula. One end of the Teflon tube was connected with a microsyringe pump. Then, a total 10 µl of phosphate buffered saline (PBS) (pH 7.2) or rat GALP (1 nmol) dissolved in PBS was injected into the third ventricle at a flow rate of 5 µl/min. Five minutes after the completion of the injection, the microinjection cannula was removed, and a dummy cannula was fixed again with a cap nut. A 300 µl blood sample was collected at 0, 10, 20, 30 and 60 min after the start of the intraventricular administration. The collected blood was centrifuged at 13,000 rpm for 5 min in a high-speed refrigerated microcentrifuge (MR-150; Tomy Seiko) to recover the supernatant (plasma). The LH levels in the plasma were measured by radioimmunoassay (Amersham).

As shown in Fig. 17, the GALP administration group of Zucker fatty rats exhibited a significant rise in blood LH levels as compared with the control group, at 10, 20 and 30 min after the administration. On the other hand, no significant rise in blood LH levels was observed in the GALP administration group of Zucker lean rats as compared with the control group. The blood LH level-raising effect of GALP was more exalted in Zucker fatty rats than Zucker lean rats.

### INDUSTRIAL APPLICABILITY

The polypeptide of the invention has an activity of enhancing the LH secretion, and thus can be used as a prophylactic and therapeutic agent for various diseases associated with the failure of the LH secretion. On the other hand, since the polypeptide of the invention has a high affinity to its receptor protein, a higher does of the polypeptide leads to the desensitization of the LH secretion, indicating that the polypeptide also shows an activity of inhibiting the LH secretion. In this case, the polypeptide can be used as a prophylactic and therapeutic agent for various diseases associated with the excess LH secretion.

## Claims

1. An agent regulating the luteinizing hormone (LH) secretion, which comprises a galanin-like peptide (GALP) or a salt thereof or a DNA encoding a galanin-like peptide.

2. The agent regulating the LH secretion according to claim 1, wherein the galanin-like peptide is a peptide comprising the same or substantially the same amino acid sequence as the sequence shown by SEQ ID NO: 35.

3. The agent regulating the LH secretion according to claim 1, wherein the galanin-like peptide is a peptide comprising the same or substantially the same amino acid sequence as the sequence shown by SEQ ID NO: 31, 33, 34 or 62.

4. The agent regulating the LH secretion according to claim 1, wherein the DNA encoding a galanin-like peptide is a DNA comprising a base sequence hybridizable to the base sequence shown by SEQ ID NO: 32, 39, 40 or 64 under high stringent condition.

5. The agent regulating the LH secretion according to claim 1, which is an agent enhancing the LH secretion.

6. The agent regulating the LH secretion according to claim 1, which is an agent inhibiting the LH secretion.

7. The agent enhancing the LH secretion according to claim 5, which is a pharmaceutical agent to prevent and/or treat infertility, menoxenia, dysmenorrhea, amenorrhea, oligomenorrhea, premenstrual syndrome, menopausal disorder, dyspituitarism or obesity.

8. The agent inhibiting the LH secretion according to claim 6, which is a pharmaceutical agent to prevent and/or treat prostate cancer, prostate hypertrophy, precocious puberty or LH-producing pituitary gland tumor.

9. An agent regulating the LH secretion, which comprises an activator of a galanin-like peptide.

10. An agent regulating the LH secretion, which comprises an inhibitor of a galanin-like peptide.

11. An agent regulating the luteinizing hormone-releasing hormone (LHRH) secretion, which comprises a galanin-like peptide or a salt thereof or a DNA encoding a galanin-like peptide.

12. The agent regulating the LHRH secretion according to claim 11, wherein the galanin-like peptide is a peptide comprising the same or substantially the same amino acid sequence as the sequence shown by SEQ ID NO: 35.

13. The agent regulating the LHRH secretion according to claim 11, wherein the galanin-like peptide is a peptide comprising the same or substantially the same amino acid sequence as the sequence shown by SEQ ID NO: 31, 33, 34 or 62.

14. The agent regulating the LHRH secretion according to claim 11, wherein the DNA encoding a galanin-like peptide is a DNA comprising a base sequence hybridizable to the base sequence shown by SEQ ID NO: 32, 39, 40 or 64 under high stringent condition.

15. The agent regulating the LHRH secretion according to claim 11, which is an agent enhancing the LHRH secretion.

16. The agent regulating the LHRH secretion according to claim 11, which is an agent inhibiting the LHRH secretion.

17. The agent enhancing the LHRH secretion according to claim 15, which is a pharmaceutical agent to prevent and/or treat infertility, menoxenia, dysmenorrhea, amenorrhea, oligomenorrhea, premenstrual syndrome, menopausal disorder, dyspituitarism or obesity.

18. The agent inhibiting the LHRH secretion according to claim 16, which is a pharmaceutical agent to prevent and/or treat prostate cancer, prostate hypertrophy, precocious puberty or LHRH-producing pituitary gland tumor.

19. An agent regulating the LHRH secretion, which comprises an activator of a galanin-like peptide.

20. An agent regulating the LHRH secretion, which comprises an inhibitor of a galanin-like peptide.

21. Use of a galanin-like peptide (GALP) or a salt thereof or a DNA encoding a galanin-like peptide for producing an agent regulating the LH secretion or an agent regulating the LHRH secretion.

22. A method of preventing or treating disorders of the LH secretion regulation or disorders of the LHRH secretion regulation in a mammal, which comprises administering a pharmacologically effective amount of a galanin-like peptide (GALP) or a salt thereof or a DNA encoding a galanin-like peptide to said mammal.
